# EUROPEAN PATENT SPECIFICATION

(11) **EP 3 535 289 B1**
(45) Date of publication and mention of the grant of the patent: **17.07.2024**
(21) Application number: 17801375.1
(22) Date of filing: 07.11.2017
(51) Int. Cl.: C07K 14/725, C12N 5/0783, C12N 15/85, C12N 15/63

(54) **AN ENGINEERED MULTI-COMPONENT SYSTEM FOR IDENTIFICATION AND CHARACTERISATION OF T-CELL RECEPTORS, T-CELL ANTIGENS AND THEIR FUNCTIONAL INTERACTION**
MANIPULIERTES MEHRKOMPONENTENSYSTEM ZUR IDENTIFIZIERUNG UND CHARAKTERISIERUNG VON T-ZELL-REZEPTOREN, T-ZELL-ANTIGENEN UND DEREN FUNKTIONELLER INTERAKTION
SYSTÈME À COMPOSANTS MULTIPLES GÉNÉTIQUEMENT MODIFIÉS POUR L'IDENTIFICATION ET LA CARACTÉRISATION DE RÉCEPTEURS DE LYMPHOCYTES T, D'ANTIGÈNES DE LYMPHOCYTES T ET DE LEUR INTERACTION FONCTIONNELLE

(30) Priority: 07.11.2016 DK PA201670875
(43) Date of publication of application: 11.09.2019
(73) Proprietor: Genovie AB, 151 36 Södertälje (SE)
(72) Inventor: JARVIS, Reagan Micheal, 151 36 Södertälje (SE); HILL, Ryan Edward, 151 36 Södertälje (SE); PASE, Luke Benjamin, 151 36 Södertälje (SE)
(74) Representative: Aera A/S
(86) International application number: PCT/EP2017/078377
(87) International publication number: WO 2018/083317

(56) References cited:
- EP-A1- 2 899 269
- WO-A1-2016/069282
- WO-A2-2014/153470
- CN-A- 105 316 362
- HIROSHI HAMANA ET AL: "A novel, rapid and efficient method of cloning functional antigen-specific T-cell receptors from single human and mouse T-cells", BIOCHEMICAL AND BIOPHYSICAL RESEARCH COMMUNICATIONS, vol. 474, no. 4, 4 May 2016 (2016-05-04), AMSTERDAM, NL, pages 709 - 714, XP055386579, ISSN: 0006-291X, DOI: 10.1016/j.bbrc.2016.05.015
- XI-ZHI J GUO ET AL: "Rapid cloning, expression, and functional characterization of paired alpha/beta and gamma/deltaT-cell receptor chains from single-cell analysis", MOLECULAR THERAPY - METHODS AND CLINICAL DEVELOPMENT, vol. 3, 27 January 2016 (2016-01-27), GB, pages 15054, XP055386592, ISSN: 2329-0501, DOI: 10.1038/mtm.2015.54
- YI ZHANG ET AL: "Transduction of Human T Cells with a Novel T-Cell Receptor Confers Anti-HCV Reactivity", PLOS PATHOGENS, vol. 6, no. 7, 29 July 2010 (2010-07-29), pages e1001018, XP055386597, DOI: 10.1371/journal.ppat.1001018
- EIJI KOBAYASHI ET AL: "A new cloning and expression system yields and validates TCRs from blood lymphocytes of patients with cancer within 10 days", NATURE MEDICINE, vol. 19, no. 11, 13 October 2013 (2013-10-13), pages 1542 - 1546, XP055248685, ISSN: 1078-8956, DOI: 10.1038/nm.3358
- SOEREN TURAN ET AL: "Recombinase-mediated cassette exchange (RMCE) - A rapidly-expanding toolbox for targeted genomic modifications", GENE., vol. 515, no. 1, 1 February 2013 (2013-02-01), NL, pages 1 - 27, XP055383251, ISSN: 0378-1119, DOI: 10.1016/j.gene.2012.11.016
- RICHMAN ET AL: "Display, engineering, and applications of antigen-specific T cell receptors", BIOMOLECULAR ENGINEERING, ELSEVIER, NEW YORK, NY, US, vol. 24, no. 4, 16 September 2007 (2007-09-16), pages 361 - 373, XP022251571, ISSN: 1389-0344, DOI: 10.1016/J.BIOENG.2007.02.009

## Description

### Field of the invention

The present invention relates to the construction, assembly and use of a multi-component system, comprised of at least three components being, an engineered T-cell receptor (TCR) presenting cell (eTPC), an engineered genomic receiver site and a matching genetic donor vector. The present invention is used for rapid, high-throughput generation of stable derivative cells that present full-length TCR pairs (eTPC-t) for identification and characterisation of the TCR pair proteins to analyte antigen. Specifically, the eTPC constitutively expresses all components of the CD3 complex, but lacks endogenous expression of TCR alpha, beta, gamma and delta chains. The eTPC also lacks expression of major antigen-presenting complexes, as to eliminate potential background of self-presentation of antigen by the eTPC in analytical workflows. Additionally, the eTPC contains at least two engineered genomic receiver sites. The sites are used for rapid, stable integration of genetic material encoding TCR pairs, delivered via a matched genetic donor vector. The eTPC may also include an optional component, a TCR-stimulation response element, for detection of TCR stimulation. The multi-component system may be used as an analytical system in clinical immunodiagnostics. Furthermore, the present invention relates to the use the multicomponent system to identify and characterise T-cell antigens and cognate TCRs for diagnostics, medicine, cosmetics and research and development.

### Introduction to the invention

Immune surveillance by T lymphocytes (T-cells) is a central function in the adaptive immunity of all jawed vertebrates. Immune surveillance by T-cells is achieved through a rich functional diversity across T-cell subtypes, which serve to eliminate pathogen-infected and neoplastic cells and orchestrate adaptive immune responses to invading pathogens, commensal microorganisms, commensal non-self factors such as molecular components of foodstuffs, and even maintain immune tolerance of self. In order to respond to various foreign and self factors, T-cells must be able to specifically detect molecular constituents of these foreign and self factors. Thus T-cells must be able to detect a large cross-section of the self and non-self molecules that an individual encounters, with sufficient specificity to mount efficient responses against pathogenic organisms and diseased self, while avoiding the mounting of such responses against health self. The highly complex nature of this task becomes clear when considering the practically unlimited diversity of both foreign and self molecules, and that pathogenic organisms are under evolutionary pressure to evade detection by T-cells.

### The T-cell Receptor (TCR)

T-cells are primarily defined by the expression of a T-cell receptor (TCR). The TCR is the component of the T-cell that is responsible for interacting with and sensing the targets of T-cell adaptive immunity. In general terms, the TCR is comprised of a heterodimeric protein complex presented on the cell surface. Each of the two TCR chains are composed of two extracellular domains, being the variable (V)-region and the constant (C)-region, both of the immunoglobulin superfamily (IgSF) domain, forming antiparallel β-sheets. These are anchored in the cell membrane by a type-I transmembrane domain, which adjoins a short cytoplasmic tail. The quality of the T-cells to adapt and detect diverse molecular constituents arises from variation in the TCR chains that is generated during T-cell genesis. This variation is generated by somatic recombination in a similar manner to antibody genesis in B-cells.

### TCR chain diversity

The T cell pool consists of several functionally and phenotypically heterogeneous subpopulations. However, T cells may be broadly classified as αβ or γδ according to the somatically rearranged TCR form they express at their surface. There exist two TCR chain pair forms; TCR alpha (TRA) and TCR beta (TRB) pairs; and TRC gamma (TRG) and TCR delta (TRD) pairs. T-cells expressing TRA:TRB pairs are referred to as αβ T-cells, while T-cells expressing TRG:TRD pairs are often referred to as γδ T-cells.

TCRs of both αβ and γδ forms are responsible for recognition of diverse ligands, or 'antigens', and each T-cell generates αβ or γδ receptor chains de novo during T-cell maturation. These de novo TCR chain pairs achieve diversity of recognition through generation of receptor sequence diversity in a process called somatic V(D)J recombination after which each T-cell expresses copies of a single distinctly rearranged TCR. At the TRA and TRG loci, a number of discrete variable (V) and functional (J) gene segments are available for recombination and juxtaposed to a constant (C) gene segments, thus referred to as VJ recombination. Recombination at the TRB and TRD loci additionally includes a diversity (D) gene segment, and is referred to as VDJ recombination.

Each recombined TCR possess potential for unique ligand specificity, determined by the structure of the ligand-binding site formed by the α and β chains in the case of αβ T-cells or γ and δ chains in the case of γδ T-cells. The structural diversity of TCRs is largely confined to three short hairpin loops on each chain, called complementarity-determining regions (CDR). Three CDRs are contributed from each chain of the receptor chain pair, and collectively these six CDR loops sit at the membrane-distal end of the TCR extracellular domain to form the antigen-binding site.

Sequence diversity in each TCR chain is achieved in two modes. First, the random selection of gene segments for recombination provides basal sequence diversity. For example, TRB recombination occurs between 47 unique V, 2 unique D and 13 unique J germline gene segments. In general, the V gene segment contributes both the CDR1 and CDR2 loops, and are thus germline encoded. The second mode to generate sequence diversity occurs within the hypervariable CDR3 loops, which are generated by random deletion of template nucleotides and addition of non-template nucleotides, at the junctions between recombining V, (D) and J gene segments.

### TCR:CD3 Complex

Mature αβ and γδ TCR chain pairs are presented at the cell surface in a complex with a number of accessory CD3 subunits, denoted ε, γ, δ and ζ. These subunits associate with αβ or γδ TCRs as three dimers (εγ, εδ, ζζ). This TCR:CD3 complex forms the unit for initiation of cellular signalling responses upon engagement of a αβ or γδ TCR with cognate antigen. The CD3 accessories associated as a TCR:CD3 complex contribute signalling motifs called immunoreceptor tyrosine-based activation motifs (ITAMs). CD3ε, CD3γ and CD3δ each contribute a single ITAM while the CD3ζ homodimer contains 3 ITAMs. The three CD3 dimers (εγ, εδ, ζζ) that assemble with the TCR thus contribute 10 ITAMs. Upon TCR ligation with cognate antigen, phosphorylation of the tandem tyrosine residues creates paired docking sites for proteins that contain Src homology 2 (SH2) domains, such as the critical ζ-chain-associated protein of 70 kDa (ZAP-70). Recruitment of such proteins initiate the formation of TCR:CD3 signalling complexes that are ultimately responsible for T-cell activation and differentiation.

### αβ T-cells

αβ T-cells are generally more abundant in humans than their γδ T-cell counterparts. A majority of αβ T-cells interact with peptide antigens that are presented by HLA complexes on the cell surface. These peptide-HLA (pHLA)-recognising T-cells were the first to be described and are by far the best characterised. More rare forms of αβ T-cells have also been described. Mucosal-associated invariant T (MAIT) cells appear to have a relatively limited α and β chain diversity, and recognise bacterial metabolites rather than protein fragments. The invariant natural killer T-cells (iNK T-cells) and germline-encoded mycolyl-reactive T-cells (GEM T-cells) are restricted to recognition of glycolipids that are cross-presented by non-HLA molecules. iNK T-cells are largely considered to interact with CD1d-presented glycolipids, whereas GEM T-cells interact with CD1b-presented glycolipids. Further forms of T-cells are thought to interact with glycolipids in the context of CD1a and CD1c, however, such cells are yet to be characterised in significant detail.

### Conventional αβ T-cells

The key feature of most αβ T-cells is the recognition of peptide antigens in the context of HLA molecules. These are often referred to as 'conventional' αβ T-cells. Within an individual, self-HLA molecules present peptides from self and foreign proteins to T-cells, providing the essential basis for adaptive immunity against malignancies and foreign pathogens, adaptive tolerance towards commensal organisms, foodstuffs and self. The HLA locus that encodes HLA proteins is the most gene-dense and polymorphic region of the human genome, and there are in excess of 12,000 alleles described in humans. The high degree of polymorphism in the HLA locus ensures a diversity of peptide antigen presentation between individuals, which is important for immunity at the population level.

### HLA class I and II

There are two forms of classical HLA complexes: HLA class I (HLAI) and HLA class II (HLAII). There are three classical HLAI genes: *HLA-A, HLA-B, HLA-C.* These genes encode a membrane-spanning α-chain, which associates with an invariant β2-micro-globulin (β2M) chain. The HLAI α-chain is composed of three domains with an immunoglobulin fold: α1, α2 and α3. The α3 domain is membrane-proximal and largely invariant, while the α1 and α2 domains together form the polymorphic membrane-distal antigen-binding cleft. There are six classical HLAII genes: *HLA-DPA1, HLA-DPB1, HLA-DQA1, HLA-DQB1, HLA-DRA,* and *HLA-DRB1.* These genes encode paired DP, DQ and DR heterodimeric HLA complexes comprising a α-chain and a β-chain. Each chain has two major structural domains with an immunoglobulin fold, where the α2 and β2 domain comprise membrane-proximal and largely invariant modules similar to that of HLAI α3 domain. The HLAII α2 and β2 domains together form the membrane-distal antigen-binding cleft and are regions of high polymorphism.

The antigen-binding cleft of HLAI and HLAII comprises two anti-parallel α-helices on a platform of eight anti-parallel β-sheets. In this cleft the peptide antigen is bound and presented in an extended conformation. The peptide-contacting residues in HLAI and HLAII are the location of most of the sequence polymorphism, which constitutes the molecular basis of the diverse peptide repertoires presented by different HLA alleles. The peptide makes extensive contacts with the antigen-binding cleft and as a result each HLA allele imposes distinct sequence constraints and preferences on the presented peptides. A given peptide will thus only bind a limited number of HLAs, and reciprocally each allele only accommodates a particular fraction of the peptide collection from a given protein. The set of HLAI and HLAII alleles that is present in each individual is called the HLA haplotype. The polymorphism of HLAI and HLAII genes and the co-dominant expression of inherited alleles drives very large diversity of HLA haplotype across the human population, which when coupled to the enormous sequence diversity of αβ TCRs, presents high obstacles to standardisation of analysis of these HLA-antigen-TCR interactions.

### αβ TCR engagement of HLAI and HLAII

αβ TCRs recognize peptides as part of a mixed pHLA binding interface formed by residues of both the HLA and the peptide antigen (altered self). HLAI complexes are presented on the surface of nearly all nucleated cells and are generally considered to present peptides derived from endogenous proteins. T-cells can thus interrogate the endogenous cellular proteome of an HLAI-presenting cell by sampling pHLAI complexes of an interacting cell. Engagement of HLAI requires the expression of the TCR co-receptor CD8 by the interacting T-cell, thus HLAI sampling is restricted to CD8⁺ αβ T-cells. In contrast, the surface presentation of HLAII complexes is largely restricted to professional APC, and are generally considered to present peptides derived from proteins exogenous to the presenting cell. An interacting T-cell can therefore interrogate the proteome of the extracellular microenvironment in which the presenting cell resides. The engagement of HLAII requires the expression of the TCR co-receptor CD4 by the interacting T-cell, thus HLAII sampling is restricted to CD4⁺ αβ T-cells.

### Thymic selection of αβ TCRs

The role of αβ TCRs as described above is the detection of pHLA complexes, such that the TCR-presenting T-cell can raise responses germane to the role of that T-cell in establishing immunity. It should be considered that the αβ TCR repertoire generated within an individual must account for the immense and unforeseen diversity of all foreign antigens likely to be encountered in the context of a specific haplotype and prior to their actual occurrence. This outcome is achieved on a background where extremely diverse and numerous αβ TCRs are generated in a quasi-randomised manner with the potential to recognise unspecified pHLA complexes while only being specifically instructed to avoid strong interactions with self pHLA. This is carefully orchestrated during T-cell maturation in a process call thymic selection.

During the first step of T-cell maturation in the thymus, T-cells bearing αβ TCRs that are incapable of interacting with self-pHLA complexes with sufficient affinity, are deprived of a survival signal and eliminated. This step called positive selection assures that the surviving T-cells carry a TCR repertoire that is at least potentially capable of recognizing foreign or altered peptides presented in the right HLA context. Subsequently, αβ TCR that strongly interact with self-pHLA and thus have the potential to drive autoimmunity are actively removed through a process of negative selection. This combination of positive and negative selection results in only T-cells bearing αβ TCRs with low affinity for self-pHLA populating the periphery. This establishes an *αβ* T-cell repertoire that is self-restricted but not self-reactive. This highly individualised nature of T-cell genesis against HLA haplotype underscores the challenges in standardised analysis αβ TCR-antigen-HLA interactions. Moreover, it forms the basis of both graft rejection and graft versus host disease and the general principle that αβ TCRs identified in one individual may have completely different effect in a second individual, which has clear implications for TCR-based and T-cell based therapeutic and diagnostic strategies emerging in clinical practice.

### Unconventional αβ T-cells

The non-HLA-restricted, or 'unconventional', forms of αβ T-cells have very different molecular antigen targets. These unconventional αβ T-cells do not engage classical HLA complexes, but rather engage conserved HLA-like proteins such as the CD1 family or MR1. The CD1 family comprises four forms involved in antigen cross-presentation (CD1a,b,c and d). These cell surface complexes have an α-chain resembling HLAI, which forms heterodimers with β2-M. A small hydrophobic pocket presented at the membrane distal surface of the α-chain forms a binding site for pathogen-derived lipid-based antigens. Innate like NK T-cells (iNK T-cells) form the best-understood example of lipid/CD1 family recognition with GEM T-cells representing another prominent example. 'Type I' iNK T-cells are known to interact strongly with the lipid α-GalCer in the context of CD1d. These iNK T-cells display very limited TCR diversity with a fixed TCR α-chain (Vα10/Jα18) and a limited number of β-chains (with restricted vβ usage) and they have been likened to innate pathogen-associated molecular patterns (PAMPS) recognition receptors such as Toll-like and Nod-like receptors. In contrast, 'type II' NK T-cells present a more diverse TCR repertoire, and appear to have a more diverse mode of CD1d-lipid complex engagement. GEM T-cells recognize mycobacteria-derived glycolipids presented by CD1b, however, the molecular details of antigen presentation by CD1a, b and c as well as their T-cell recognition are only beginning to be understood.

MAIT cells largely express an invariant TCR α-chain (TRAV1-2 ligated to TRAJ33, TRAJ20, or TRAJ12), which is capable of pairing with an array of TCR β-chains. Instead of peptides or lipids MAIT TCRs can bind pathogen-derived folate- and riboflavin-based metabolites presented by the HLAI-like molecule, MR1. The limited but significant diversity in the TCRs observed on MAIT TCRs appear to enable the recognition of diverse but related metabolites in the context of the conserved MR1.

It is not well-understood how non-classical HLA-restricted αβ T-cell TCRs are selected in the thymus during maturation. However, it appears likely that the fundamental process of negative and positive selection outlined above still applies and some evidence suggests that this occurs in specialized niches within the thymus.

### γδ T-cells

In contrast to the detailed mechanistic understanding of αβ TCR genesis and pHLA engagement, relatively little is known about the antigen targets and context of their γδ T-cell counterparts. This is in part due to their relatively low abundance in the circulating T-cell compartment. However, it is broadly considered that γδ T-cells are not strictly HLA restricted and appear to recognize surface antigen more freely not unlike antibodies. Additionally, more recently it has become appreciated that γδ T-cells can dominate the resident T-cell compartment of epithelial tissues, the main interaction site of the immune system with foreign antigen. In addition, various mechanisms for γδ T-cell tumour immunuosurveillance and surveillance of other forms of dysregulated-self are beginning to emerge in the literature. The specific antigen targets of both innate-like and adaptive γδ T-cells remain poorly defined but the tissue distribution and fast recognition of PAMPs suggests a fundamental role for γδ T-cells both early in responses to foreign antigens as well as early in life when the adaptive immune system is still maturing.

The diverse functions of γδ T-cells appear to be based on different Vγ Vδ gene segment usage and can be broadly understood in two main categories in which γδ T-cells with largely invariant TCRs mediate innate-like recognition of PAMPs very early during infection. Beyond PAMPs these type of γδ T-cells are furthermore believed to recognize self-molecules, including phosphoantigens that could provide very early signatures of cellular stress, infection and potentially neoplastic development. Recognition of PAMPs and such so-called danger associated molecular patterns (DAMPS) as well as the large numbers of tissue-restricted innate-like γδ T-cells strongly suggests that these cells are suited to respond rapidly to antigenic challenge without the need for prior activation, homing and clonal expansion.

A second form of γδ T-cells are considered to be more adaptive in nature, with a highly diverse γδ TCR repertoire and the ability to peripherally circulate and access lymphoid tissues directly. Such antigen-specific γδ T-cells to common human pathogens such as CMV have been described and they appear to form a memory response. However, it has also been observed that γδ T-cells show only relatively limited clonal proliferation after activation and little data is available on the extent of TCR diversity and specific responses of γδ T-cells in peripheral circulation, or in tissues. Furthermore, while it is generally considered that γδ TCRs do not interact with pHLA complexes, and thus do not engage with peptide antigens in this context only few antigen targets of γδ T-cells have been characterised and the underlying molecular framework is only poorly understood.

The low frequency of peripheral γδ T-cells and the difficulty to study tissue-resident T-cells in humans has limited our knowledge of how this important and diverse type of T-cells participate in adaptive immune responses. This emerging area of research would require more reliable technologies with which to capture and characterise rare γδ T-cells, isolate their TCR pairs, and to identify their cognate antigens.

### Antigens and Antigen-presenting cells

In the context of T-cells and TCRs, antigens may be defined as any molecule that may be engaged by a TCR and resulting in a signal being transduced within the T-cell. The most well characterised T-cell antigens are peptides presented in an HLAI and HLAII complex, and which are engaged by conventional αβ T-cells. However, in recent years it has become apparent that non-conventional αβ T-cells and γδ T-cells are able to engage a wide range of biomolecules as antigens, including lipids, lipopeptides, glyco-peptides, glycolipds and a range of metabolites and catabolites. In addition, it has emerged that γδ T-cells may be able to engage fully folded proteins directly in an antibody-like fashion. Therefore, the view of T-cell antigens being largely restricted to HLA-presented peptides has expanded over the past two decades to include almost any biomolecule. With this concept in mind, it is relevant to define what may be considered an antigen-presenting cell (APC).

As defined in the above sections, HLAI and HLAII have a disparate expression profiles across cell types. It is widely accepted that nearly all nucleated cells present HLAI complexes on the cell surface, and are thus competent to present peptide antigens for T-cell sampling. In contrast, HLAII has a restricted expression profile, and at least in steady state conditions is only expressed on the surface of cells that have a specialist role in antigen presentation, including dendritic cells (DC), macrophage and B-cells. These specialist cell types are often referred to as professional APC. For the purposes of this document, the term APC is used to describe any nucleated cell that is capable of presenting an antigen for sampling by αβ or γδ T-cells. Such antigens are not restricted to those presented as 'cargo' in specific antigen-presenting complexes such as HLA and HLA-like molecules, but may also include any cell-surface presented moiety that is able to engage a αβ or γδ TCR-bearing cell.

### Therapeutic use of TCRs

Adoptive transfer of primary T-cells was first trialled in a clinical setting in the early 1990s, starting with ex vivo expanded T-cells polarised towards viral antigens to confer viral immunity in immunocompromised patients. Similar approaches using primary T-cells expanded ex vivo against specific cancer antigens were soon after trialled in treatment of malignancies. One limitation in these early approaches that continues to be a challenge today is a lack of understanding of the nature and diversity of T-cells clashing with the need to finely-optimize their composition in the therapeutic product. At present, the use of ex vivo expanded primary T-cells has largely been abandoned by the pharmaceutical industry with the exception of a handful of initiatives using primary T-cells with specificity for viral antigens.

In recent years the ability to reliably introduce genetic material into primary human cells has seen a variety of experimental genetically modified T-cell therapeutics arise. Such therapeutic cell products aim to harness the power of T-cell responses and redirect T-cell specificity towards a disease-associated antigen target, for example, an antigen uniquely expressed by malignant cells. These have largely relied on the transfer of a chimeric antigen receptor (CAR) into recipient T-cells, rather than actual TCR chain pairs. A CAR represents a targeting moiety (most often a single-chain antibody element targeting a surface expressed protein of malignant cells) grafted to signal receptor elements such as the ζ-chain of the CD3 complex, to produce a synthetic chimeric receptor that mimics CD3-TCR function. These so-called CAR T-cell (CAR-T) products have met mixed success in clinical trials to date and despite their potential are not easy to translate beyond tumours with inherent unique molecular targets such as B-cell malignancies. Alternatively, the transfer of full-length TCR chain pair ORFs into T-cells is of emerging interest. Such TCR-engineered T-cell therapeutics are at present limited by challenging manufacturing processes, and like the CAR-T products, a dearth of validated antigen targets and targeting constructs. To date this has been focused on the use of αβ TCRs for recognition of peptide antigens presented by HLAI on malignant cells and a fundamental challenge of this approach is the need for antigens that are specific to malignant cells.

It has been considered that since the TCR-pHLA interaction is of relatively low-affinity, native TCRs are likely to be suboptimal for TCR-engineered T-cell therapies. Several approaches have been devised to affinity-mature TCRs in vitro, in much the same manner as single-chain antibody affinity maturation. These TCR affinity maturation approaches generally also utilise a single-chain formats, wherein the V-region of one chain is fused to V-region of another chain to make a single polypeptide construct. Such single polypeptides may then be used in phage- or yeast- display systems adapted from antibody engineering workflows, and passed through rounds of selection based on target binding. Two inherent limitations exist in such a single-chain TCR approach in terms of yielding functional TCR chain pairs. Firstly, the selection is based on binding affinity to the target. However, it has been well documented that TCR affinity does not always correlate to the strength or competency of TCR signalling output. Secondly, the selection of single-chain constructs based on affinity does not always translate to equivalent affinities once they are reconstituted as full-length receptors.

In a therapeutic context, there exists an additional and crucial limitation in affinity-matured TCR pairs. That is, considering their sequences have been altered, the resulting constructs by definition have no longer been subject to thymic selection, wherein TCRs that react strongly to self-antigens are deleted from the repertoire. Therefore, these modified TCRs carry an inherent risk of being auto-reactive, which is very difficult to rule out in vitro using current methods. For the same reason, any selected or engineered TCR for therapeutic application needs to be individualised. If TCRs are artificially engineered or native TCRs applied across individuals, cross-reactivities have to be ruled out on the basis of the HLA haplotype and presented peptide repertoire of each specific individual in order to avoid potentially catastrophic autoimmunity. This is due to the fact that thymic selection is conducted on a background of all available HLA molecules specific only to that given individual. The likelihood of such cross-reactivity is unclear. However, the ability of our TCR repertoire to recognize pHLA complexes of other individuals of the same species as foreign is a fundamental property of adaptive immunity and underpins graft rejection and graft versus host disease. Recent clinical trials using a matured TCR chain pair against the cancer-specific melanoma associated antigen (MAGE) highlighted the potential problem of bypassing thymic selection. When autologous T-cells harbouring the matured TCRs were infused back to two cancer patients, these patients rapidly developed a fatal heart disease. Subsequent studies determined that the MAGE-specific matured TCRs were cross-reactive with an HLAI-presented peptide from the heart protein titin. This strongly suggests that cross-reactivity is a distinct possibility in therapeutic use of TCRs.

A distinct avenue of utilising TCRs for therapeutic purposes is in their use as affinity reagents in much the same manner as antibody therapeutic substances. Single-chain TCR molecules have been trialled for delivery of conjugated drug substances to specific HLA-antigen expressing cell populations. Such an approach is generally considered safer than CAR-T or TCR engineered T-cell therapeutics, as administration of the drug substance may simply be withdrawn. However, the potential for cross-reactivity and off target effects that are difficult to predict remains a potential limitation in this setting.

### TCR repertoire detection in clinical diagnostics

In a related aspect, there is an emerging interest in using the detection of the abundance of specific TCR sequences for clinical diagnostic purposes. With the rise of deep-sequencing methods in particular, it is possible to capture the full TCR diversity within an individual globally and for matched αβ pairs in specific contexts. This potentially represents a means to diagnose specific conditions and disease states simply by detecting the abundance of expanded T-cell clones, as proxy readout for established immune response against a disease-associated antigen in the patient. However, such global approaches are currently limited to very strong immune responses with established clinical time-points and suffer from the underlying difficulty in identifying the specific antigen target of any particular TCR identified via sequencing.

### Therapeutic and diagnostic use of T-cell antigens

The fundamental strength of harnessing adaptive immune responses translates into a central technical challenge in that the exquisite specificity of the TCR-antigen interaction requires detailed knowledge of the antigens specifically associated with each pathogen, cancer cell or autoimmune disease. Furthermore, each antigen may be presented by a specific antigen presenting complex, or allele thereof, such that antigen discovery has be performed for each relevant HLA gene and allele. For several infectious diseases like HIV, influenza and CMV that are associated with strong adaptive immune responses and generally display conserved epitope response hierarchies, the most important epitopes have been mapped in context of some common HLA. Similarly, the fields of cancer, allergy and autoimmunity have seen increased and systematic efforts to map the associated T-cell antigens. However, these are challenging procedures and the efforts to systematically describe T-cell antigens associated with different clinical contexts are hindered by the absence of efficient, robust, fast and scalable protocols.

Specifically, cancer cells represent a challenging and important aspect as most of the peptides presented on the surface of malignant cells are self antigens or very similar to self antigens. Therefore, thymic selection will have deleted TCRs that could strongly recognize these peptides, while at the same time the tumour has evolved to evade immune recognition. This means that potent immune responses against established tumours are relatively rare and targets difficult to predict or discover. However, these responses do exist and, importantly, are generally associated with better outcome. The target of such responses, tumour-associated-antigens (TAA), will in most cases have distinguishing characteristics from self and be derived from proteins that are overex-pressed during cancer development, otherwise absent from the cell type at this stage of development or specifically altered through genetic mutation or post-translational modifications such as phosphorylation.

When available, the knowledge of such epitopes makes it possible to interrogate the associated T-cell response for fundamental discovery, diagnostic purposes and for example as a test of vaccine efficacy. Importantly, they also provide highly specific targets for T-cell tolerization in allergy and autoimmunity and, crucially, point towards valuable targets for specific immunotherapy and against malignant cells. Malignancies represent a particularly valuable target as the promise of cellular immunotherapies and the progress in the T-cell manipulations are slowed by a lack of validated target TAAs that go beyond the few cases where specific markers for the type of cancer happen to be available.

In the light of the potential of cellular therapy and lack of validated targets the identification of promising TCR antigens remains one of the most pressing bottlenecks of TCR-based immunotherapy, particularly in the effort to treat cancer.

### Technological aspects of TCR and T-cell antigen analyses

Overall, the development of TCR-based therapies is still in its early stages, and success has been limited. Diagnostic approaches, while of immense potential, have seldom been deployed into controlled clinical studies that aim to assess patient disease states or response to therapy. Underdeveloped techniques for the reliable capture of native TCR chain pairs, and the systematic analysis of TCR-antigen interactions at high-throughput and in a functional context of cell-cell communication, has been the main hurdle to the development of TCR-based therapies and diagnostics.

Deep sequencing approaches have led to an improved understanding of T-cell receptor diversity in heath and disease. However, these approaches have generally focused on short stretches spanning the CDR3 regions, mainly of the TCR β-chain. Most studies have ignored the contribution of the TCR α-chain, and few have sought to analyse paired αβ chains as well as the antigen specificity of TCRs determined to be of interest. Recent workflows using single cell encapsulation and genetic barcoding has enabled the pairing of native TCR αβ or γδ chain pairs and analysis of full-length sequences, however, such workflows remain experimental.

Isolated TCR chain pairs may be analysed in terms of antigen specificity in either biophysical or functional modes. Biophysical analysis requires the recombinant production of both the TCR as well as the analyte antigen in soluble form. In the case of HLA-restricted TCRs this would thus require the generation of all individual TCRs as well as the cognate pHLA complexes. This is technically highly challenging, slow and very low-throughput. Furthermore, such analysis would only provide interaction affinities, which are not well-correlated with functional characteristics in predictable ways.

Until recently, the detailed functional analysis of isolated TCR sequences in a cellular context has been limited to laborious protocols of transfection of analyte TCR chain pairs into primary T-cells or immortal T-cell lines, and detection of cellular responses by traditional flow cytometric analysis of cell activation, or detection of secreted factors from the transfected cells upon antigen challenge. In a recent publication by Guo et al, rapid cloning, expression, and functional characterization of paired TCR chains from single-cells was reported (Molecular Therapy - Methods and clinical development (2016) 3:15054). In this study, analyte human αβ TCR pairs were expressed in a reporter cell line that lacked αβ TCR expression, and which contained a green fluorescent protein (GFP) reporter system linked to the Nur77 promoter that is activated upon TCR stimulation. This system remains inefficient due to the lack of standardised TCR integration into the reporter cell line genome, and does not provide a systematic manner for cell-bound antigen challenge by an APC element.

Similar to workflows for identification of TCRs against known T-cell antigens, the de novo discovery of novel T-cell antigens in health and disease remains highly challenging. Most approaches remain biophysical in nature, and aim to produce candidate antigens that may be tested in immunisation protocols, or through identifying cognate TCRs as addressed above. Little or no standardisation exists in the field of T-cell antigen discovery, and the field is largely restricted to academic study.

With the accumulating interest in TCRs and their cognate in both therapeutic and diagnostic use, and the emergence of means to capture significant numbers of native TCR αβ and γδ chain pairs, there remains a lack of reliable high-throughput and standardised technologies for the systematic analysis of TCR-antigen interactions. Importantly, there is a lack of standardised systems for functional analysis of TCR chain pairs in the native context of cell-cell communication wherein both the TCR and antigen are presented by a viable cell. Moreover, there is a lack of systems that may achieve TCR candidate selection, and/or affinity maturation of TCR chain pairs, in the relevant context of cell-cell communication.

As described, there is currently a lack of standardised technologies for the high-throughput generation of cells with TCR pairs expressed in a native cellular context. It is highly desirable to possess a system in which full-length TCR pairs may be inserted as single copies into the genome of a TCR-presenting cell such that said TCRs are presented in a native CD3 cell-surface complex for analysis and selection. A CD3 complex-presented TCR pair assures that affinity analyses are reflective of the actual native TCR composition, which is not the case for single-chain TCR and other non-native TCR-display technology. Moreover, the presentation of TCR pairs in a CD3 complex on a viable cell is an absolute requirement for functional analysis of TCR pairs. Functional analysis, meaning analysis of TCR signalling output, is of critical importance in TCR selection and engineering workflows where signal output is the parameter that is generally of greatest importance for use in the context of cellular therapeutics, and is not well correlated with the affinity of a TCR with cognate antigen/HLA as is determined within other display platforms.

### Detailed description of the invention

The present invention addresses the above-mentioned needs. In particular the present invention relates to the construction and use of an engineered multicomponent cellular system used for discovery and characterisation of TCRs and T-cell antigens. The multi-component system, comprised of at least three components being, an engineered T-cell receptor (TCR) presenting cell (eTPC), two engineered genomic receiver sites and a matching genetic donor vector. The present invention is used for rapid, high-throughput generation of stable derivative cells that present full-length TCR pairs (eTPC-t). The eTPC contains at least two engineered genomic receiver sites. The site is used for rapid, stable integration of genetic material encoding TCR pairs, delivered via the matched genetic donor vector. The eTPC may also include an optional component, a TCR-stimulation response element, for in vitro detection of TCR stimulation. The multi-component system may be used to compile key TCR-centric elements of analytical systems in clinical immunodiagnostics. Furthermore, the present invention relates to the use of the said identified and characterised TCR pair sequences for production of immunotherapeutics and immunodiagnostics.

The present invention enables a highly standardised system for assembly of various analyte eTPC forms in a systemised manner. This standardisation and systemisation is achieved through highly defined and controllable genome integration of TCR chain ORF with matched donor vector / genomic receiver site subsystems. This controllable and predictable system provides significant efficiency to the process generating analytical eTPC populations expressing analyte TCR at their cell surface, reducing cycle time and costs for such a process, compared to previous systems that have relied on random integration using unguided genome integration and/or viral approaches. Moreover, the design of the system is partly to ensure controllable copy-number of integrated TCR ORF, usually a single copy of each chain, which permits tight control over expression levels of integrated surface-expressed TCR. This is an important aspect for analysis of relative affinity or signalling output of collections of TCR pairs. Moreover, the ability of single-copy integration of TCR ORF from a vector pool allows so-called 'shotgun integration', wherein each cell integrated with a donor vector may only receive a single ORF from a library of vectors, potentially encoding a diverse population of TCR ORF. This enables the conversion of TCR ORF libraries encoded in donor vectors, into eTPC libraries expressing a single desired analyte TCR pairs on the cell surface; essentially representing a cell-based array system akin to a bacteriophage or yeast display system. Such array systems can facilitate the identification of TCR sequences against specific antigens, and/or identify the antigen specificities contained within a library of TCR sequences by ensuring a single analyte TCR pair is expressed in a single cell that can be recovered for sequencing-based identification.

One further key aspect of an eTPC, is the lack of expression of antigen-presenting complexes. Generally, eTPC must be engineered to lack at least expression of HLA class I. This is an important feature, as it eliminates the possibility of self-presentation of antigens by the eTPC-t, to and eTPC-t expressed TCR. This will reduce the false-positive rate in detection of antigen-specific responses in workflows that utilise addition of exogenous free antigen. Moreover, this allows for mixed culture analytical workflows, where analyte APC cells presenting particular HLA, may be pre-mixed with eTPC-t, and exogenous antigen added directly to the mixed culture system without the possibility of eTPC-t self-presenting the analyte antigen. This greatly improves the flexibility and efficiency of the eTPC-t product.

The present invention relates to the provision of an engineered multi-component system the components of which are are used to prepare one or more analyte eTPC-t. These analyte eTPC-t are then combined with one or more analyte antigens (collectively the eTPC:Antigen system, eTPC:A) to obtain one or more outputs. The analyte antigen is provided by analyte antigen-presenting cells (APC) and/or as soluble or immobilised analyte antigen and/or presented on non-cell based particles (NCBP).

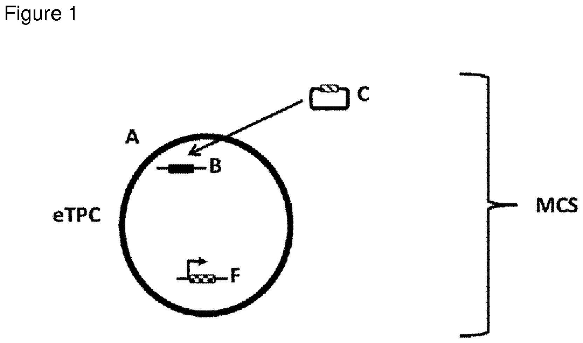

The minimal form of the multicomponent system comprises a first component **eTPC**, designated **component A,** and a second component is a genetic donor vector, **designated component C (****Figure 1****).**

An eTPC represents the base component of the multicomponent system, to which all other components of the system relate. Therefore, the eTPC contains certain features, that are native or engineered, that make the eTPC suitable for use to create analyte eTPC-t populations, and their use.

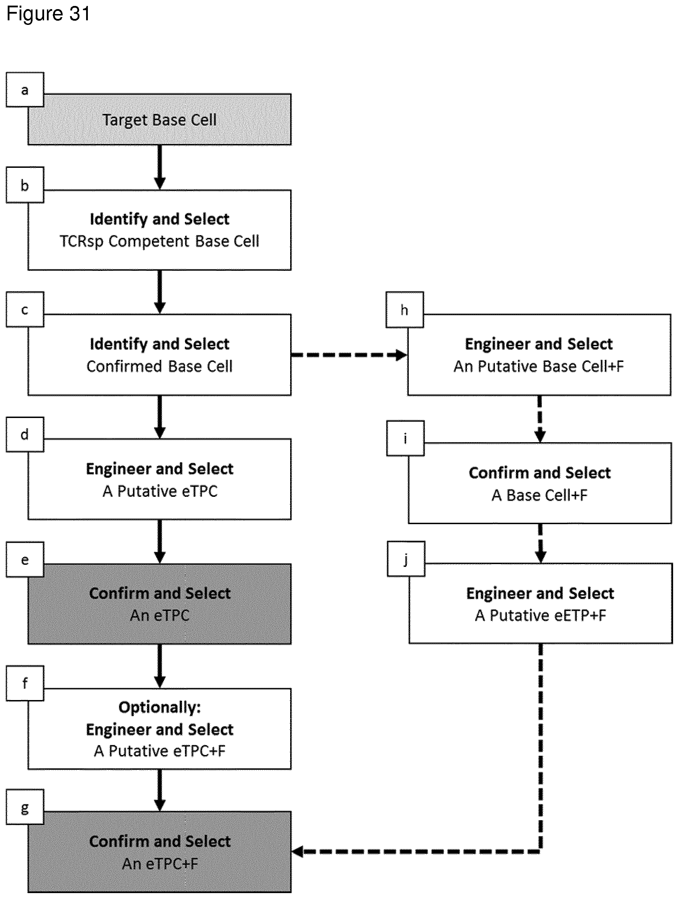

The eTPC, **component A,**
i. Lacks endogenous expression of TCR chains alpha, beta, delta and gamma, and
ii. Expresses CD3 proteins which are conditionally presented on the surface of the cell only when the cell expresses a complementary pair of TCR chains and
iii. Contains a further component **designated B and D** two genomic receiver sites for integration of a single ORF encoding at least one analyte TCR chain of alpha, beta, delta or gamma, and/or two ORFs encoding a pair of analyte TCR chains
wherein i) may be obtained by selection of a naturally occurring cell population lacking said expression, or may be engineered to lack such expression; ii) may by obtained by selection of a naturally occurring cell population comprising said expression, or may be engineered to comprise such expression; iii) may be or introduced to the genome by means of genetic engineering. Methods detailing the different means in which such a cell is obtained are presented in Figures 31 to 37.

The selection of an eTPC cell candidate that lacks TCR chains alpha, beta, delta and gamma from naturally occurring cell populations can be achieved by methods well known in the art. Staining of target cells with affinity reagents specifically for TCR chains alpha, beta, delta and gamma, and selection of cells TCR chains alpha, beta, delta and gamma may directly achieve this.

Engineering an eTPC to lack TCR chains alpha, beta, delta and gamma expression may be achieved by untargeted and targeted means. Untargeted mutagenesis of the cell can be achieved by providing a chemical, radiological or other mutagen to the cell, and then selecting cells lacking expression target TCR chains alpha, beta, delta and gamma expression. Targeted mutation of the genomic loci can be achieved via different means, including but not limited to site directed mutagenesis via
i. zinc-finger nucleases
ii. CRISPR/Cas9 mediated targeting
iii. Synthetic transcription activator-like effector nucleases (TALEN)
wherein said site-directed nucleases induce site-specific DNA-repair error mutagenesis at target loci, after which mutated cells are obtained by selecting cells lacking TCR alpha, beta, delta and gamma expression.

Options for Integration of CD3 and the components B and/or D are well known to those skilled in the art but may include homology directed recombination (HDR) and/or random integration methods, wherein HDR may be promoted by targeted mutation of the genomic loci at which HDR is to occur, and can be achieved via different means, including but not limited to site directed mutagenesis via
i. zinc-finger nucleases
ii. CRISPR/Cas9 mediated targeting
iii. Synthetic transcription activator-like effector nucleases (TALEN)
   wherein said site-directed nucleases induce site-specific DNA-repair by HDR at target loci. After such events, a proportion of cells will have incorporated HDR vector, an can be selected and/or determined via any combination of the following,
iv. Non-destructive phonotypical expression analysis
v. Destructive phonotypical expression analysis
vi. Genetic analysis

Wherein iv and vi are the preferred methods for selection and determination of successful genomic integration events.

Alternatively, viral vectors could be used to deliver the required components in a site-directed or undirected manner.

Considering that the eTPC component A is designed to be used in conjunction with the analyte antigens within analytical workflows, in the preferred aspect the eTPC contains features that minimise the eTPC presenting factors that would interfere in such analyses.

The eTPC component **A** optionally lacks endogenous surface expression of at least one family of analyte antigen presenting complexes **(aAPX)** and/or analyte antigenic molecules **(aAM),** wherein the lack of surface expression is selected as to minimise interference in matched analyses of target analyte antigens.

The family of **aAPX** may be any of the following
i. HLA class I
ii. HLA class II
iii. non-HLA antigen-presenting complex.

An **aAM** is selected from
i. a polypeptide or complex of polypeptides translated from the analyte antigenic molecule ORF(s)
ii. a peptide derived from a polypeptide translated from the analyte antigenic molecule ORF(s)
iii. a peptide derived from altering the component A proteome
iv. a polypeptide derived from altering the component A proteome
v. a metabolite derived from altering the component A metabolome

The component A eTPC may optionally additionally include T-cell co-receptors, wherein such features permit robust or varying forms of communication of the analyte eTPC to the analyte APC, wherein the tuneable communication is relevant to identification or characterisation of specific analyte TCRsp and/or analyte antigens.

In the present context, the eTPC component **A** may optionally express CD4 and/or CD8, wherein expression of CD4 or CD8 restrict eTPC to engaging aAPX of type HLAII and HLAI, respectively.

The eTPC component **A** may optionally expresses CD28 and/or CD45, wherein CD28 and CD45 contribute to signal sensitivity through positive feed forward effects on signalling, whereas they may also contribute to signal specificity through negative feed back effects on signalling, as it relates to signalling though an expressed analyte TCRsp.

The component A eTPC may optionally additionally include introduced cell surface adhesion molecule components, or ablation of endogenous cell surface adhesion molecules, to promote the eTPC engagement with analyte APC and formation of the immunological synapse, or to avoid tight binding and formation of deleterious cell clustering within analytical workflows involving APC, respectively.

Such adhesion molecules that may be introduced as additional ORFs to component A, or genetically ablated from A, can be selected from the integrin family of adhesion proteins.

The second component of the minimal multicomponent is a genetic donor vector, **component C,** which is used for integration of at least one ORF encoding at least one analyte TCR chain **(****Figure 1****).**

Component **C** is a genetic donor vector that is coupled with the genomic receiver site of the, **B**, contained within the genome of the eTPC, **Component A. Component C is** designed for the integration of one or more ORFs encoding an analyte TCR chain, encoded in the genetic donor vector, into the genomic receiver site, **B**, wherein integration results in the expression of analyte TCR chains by the target eTPC.

A paired genetic donor vector and genomic receiver site is described as an integration couple.

An eTPC is designed to respond to stimulation by cognate antigen, within analytical workflows using the eTPC:A system. It is thus desirable to have a standardised reporter readout for signalling response from stimulation of the expressed TCRsp.

The eTPC component **A**, further contains a component **designated component F,** a synthetic genomic TCR-stimulation response element selected from
i. A single component synthetic construct containing at least one native promoter and/or at least one synthetic promoter and at least one reporter
ii. A multi-component synthetic construct designed with at least one native promoter and/or at least one synthetic promoter and at least one reporter
wherein activation of i and/or ii is dependent on at least one signal transduction pathway selected from a synthetic pathway, a native pathway or a combination thereof.

A eTPC is designed to assay engagement of the presented TCRsp with a analyte antigen. The readout of engagement may be achieved through induction of a synthetic reporter element that responds to TCRsp engagement. Therefore, the eTPC, may further contain a component **designated F,** a synthetic genomic TCR-stimulation response element selected from
iii. A single component synthetic construct containing at least one native promoter and/or at least one synthetic promoter and at least one reporter
iv. A multi-component synthetic construct designed with at least one native promoter and/or at least one synthetic promoter and at least one reporter
wherein activation of i and/or ii is dependent on at least one signal transduction pathway selected from a synthetic pathway, a native pathway or a combination thereof.

Synthetic TCR-stimulation response elements **(component F)**, with synthetic as opposed to native promoters, are less likely to mimic a natural TCR stimulation response and thus represent a more distant approximation to natural T-cell stimulation. However, synthetic promoters provide greater flexibility for tuning and adjustment of the reporter response for the robust identification of eTPC-t presenting TCRsp that productively engage analyte antigen.

Single component synthetic constructs provide limited space for amplification or modulation of the signal reporter response, but are more straightforward to implement and predict the outcome. Multi-component constructs have the advantage of having a virtually unlimited space to construct complex response networks, however, this comes with the cost of being more difficult to implement and predict. A network also provides instances to initiate feedback loops, repression and activation of secondary response reporters.

Synthetic promoters can be linked to artificial synthetic signalling components, which can be either downstream of the initial natural TCR signalling pathway or substitute it. In such an instance, the CD3 complex can be coupled in-part or wholly to an artificial synthetic signalling pathway and response network. Such artificial pathways provide the advantage of being very modular and adaptable, for greater fine tuning or increased variety of responses.

The preferred embodiment utilises a multi-component synthetic construct with synthetic promoters and at least a partial synthetic signalling pathway. This provides the most robust means to ensure limited resting state signal response but with high coupled reporter signal when ligation of analyte antigen to the TCRsp occurs.

Regardless of the exact architecture of coupling the TCRsp stimulation to a component F the desired end outcome is a detectable reporter. In the preferred embodiment, a reporter that is amenable to fluorescent detection (i.e. FACS) and/or physical selection methods such as Magnetic Activated Cell Soting (MACS) is desired. Alternatively, the reporter could be a secreted or intracellular molecule for detection by spectrometric, fluorescent or luminescent assays, preferably in a non-destructive nature, wherein the populations can subsequently be selected based on the presence or absence of the reporter molecule. In addition, the response is not limited to a single reporter type but could be a combination of one or more different reporters.

In one context, a multi-component synthetic construct with synthetic promoters and a partial synthetic signalling pathway (Driver-Activator/Repressor + Amplifier-Reporter) would comprise of:
Driver-Activator/Repressor:
   a) a synthetic promoter
   b) a Kozak sequence
   c) a transcription factor,
   d) a first terminator
Amplifier-Reporter
   e) a second synthetic promoter
   f) a Kozak sequence
   g) a reporter
   h) a second terminator
wherein; a) Represents a synthetic promoter (Driver) that is designed to be coupled to the initial natural signalling pathway generated by TCRsp ligation to the analyte antigen. A minimum driver comprises of one or more transcription factor binding site and a core promoter, wherein the core promoter comprises, at a minimum, of a TATA Box, Initiator element (Inr) and transcriptional start site;b) Represents a Kozak sequence for efficient translational initiation of the transcription factor;c) Represents an ORF encoding a synthetic or natural transcription factor (Activator, or Repressor), which can bind to the DNA sequence of the second synthetic promoter;d) Represents a transcriptional terminator for efficient transcription and polyadenylation of the transcription factor mRNA transcript, and optional one or more untranslated 3' genetic element(s). These genetic elements may include, but are not limited to, transcript stabilizing or destabilizing elements, and unique identifier sequences;e) Represents a second synthetic promoter (Amplifier), which encodes one or more recognition sites for binding of the c) transcription factor and a core promoter, wherein the core promoter comprisess, at a minimum, of a TATA Box, Inr and transcriptional start site; f) Represents a Kozak sequence for efficient translational initiation of the reporter; g) Represents an ORF encoding a reporter protein; h) Represents a transcriptional terminator for efficient transcription and polyadenylation of the reporter mRNA transcript, and optional one or more untranslated 3' genetic element(s). These genetic elements may include, but are not limited to, transcript stabilizing or destabilizing elements, and unique identifier sequences.

It is important to recognise that the Driver-Activator/Repressor coupled to Amplifier-Reporter paradigm can be extended to include additional Driver-Activator/Repressor and Amplifier-Reporter pairs and/or additional Amplifier-Reporter units. Furthermore, Activators can be replaced with Repressors to shutdown Amplifier-Reporter units and/or Driver-Activator/Repressor units, for negative selection methods and/or negative-feedback loops.

In a second context, a single-component synthetic construct with synthetic promoters (Driver-Reporter) would comprise of:
a) a synthetic promoter
b) a Kozak sequence
c) a reporter
d) a terminator
wherein;
a) Represents a synthetic promoter (Driver) that is designed to be coupled to the initial natural signalling pathway generated by TCRsp ligation to the analyte antigen. A minimum driver comprises of one or more transcription factor binding site and a core promoter, wherein the core promoter comprises, at a minimum, of a TATA Box, Inr and transcriptional start site; b) Represents a Kozak sequence for efficient translational initiation of the reporter; c) Represents an ORF encoding a reporter protein; d) Represents a transcriptional terminator for efficient transcription and polyadenylation of the reporter mRNA transcript, and optional one or more untranslated 3' genetic element(s). These genetic elements may include, but are not limited to, transcript stabilizing or destabilizing elements, and unique identifier sequences

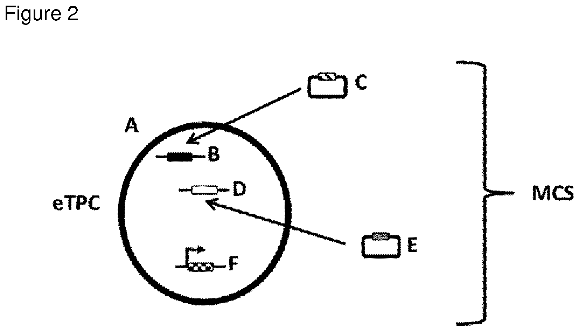

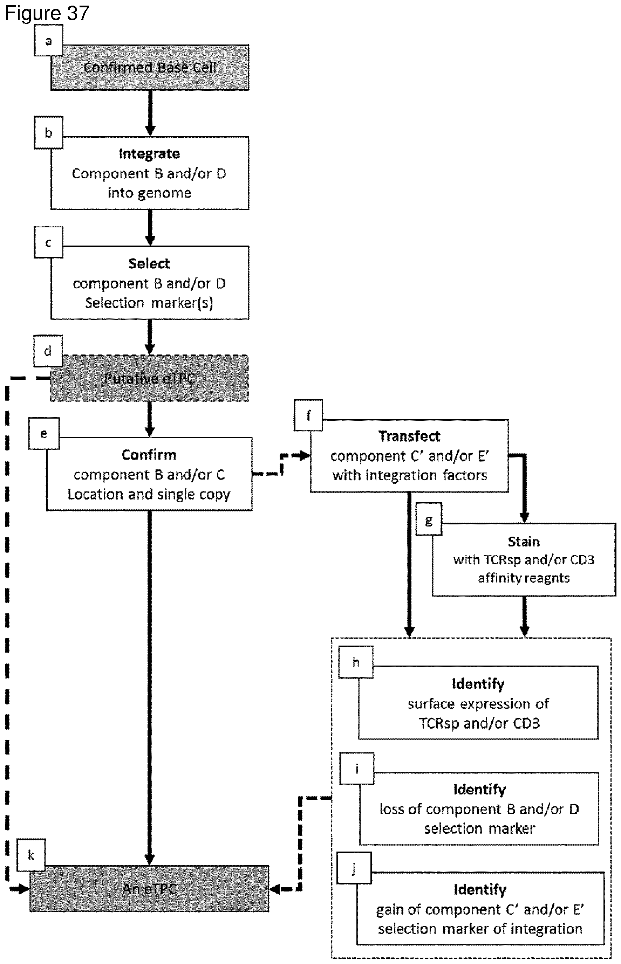

The component **A** eTPC further contains a second genomic receiver site, designated component **D**, which is coupled to a second genomic donor vector, designated component **E**, that is also added to the system **(****Figure 2****).** Methods detailing the different means in which such a cell is obtained are presented in Figures 37 to 38.

The multicomponent system component A may further comprise one or more additional integration couples.

The multicomponent system, comprising an eTPC and either one or two integration couples, is used for preparation of the eTPC-t used within analytical or preparative workflows.

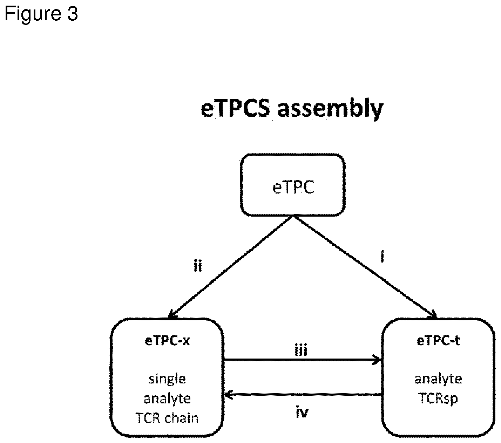

An eTPC-t may be prepared by integration of two complementary TCR chains to form a TCRsp, or may alternatively may be prepared by two steps by providing each complementary chain sequentially **(****Figure 3****).**

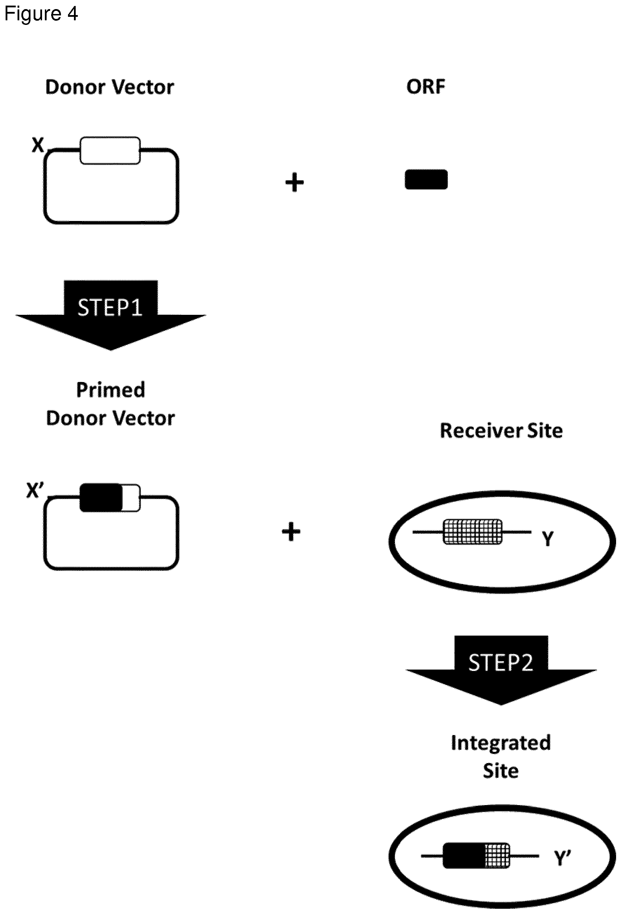

The genetic donor vector and genomic receiver sites operate as an integration couple subsystem of the multicomponent system. A genetic donor vector must first be combined with target ORFs, such that base donor vector now encodes those target ORFs. The assembled primed donor vector is then introduced to the target eTPC to exchange target ORF(s) to the genomic receiver site, thus integrating the target ORFs to coupled receiver site of the target cell **(****Figure 4****).**

A multicomponent system that comprises genetic donor vectors component C and/or E is combined with at least one ORF encoding at least one analyte TCR chain to obtain component C' and/or E', wherein the combination is defined as the ligation of genetic material into the correct coding frame(s), and in the correct orientation(s), of the genetic donor vector.

The combination of one or more ORFs into genetic donor vectors C and/or E may be performed multiple times with a library of unique ORFs as
i. single discrete reactions to obtain a discrete library of C' and/or E' vectors encoding multiple ORFs
ii. a single reaction to obtain a pooled library of C' and/or E' vectors encoding multiple ORFs
wherein a discrete library may be combined with component A multiple times as to obtain a discrete library of eTPC-t with unique ORFs encoding unique TCR chains, or a pooled library may be combined with component A one or more times as to obtain a one or more pooled library of eTPC-t wherein each eTPC-t integrates a randomised one or more ORFs encoding analyte TCR chains derived from the original vector pool, such that each eTPC-t expresses a single random unique TCRsp.

The efficient integration of a predictable copy number of one or more ORFs into the genomic receiver site is highly advantageous for operation of a standardised eTPC, where analyte eTPC populations may be rapidly prepared and characterised. Thus, the genomic receiver site(s) and coupled donor vector(s) are critical to the function of the eTPC. Furthermore, it is strongly desirable to have an eTPC wherein component B and D, are insulated from one another, such that the donor vector component C cannot integrate at component B, and vice versa. In addition, it is also desirable that the component B and/or component D are amenable to a method of preparation of an eTPC-t wherein, the introduction of a single pair of complementary TCR chains is rapid, repeatable, with a high likelihood of correct integration and delivery of only a single pair.

The genomic receiver site may be selected from the following
i. A synthetic construct designed for recombinase mediated cassette exchange (RMCE)
ii. A synthetic construct designed for site directed homologous recombination wherein i) is the preferred form a genomic receiver site for RCME. The RMCE method may employ selected heterospecific sites that are specific for individual recombinase enzymes, such that each component B and D possess insulated specificity.

The genomic receiver site, component B and/or component D comprises of at least one of the following genetic elements
i. Heterospecific recombinase sites
ii. Homologous arms
iii. Eukaryotic promoter
iv. Eukaryotic conditional regulatory element
v. Eukaryotic terminator
vi. Selection marker
vii. Splice acceptor site
viii. Splice donor site
ix. Non-protein coding gene
x. Insulator
xi. Mobile genetic element
xii. Meganuclease recognition site
xiii. Internal ribosome entry site (IRES)
xiv. Viral self-cleaving peptide element
xv. A kozak consensus sequence

A preferred genomic receiver site would comprise of two different arrangements using the following selected elements from the previously stated list of element.

The first arrangement is for receiving a single ORF encoding one or more TCR chains and/or a selection mark of integration, via RMCE integration wherein the arrangement is

5' -[A] [B] [C] [D] [E] [F]- 3'

wherein
A) is element iii) a constitutive or inducible Eukaryotic promoter
B) is element i) heterospecific recombinase site 1
C) is element xv) a Kozak consensus sequence
D) is element vi) a FACS and/or MACS compatible encoded protein marker
E) is element i) heterospecific recombinase site 2
F) is element v) Eukaryotic terminator

The second arrangement is for receiving a two ORF encoding one or more TCR chains and/or a selection mark of integration, via RMCE integration wherein the arrangement is
5' -[A] [B] [C] [D] [E] [F] [G] [H] [I]- 3' wherein
A) is element iii) a constitutive or inducible Eukaryotic promoter
B) is element i) heterospecific recombinase site 1
C) is element xv) a Kozak consensus sequence
D) is element vi) a FACS and/or MACS compatible encoded protein marker 1
E) is element v) a Eukaryotic bidirectional transcriptional terminator
F) is element vi) a FACS and/or MACS compatible encoded protein marker 2
G) is element xv) a Kozak consensus sequence
H) is element i) heterospecific recombinase site 2
I) is element iii) a constitutive or inducible Eukaryotic promoter furthermore, in this second arrangement the elements F, G, and I are encoded in the antisense direction.

**Component C and/or E** comprises of at least one of the following genetic elements
i. Heterospecific recombinase sites
ii. Homologous arms
iii. Eukaryotic promoter
iv. Eukaryotic conditional regulatory element
v. Eukaryotic terminator
vi. Selection marker
vii. Splice acceptor site
viii. Splice donor site
ix. Non-protein coding gene
x. Insulator
xi. Mobile genetic element
xii. Meganuclease recognition site
xiii. Internal ribosome entry site (IRES)
xiv. Viral self-cleaving peptide element
xv. A kozak consensus sequence
xvi. Selection marker of integration
xvii. An antibiotic resistance cassette
xviii. A bacterial origin of replication
xix. A yeast origin of replication
xx. A cloning site

A preferred genetic donor vector, component C and/or component E, would comprise of two different possible arrangements using the following selected elements from the previously stated list of elements.

The first arrangement is for delivery of a single ORF encoding one or more TCR chains and/or a selection mark of integration, via RMCE integration wherein the arrangement is
5' - [A] [B] [C] [D] [E] - 3' wherein
A) is element i) heterospecific recombinase site 1
B) is element xv) a Kozak consensus sequence
C) is element xx) a cloning site of a single ORF encoding one or more TCR chains and/or element xvi) a selection marker of integration
D) is element i) heterospecific recombinase site 2
E) is element xvii) An antibiotic resistance cassette and element xviii) a bacterial origin of replication, in no specific orientation
furthermore, the elements viii and/or xiv may be used to link multiple TCR chains and/or element xvi together.

The second arrangement is for delivery of two ORF encoding one or more TCR chains and/or a selection mark of integration, via RMCE integration wherein the arrangement is

5' - [A] [B] [C] [D] [E] [F]- 3'

wherein
A) is element i) heterospecific recombinase site 1
B) is element xv) a Kozak consensus sequence
C) is element xx) a cloning site for introduction of two or more ORF, with eukaryotic terminators, encoding one or more TCR chains and/or element xvi) a selection marker of integration
D) is element xv) a Kozak consensus sequence (antisense direction)
E) is element i) heterospecific recombinase site 2
F) is element xvii) An antibiotic resistance cassette and element xviii) a bacterial origin of replication, in no specific orientation
furthermore, the elements viii and/or xiv may be used to link multiple TCR chains and/or element xvi together within each ORF.

### Preparing analyte eTPC-t populations

The above described multicomponent system may be used in multiple ways to prepare distinct forms of analyte eTPC populations, or libraries thereof, that serve to present analyte TCRsp to analyte antigen within analytical or preparative workflows of the eTPC:A system.

The eTPC-t populations that are created need to derive analyte TCR chains from certain sources with which to analyse candidate antigens.

The sources of analyte TCR chain encoding sequences can be derived from
i. Paired cloning of TCR chain ORF sequence(s) from primary T-cells
ii. Unpaired cloning of TCR chain ORF sequence(s) from primary T-cells
iii. Synthetic TCR chain ORF sequence(s)
wherein i) is preferable for discovery of native TCRsp that are not likely to be generally cross reactive against self aAPX and/or the aAPX cargo due to thymic selection; ii) may be used to identify candidate TCR affinity reagents; iii) may be used in affinity maturation of TCR affinity reagents.

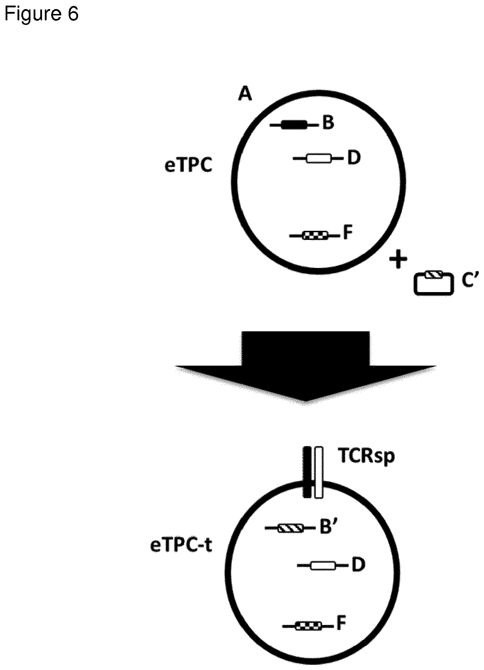

A multicomponent system comprising two integration couples may be used to prepare an eTPC-t from component A in one step, by providing component C' combined with an ORF for complementary pair of TCR chains, such that this analyte TCR pair is integrated to site B, to create B'. The resulting cell line expresses the provided TCR pair, and it is presented at the cell surface as a TCRsp. The second integration couple D/E remains unmodified and may be used for downstream integration steps **(****Figure 6**).

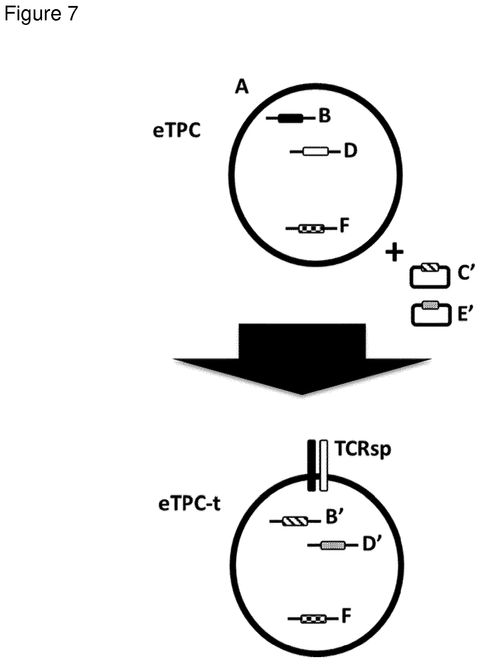

Aa multicomponent system comprising two integration couples may be used to prepare an eTPC-t from component A in one step, by providing component C' and E' each combined with one ORF encoding one chain of a complementary TCR chain pair, such that both analyte TCR chains are integrated to site B or D, to create B' and D'. The resulting cell line expresses the provided TCR pair, and it is presented at the cell surface as a TCRsp. **(****Figure 7****).**

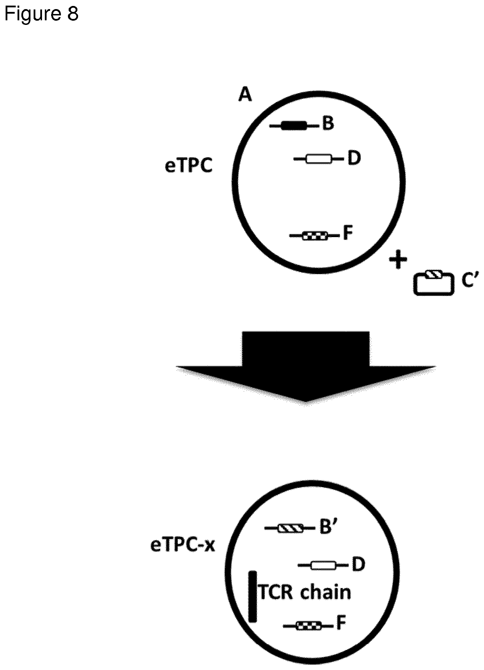

A multicomponent system comprising two integration couples may be used to prepare an eTPC-x from component A in one step, by providing component C' combined with an ORF for a single analyte TCR chain, such that this analyte TCR chain is integrated to site B, to create B'. The resulting cell line expresses the provided TCR chain, but lacks a complementary chain and thus does not express the any surface TCR. The second integration couple D/E remains unmodified and may be used for downstream integration steps **(****Figure 8**).

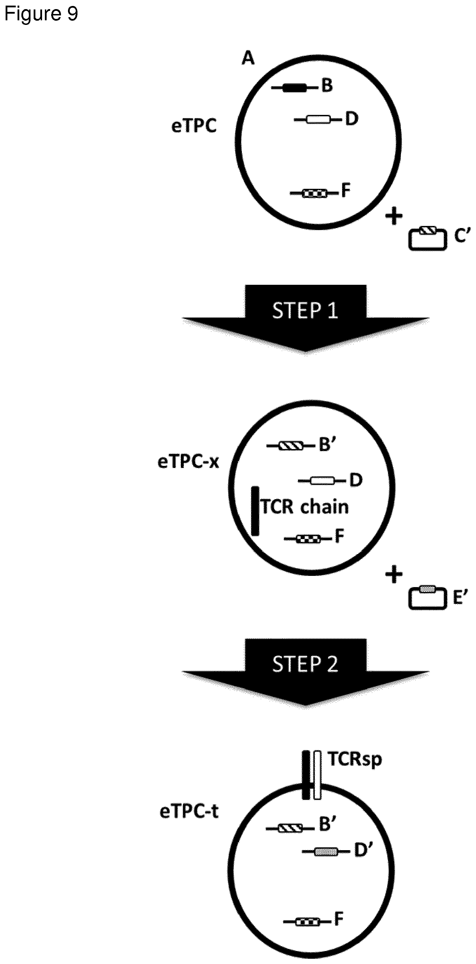

A multicomponent system comprising two integration couples may be used to prepare an eTPC-t from component A in two steps, by first providing component C' combined with an ORF for a single analyte TCR chain, such that this analyte TCR chain is integrated to site B, to create B'. The resulting cell line expresses the provided TCR chain, but lacks a complementary chain and thus does not express the any surface TCR (eTPC-x intermediate). The second integration couple D/E remains unmodified. In the second step E' is provided, wherein the vector is combined with an ORF for a second single analyte TCR chain, complementary to the previously integrated TCR chain, such that this second complementary analyte TCR chain is integrated to site D, to create D'. The resulting cell line expresses the provided complementary analyte TCR chain pair and it is presented at the cell surface as a TCRsp. **(****Figure 9**).

In the abovementioned examples of preparing analyte eTPC-x and/or eTPC-t populations from eTPC, the multicomponent system is used to provide known analyte TCR chains in a defined manner to prepare discrete populations of analyte eTPC expressing defined TCRsp. Such a process may be repeated many times to build libraries of eTCP-x and/or eTCP-t to provide to analytical or preparative workflows. An alternative approach is to take pooled libraries of analyte TCR chains combined with genetic donor vectors, and integrate these in a shotgun fashion to obtain pooled libraries of analyte eTPC-t, wherein each eTPC-t integrates a single random pair of TCR ORF from the original vector pool, and thus each eTPC-t expresses a single random TCRsp, but the collectively the pool encodes multiple species of TCRsp represented in the original vector pool. The same shotgun principle can be applied to create pools of eTPC-x. This is particularly useful when analysing large libraries of candidate TCRsp against analyte antigens.

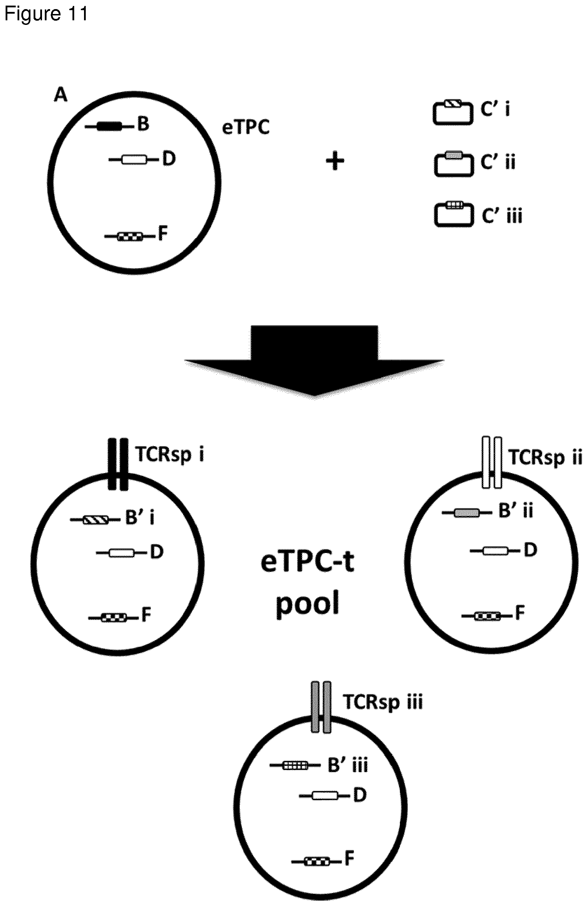

A multicomponent system comprising two integration couples may be used to prepare an eTPC-t pool from component A in one step, by providing component C' combined with a library of multiple ORF encoding a pool of analyte TCR pairs, such that a pair is integrated to site B, to create B', within each cell and wherein each eTPC integrates a single random pair of TCR ORF from the original vector pool, and thus each eTPC-t expresses a single random TCRsp, but the collectively the pool encodes multiple species of TCRsp represented in the original vector pool. The resulting pool of cells contains a collection of cells that collectively express multiple analyte TCRsp. The second integration couple D/E remains unmodified **(****Figure 11****).**

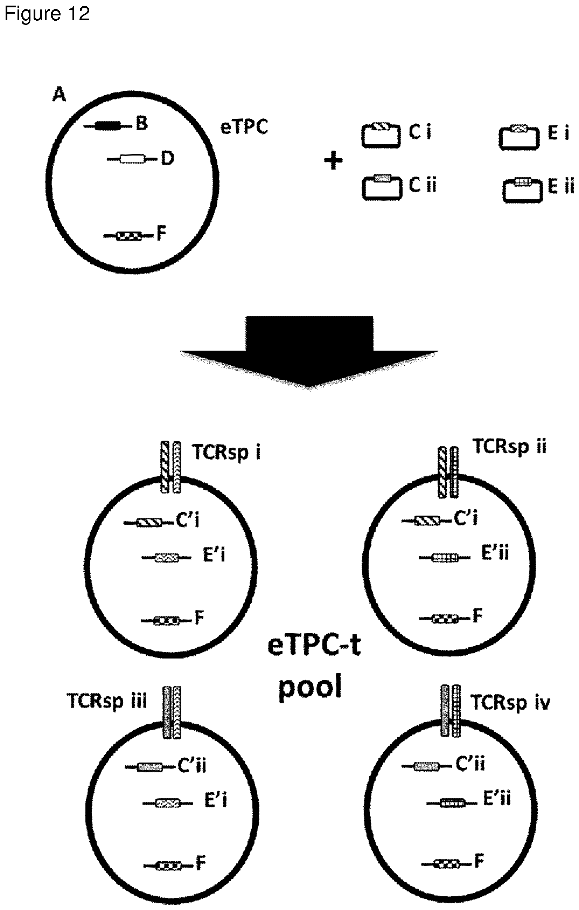

A multicomponent system comprising two integration couples may be used to prepare an eTPC-t pool from component A in one step, by providing component C' combined with a library of multiple ORF encoding a pool of single analyte TCR chains such that each eTPC integrates a single randomised TCR chain ORF encoded in component C' to site B, to create B'. Simultaneously, providing component E' combined with a library of multiple ORF encoding a pool of single analyte TCR chains complementary to first library provided in C', such that each eTPC-t integrates a single randomised complementary TCR chain ORF encoded in component E' into site D, to create D'. Each resulting cell in the eTPC-t pool has a single pair of randomised complementary TCR chain ORF, such that each cell in the pool expresses a randomised TCRsp. Such a pooled library would contain all possible combinations of provided complementary TCR chains from the sets proceed in components C' and E' **(****Figure 12****).**

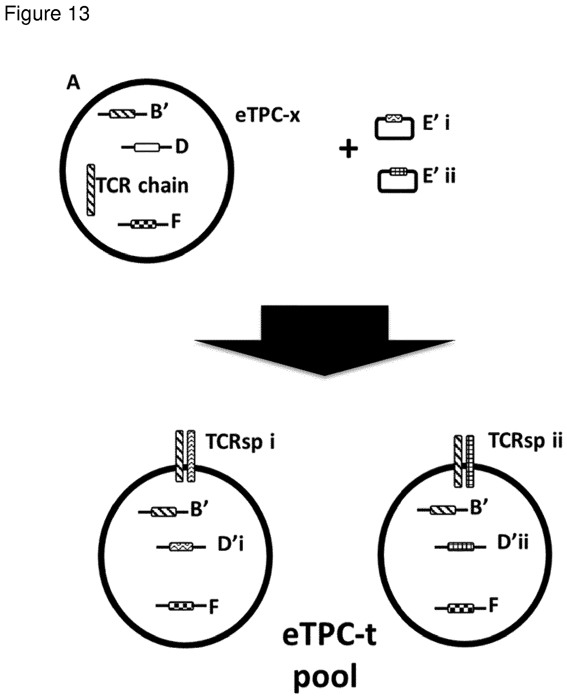

A multicomponent system comprising two integration couples may be used to prepare an eTPC-t pool from a previously obtained e-TPC-x in one step, wherein the site B has been converted to B' and contains the single analyte TCR chain. An eTPC-t is prepared by providing component E' combined with a library of multiple ORF encoding a pool of single analyte TCR chains complementary to the already integrated chain, such that each eTPC-t integrates a single randomised TCR chain ORF of the provided E' library in to site D, to create D'. Each resulting cell in the eTPC-t pool has the analyte TCR chain provided by the starting eTPC-x, and a selection of each single complementary analyte TCR chain, such that each cell in the pool expresses a random pair of ORF as an TCRsp. Such an approach is used when analysing the effect of varying a single chain against a fixed chain in a complementary TCR chain pair **(****Figure 13****).**

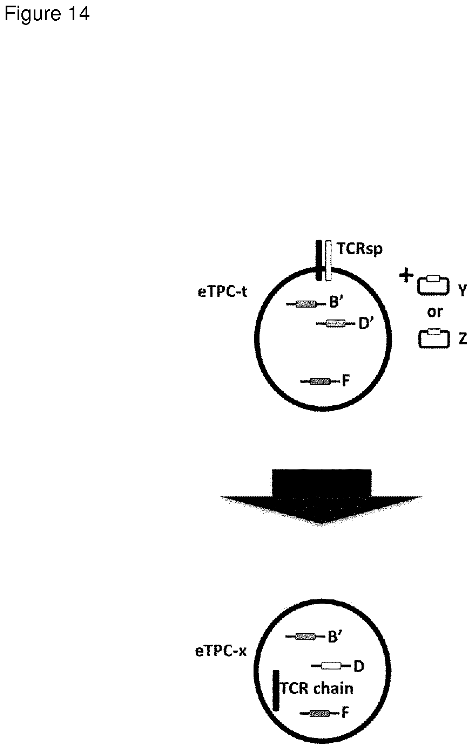

An eTPC-x may be prepared from an eTPC-t comprising two integration couples by providing either one of further integration vectors, **components Y or Z,** which encode markers of integration or no ORF. Combination of component Y or Z to an eTPC-t would exchange either of the sites to obtain a single TCR chain expressing eTPC-x **(****Figure 14****).**

A preferred genetic integration vector, **component Y and component Z,** for conversion of eTPC-t to eTPC-x would comprise the same integration vector requirements as components C and E above, though not encoding any TCR chain ORF, and preferably encoding a marker of integration.

An eTPC-x, or libraries thereof, can be used for transient transfection of a TCR chain ORF that is complementary to the integrated TCR chain ORF at B' or D' in the eTPC-x, in order to rapidly screen TCRsp derivatives in a target assay.

### Contacting analyte eTPC-t with analyte antigen

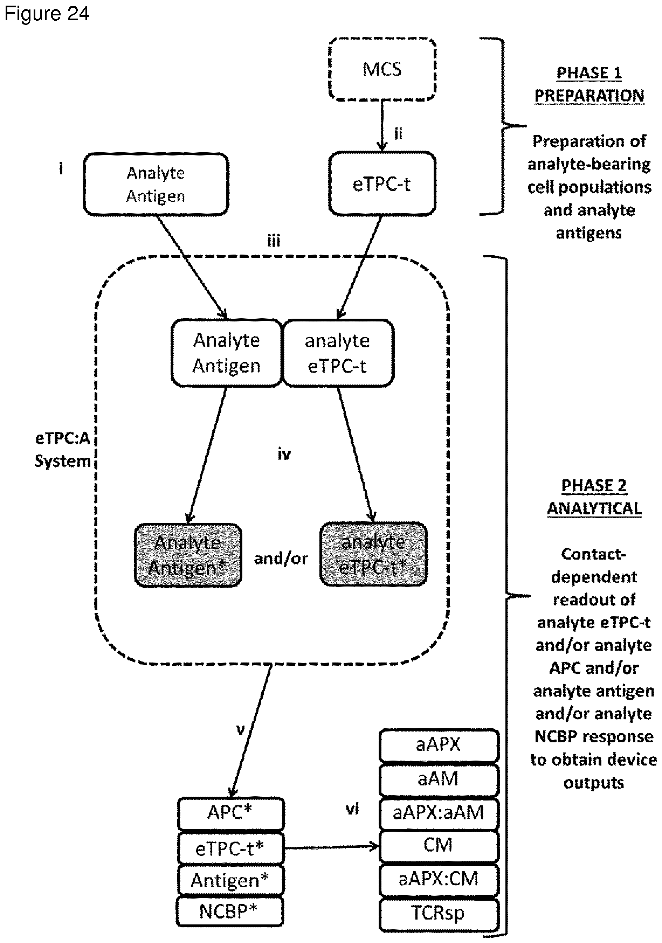

The present invention relates to the provision of an engineered multi-component system. The component A and one or more component C' and/or one or more Component E' are used to assemble one or more analyte eTPC-t. Analyte eTPC-t populations for compilation of analytical devices that are collectively termed eTPC:Antigen (eTPC:A) systems **(****Figure 24****).** Within the multicomponent system, one or more component C' and/or one or more component E' are combined with component A to generate various forms of analyte eTPC-t. These analyte eTPC-t are then combined with one or more analyte antigens within an analytical eTPC:A system to obtain one or more outputs. The analyte antigen is provided by analyte antigen-presenting cells (APC) and/or as soluble or immobilised affinity reagent and/or presented on non-cell based particles (NCBP).

An analyte antigen represents any entity that a TCR can putatively engage in the eTPC:A system, and may be represented by;
i. aAPX (analyte Antigen-presenting complex) and/or
ii. aAM (analyte antigenic molecule) and/or
iii. aAPX:aAM (analyte Antigen-presenting complex presenting an analyte antigenic molecule) and/or
iv. CM (a non-analyte cargo molecule) and/or
v. aAPX:CM (analyte Antigen-presenting complex presenting a cargo molecule) wherein an aAPX represents a complex that is able to present an aAM; an aAM is any molecule that is directly recognised by a TCR or when loaded in an aAPX; an aAPX:aAM is an aAPX with a loaded aAM; a CM is a cargo molecule that may be loaded in the aAPX, but which is not an analyte, thus may be derived from an analyte antigen presenting cell (APC) or the assay system itself; aAPX:CM is an aAPX with a CM loaded.

These forms of **analyte antigens** may be presented to the eTPC in different modes within an eTPC:A system, represented as;
i. an analyte antigen presenting cell (APC) and/or
ii. a soluble or immobilised affinity reagent and/or
iii. a non-cell based particle (NCBP),
wherein an analyte antigen presenting cell (APC) is considered any APC that is able to present an antigen to the eTPC-t; an affinity reagent is considered any reagent that is prepared as analyte to probe TCRsp binding and/or stimulation at the cell surface of the eTPC-t in an eTPC:A system. Such reagents will often represent analyte antigenic molecules (aAM), analyte antigen-presenting complexes (aAPX), or aAPX loaded with aAM (aAPX:aAM). A typical example of an aAPX:aAM is an pHLA-multimer reagent (e.g. 'tetramers') used to stain TCRs. Affinity reagents in this context could also represent antibodies or similar entities; a non-cell based particle (NCBP) acts in a similar manner to an affinity reagent, inasmuch that the particle presents an analyte antigen or other entity that is to be assessed for TCRsp engagement at the surface of a eTPC-t within and eTPC:A system. However, an NCBP is considered as a larger entity that can further carry genetic or other information that is to act as an identifier, either directly or by proxy, of the presented analyte antigen or other binding entity. A typical example of an NCBP would be a bacteriophage in a phage-display scenario, wherein phage may display antibody fragment antigen binding (FAB). Positively labelled eTPC-t may be recovered along with the phage, and sequenced to identify FABs specific for the TCRsp at the surface of a eTPC-t.

An analytical eTPC:A system is comprised of a selection of one or more of analyte antigen with one or more eTPC-t populations **(****Figure 24****).** The analyte eTPC-t populations are prepared using the multicomponent system as described above **(****Figures 1 to 14****).** The eTPC:A system is provided in a format that permits physical contact between the analyte antigens and analyte eTPC-t populations, wherein such contact is permissive of complex formation between one or more analyte antigen and TCRsp of one or more analyte eTPC-t, wherein the analyte antigen is any of the following
i. aAPX (analyte Antigen-presenting complex) and/or
ii. aAM (analyte antigenic molecule) and/or
iii. aAPX:aAM (analyte Antigen-presenting complex presenting a analyte antigenic molecule) and/or
iv. CM (a non-analyte cargo molecule) and/or
v. aAPX:CM (analyte Antigen-presenting complex presenting a cargo molecule)
wherein the analyte antigen is either provided as, presented by an analyte APC, or presented by a soluble and/or immobilised affinity reagent, or NCBP such that complex formation may lead to stabilisation of such a complex and wherein such complex formation leads to observable labelling of the eTPC-t and/or the induction of signalling within the analyte eTPC via component F, if included and/or an observable signal in the analyte APC, which may be measured.

In the present context, an eTPC:A system comprises of:
i. an input of a single analyte eTPC-t; or
ii. an input of a pooled library of analyte eTPC-t
and combined with one of the following:
iii. an input of a single analyte APC; or
iv. an input of a single analyte affinity reagent; or
v. an input of a single analyte NCBP; or
vi. an input of a pooled library of analyte APC; or
vii. an input of a pooled library of analyte affinity reagent; or
viii. an input of a pooled library of analyte NCBP

### Contacting in a buffer system

A contact between an analyte APC and analyte eTPC-t is performed in a permissive cell culture or buffer system, wherein said system comprises media that is permissive to the function of both analyte APC and analyte eTPC-t cells.

A contact between a soluble analyte affinity, immobilised affinity reagent and/or analyte NCBP and an analyte eTPC-t is performed in a permissive buffered system, wherein said system comprises a buffered medium that is permissive to function of both the analyte antigen and analyte eTPC-t cells.

### Labelling eTPC-t with affinity reagents or NCBP

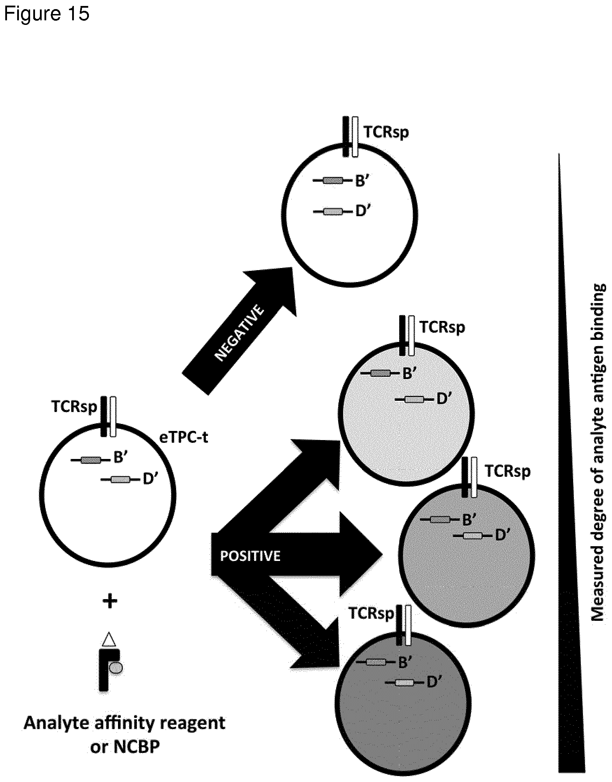

An analyte eTPC-t obtained from the two-part device may be used for characterisation of a TCRsp presented by the eTPC-t. Such characterisation may be conducted in a manner where the analyte eTPC-t is contacted with an immobilised or soluble affinity reagent or non-cell based particle (NCBP) in such a manner as to label the eTPC-t **(****Figure 15****)**.

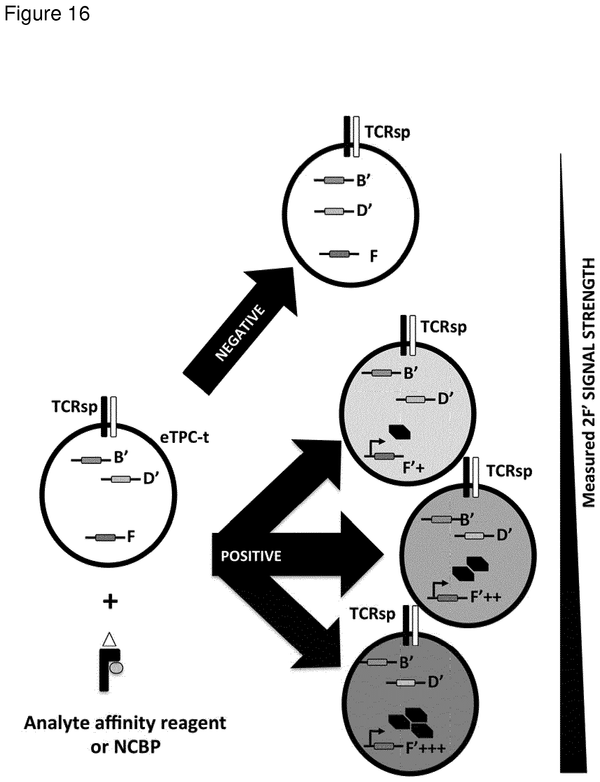

Labelling may be considered to be detected by direct observation of the label through such methods as flow cytometry, microscopy, spectrometry or luminometry or alternatively by means of capture with an immobilised affinity reagent or NCBP. In a similar manner, the affinity reagent or NCBP may stimulate the reporter element, component **F**, if included. Stimulation of component **F** would allow selection of eTPC-t and/or affinity reagent or NCBP for identification **(****Figure 16****).**

### Signal responses definition

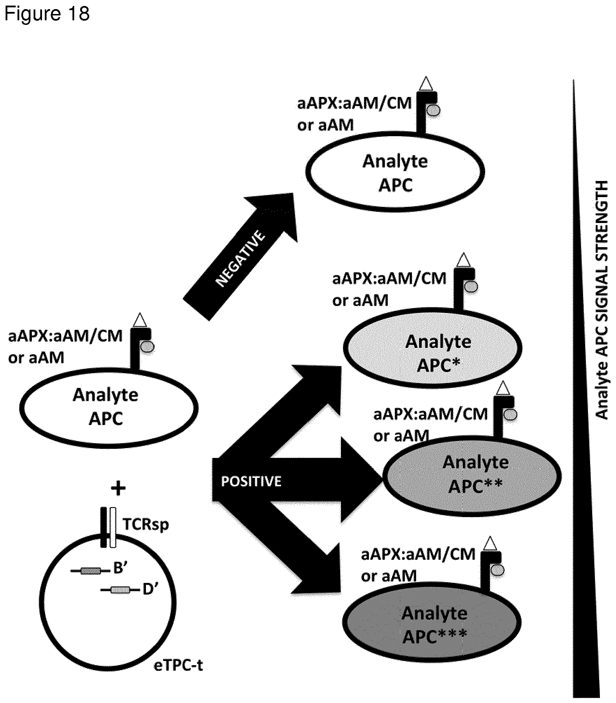

An analyte eTPC-t obtained from the two-part device is used for characterisation of a signal response of the analyte eTPC-t, expressing analyte **TCRsp,** to an **analyte antigen,** wherein such a signal response may be either binary or graduated, and may be measured as intrinsic to the eTPC-t **(****Figures 15****,** **16** **and** **17****)** and/or intrinsic to the APC, if included **(****Figure 18****).** Such signals may be detected through methods such as flow cytometry, microscopy, spectrometry or luminometry or other methods known to those skilled in the art.

### Contacting with an APC with signal responses

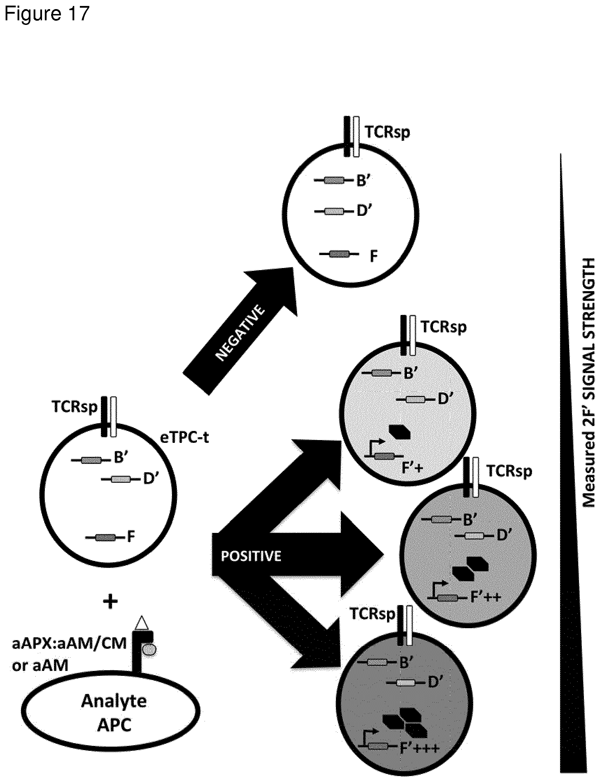

An analyte APC may also be contacted with the eTPC-t within an eTPC:A system. Generally, the response will be measured by reported signal within the eTPC-t **(****Figure 17****),** but may also be measured by reported signal within the APC **(****Figure 18****).**

### General Method - Selecting an eTPC-t

The method for selecting one or more analyte eTPC-t from an input analyte eTPC-t or a library of analyte eTPC-t, from the combined eTPC:A system, to obtain one or more analyte eTPC-t wherein the expressed TCRsp binds to one or more analyte antigen comprises
i. Combining one or more analyte eTPC-t with one or more analyte antigen resulting in a contact between an analyte TCRsp with an analyte antigen and at least one of
ii. Measuring a formation, if any, of a complex between one or more analyte TCRsp with one or more analyte antigen and/or
iii. Measuring a signal from a labelled analyte antigen and/or
iv. Measuring a signal response by the analyte eTPC-t, if any, induced by the formation of a complex between one or more analyte TCRsp with one or more analyte antigen and/or
v. Measuring a signal response by the analyte APC, if any, induced by the formation of a complex between one or more analyte TCRsp with one or more analyte antigen and
vi. Selecting one or more analyte eTPC-t based on step ii, iii, iv and/or v wherein the selection is made by a positive and/or negative measurement
wherein i, iv and vi or i, v and vi comprise the preferred arrangements.

### General Method - Selecting an analyte antigen

The method for selecting one or more analyte antigen from an input analyte antigen or a library of analyte antigen, to obtain one or more analyte antigen wherein the expressed analyte antigen binds to one or more analyte TCRsp presented by the analyte eTPC-t comprises
i. Combining one or more analyte antigen with one or more analyte eTPC-t, resulting in a contact between an analyte antigen presented by the analyte antigen with analyte TCRsp of one or more analyte eTPC-t and
ii. Measuring a formation, if any, of a complex between one or more analyte antigen with one or more analyte TCRsp and/or
iii. Measuring a signal from a labelled analyte antigen and/or
iv. Measuring a signal response in the one or more analyte eTPC-t, if any, induced by the formation of a complex between the analyte TCRsp with the analyte antigen and/or
v. Measuring a signal response, if any, by the analyte APC induced by the formation of a complex between one or more analyte TCRsp with one or more analyte antigen and
vi. Selecting one or more analyte antigen from step ii, iii, iv and/or v wherein the selection is made by a positive and/or negative measurement
wherein i, iv and vi or i, v and vi comprise the preferred arrangements.

### General Method - Selecting by Signalling

A method for selecting analyte eTPC-t and/or analyte APC and/or affinity reagents and/or NCBP from the combined eTPC:A system on the basis of a reported signal response comprises
i. Determining a native signalling response and/or
ii. Determining a synthetic signalling response, if the eTPC-t contains component F, and/or if the APC contains an equivalent synthetic reporter element.

An induced native or synthetic signal response that is intrinsic to APC and/or eTPC-t is measured by detecting an increase or decrease in one or more of the following
i. a secreted biomolecule
ii. a secreted chemical
iii. an intracellular biomolecule
iv. an intracellular chemical
v. a surface expressed biomolecule
vi. a cytotoxic action of the analyte eTPC-t upon the analyte **APC**
vii. a paracrine action of the analyte eTPC-t upon the analyte **APC** such that a signal response is induced in the analyte APC and is determined by detecting an increase or decrease any of a to e
viii. a proliferation of the analyte eTPC-t
ix. an immunological synapse between the analyte eTPC-t and the analyte APC
wherein said detected signal responses are compared to the non-induced signal response state intrinsic to analyte APC and/or analyte eTPC-t prior to assemble of the combined eTPC:A system and/or a parallel assembled combined system wherein analyte APC and/or analyte eTPC-t may present control analyte antigen and/or analyte TCR species and/or soluble analyte antigen that are known not to induce a signal response within the combined eTPC:A system in use.

### Method of Selection by Labelling and/or signal response

A method for selecting analyte eTPC-t and/or analyte affinity reagents and/or analyte NCBP from the combined eTPC:A system on the basis of a measureable labelling of an eTPC-t by said affinity reagent or NCBP comprises;
i. Determining a labelling of the eTPC-t by an affinity reagent or NCBP and may also comprise
ii. Determining a native signalling response and/or
iii. Determining a synthetic signalling response, if the eTPC-t contains component F.
wherein selecting an eTPC-t and/or affinity reagent and/or NCBP by detecting labelling of the eTPC-t may comprise detection of the surface labelling of the eTPC-t by an affinity reagent and/or NCBP via including a detectable label on the affinity reagent and/or NCBP. Such detectable labels may be fluorescent, luminescent, spectrometric, chemical, radiochemical or affinity moieties. Thus, such selection of eTPC-t may be conducted on the basis of FACS, MACS or equivalent high-throughput screening and selection methodologies.

### Summary

Within the combined eTPC:A system, measuring a signal response in the one or more analyte eTPC-t or one or more analyte APC, or the labelling of an eTPC-t, which may be mediated by the formation of a complex between the analyte TCRsp with the analyte antigen is critical to selection of primary system outputs **(****Figure 24** **step v),** wherein the primary system outputs are single cells or pools of cells, and/or or single affinity reagent or pools of affinity reagents and/or or single NCBP or pools of NCBP. Wherein the selection of cells or reagents may be made on the presence or absence of a reported signal response in either and/or both of the contacted analyte APC or analyte eTPC-t cells, or through the measurable labelling of eTPC-t with an affinity reagent or NCBP.

### Obtaining primary system outputs from the e TPC:A system

The present invention relates to the provision of a two-part device from which analyte eTPC-t are derived. These analyte eTPC-t are then combined with one or more analyte antigens via the eTPC:A system as described previously to obtain one or more outputs. The analyte antigen is provided by analyte antigen-presenting cells (APC) and/or as soluble or immobilised analyte antigen and/or presented on non-cell based particles (NCBP). The system is comprised of a selection of one or more of analyte antigen with one or more eTPC-t populations **(****Figure 24**). The eTPC:A system is provided in a format that permits physical contact between the analyte antigens and analyte eTPC-t populations, wherein such contact is permissive of complex formation between one or more analyte antigen and TCRsp of one or more analyte eTPC-t, wherein the analyte antigen is any of the following
i. aAPX and/or
ii. aAM and/or
iii. aAPX:aAM and/or
iv. CM and/or
v. aAPX:CM
wherein the analyte antigen is either provided as, presented by an analyte APC, or presented by a soluble and/or immobilised analyte affinity reagent or analyte NCBP such that complex formation may lead to stabilisation of such a complex and wherein leads to labelling of the eTPC-t and/or the induction of signalling within the analyte eTPC, if included and/or the analyte APC, may be reported and measured.

The modes of induced signal response reporting, and/or eTPC-t labelling, are described above, and it is these reported responses and/or labelling that are required to be measured in obtaining the primary output of the two-part device compiled into an eTPC:A system.

Primary outputs from the eTPC:A system are selected cell populations and/or selected affinity reagents or selected NCBP, wherein the selection is made on the basis of;
i. a measurable labelling of eTPC-t by affinity reagent or NCBP and/or
ii. a detected signal response in an eTPC-t and/or
iii. lack of a detected signal response in an eTPC-t and/or
iv. a detected signal response in an analyte APC and/or
v. a lack of detected signal response in an analyte APC;
wherein a primary output may be represented as a single cell, or a pool of cells and/or one or more eTPC-t-associated affinity regent or NCBP.

A selection of analyte affinity reagent, NCBP or analyte APC and/or analyte eTPC-t from the combined eTPC:A system may be made on the basis of a response in the contacting cell. That is, an analyte APC may be selected on that basis of a reported response, or lack thereof, in the contacting analyte eTPC-t. Conversely, an analyte eTPC-t may be selected on that basis of a reported response, or lack thereof, in the contacting analyte antigen, or in the case wherein the analyte antigen is an analyte affinity reagent or NCBP, the analyte affinity reagent or NCBP can selected from the eTPC-t response.

Primary APC outputs from the system are selected cells, wherein selection is made based on the presence or absence of a reported signal response in either analyte APC or eTPC-t, and these cells may comprise one or more of APC and/or eTPC-t wherein the selected cells may comprise a single cell, a pool of cells of the same identity, a pool of cells of different identities **(****Figure 24** **step v).**

Primary eTPC-t outputs from the system are selected cells, wherein selection is made based on the presence or absence of a reported signal response, and these cells comprise eTPC-t, wherein selected cells may comprise a single cell, a pool of cells of the same identity, a pool of cells of different identities **(****Figure 24** **step v).**

Primary analyte affinity reagents or NCBP outputs from the system are selected cells with or without associated affinity reagent or NCBP, wherein selection is made based on the presence or absence of a labelling or reported signal response by the analyte eTPC-t, wherein selected affinity reagent or NCBP may comprise a single affinity reagent or NCBP, a pool of affinity reagent or NCBP of the same identity, a pool of affinity reagent or NCBP of different identities **(****Figure 24** **step v).**

The reported signals in the analyte APC and/or analyte eTPC-t in a combined eTPC:A system may be used to select analyte cell populations or analyte affinity reagents or NCBP to provide the primary outputs.

A primary output of APC and/or eTPC-t types may be achieved in an instance wherein the combined eTPC:A system is of binary culture nature **(e.g.** **Figures 15 to 18****)** by selecting the desired analyte APC and/or analyte eTPC-t population from the binary system.

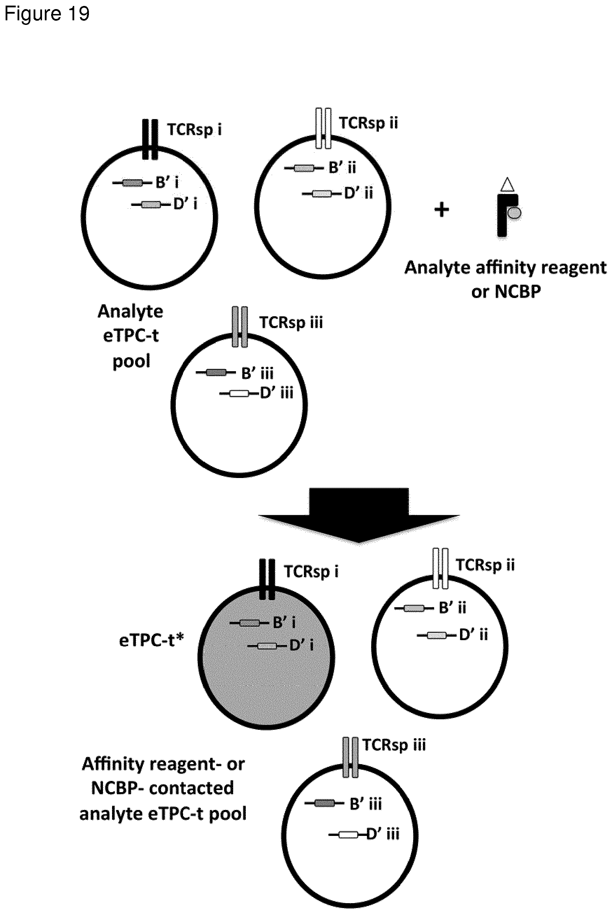

A primary output of an eTPC-t may be achieved in an instance wherein the combined eTPC:A system is of binary composition of one or more analyte eTPC-t with a analyte antigen (e.g. **Figures 19 to 21**) by selecting the desired analyte eTPC-t population that is labelled with the analyte affinity reagent or NCBP, or activated by the analyte affinity reagent or NCBP or analyte APC within the eTPC:A system.

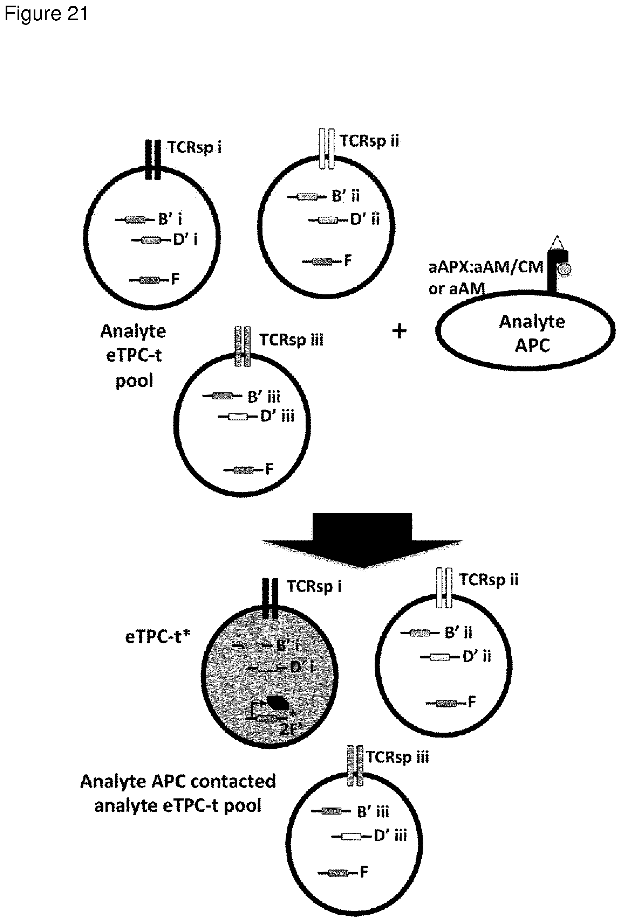

A primary output of an analyte affinity reagent or NCBP may be achieved in an instance wherein the combined eTPC:A system is of binary composition of one or more analyte eTPC-t with a analyte affinity reagent or NCBP (e.g. **Figure 21**) by selecting the desired analyte eTPC-t population that is labelled with, and/or has a signal induced by, the analyte affinity regent or NCBP from the eTPC:A system.

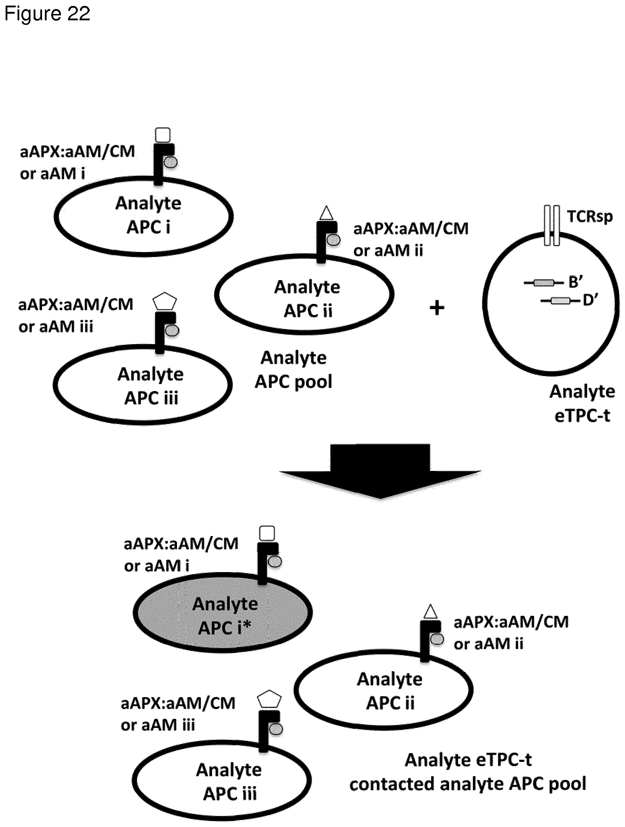

A primary output of APC may be achieved from an instance wherein the combined eTPC:A system is of fixed analyte eTPC-t and pooled library analyte APC (e.**g.** **Figure 22**) by selecting analyte APC based on a detection of a response, or lack thereof, within the analyte APC.

There are several distinct modes in which the primary outputs may be obtained, wherein each mode entails a step of sorting. Sorting may be achieved through fluorescence-activated cell sorting (FACS) and/or magnetic-activated cell sorting (MACS) and/or distinct affinity-activated cell sorting methods.

Primary output APC and/or eTPC-t cells, and/or eTPC-associated affinity reagents or NCBP, may be obtained by single cell sorting to obtain a single cell and/or cell sorting to a pool to obtain a pool of cells.

Primary output APC and/or eTPC-t cells may be obtained by single cell sorting to obtain a single cell, and optionally subsequent outgrowth of the single cells to obtain monoclonal pool of selected APC or eTPC-t cells.

Primary output APC and/or eTPC-t cells may be obtained by cell sorting to a pool to obtain a pool of cells, and optionally subsequent outgrowth of the pool of cells to obtain a pool of selected APC and/or eTPC-t cells.

### Obtaining terminal system outputs from the eTPC:A system

Subsequent to the above-described methods of obtaining primary outputs, wherein primary outputs are selected analyte APC and/or analyte eTPC-t that are selected on the basis of a measured signal response, or stable complex formation, such that the terminal outputs from the eTPC:A system may be obtained via further processing of the selected APC and/or eTPC primary outputs.

In the present context, terminal outputs from the multicomponent system are the identities of
i. aAPX and/or
ii. aAM and/or
iii. aAPX:aAM and/or
iv. CM and/or
v. aAPX:CM and/or
vi. TCRsp
presented by the analyte APC or analyte eTPC-t or an analyte affinity reagent or NCBP, and obtained as primary outputs from the multicomponent system by their selection from the combined eTPC:A system.

Within the eTPC:A system, it is often the case that analyte molecules that are presented by the analyte APC and analyte eTPC-t are genetically encoded. It may also be the case that an analyte NCBP has a genetically encoded identity, in the case of bacteriophage displayed NCBP, for example. Therefore, to identify the analyte molecules presented by the analyte APC or analyte eTPC-t, genetic sequencing of the prepared analyte APC, eTPC-t and analyte NCBP may be performed.

APC may be processed such that genetic sequence is obtained for the genome or transcriptome of the sorted and/or expanded APC cells to determine the identity of
i. aAPX and/or
ii. aAM and/or
iii. aAPX:aAM
iv. CM and/or
v. aAPX:CM and/or
wherein the obtained identities represent terminal outputs from the eTPC:A system.

In the present context, analyte NCBP that possess a genetic component may be processed such that genetic sequence is obtained for the genome or transcriptome of the sorted and/or expanded analyte NCBP to determine the identity of analyte NCBP, wherein the obtained identities represent terminal outputs from the eTPC:A system.

An eTPC-t may be processed such that genetic sequence is obtained for component B' and/or component D' of the sorted and/or expanded eTPC-t cells to determine the identity of TCRsp, wherein the obtained identify of TCRsp represents a terminal output from the eTPC:A system.

eTPC may be processed such that genetic sequence is obtained for the genome or transcriptome of the sorted and/or expanded eTPC-t cells to determine the identity of TCRsp, wherein the obtained identify of TCRsp represents a terminal output from the eTPC:A system.

Genetic sequencing can be achieved by a range of modes, and from a range of genetic material sources, with and without specific processing.

In the present context, the sequencing step may be preceded by
i. Extracting of genomic DNA and/or
ii. Extracting of components B' and/or D' RNA transcript and/or
iii. Amplifying by a PCR and/or a RT-PCR of the DNA and/or RNA transcript of component B' and/or D'.

The sequencing step may be destructive to the APC or eTPC-t or analyte NCBP, or pool thereof, obtained as primary outputs from the multicomponent system.

If it is desirable to obtain primary outputs from the eTPC:A system wherein the sequencing step has been destructive to the primary output eTPC-t, the sequence information obtained as terminal output of the two-part device may be used to prepare equivalent output eTPC-t as analyte eTPC-t.

In the above described scenarios of genetically encoded analyte molecules, the terminal outputs of the eTPC:A system may be obtained by obtaining sequence information from component B' and/or D', and/or from the cell genome and/or transcriptome. However, in some embodiments the antigen information will not be genetically encoded. Post-translationally modified antigens, antigens provided to the combined eTPC:A system through non-genetic means, antigens that are emergent from a induced or modified state of the analyte APC proteome or metabolite, CM intrinsic to the eTPC:A system, and affinity reagents or NCBP without a genetic element, may not reasonably be identified through genetic sequencing means.

In the important case of aAM that may be provided to the eTPC:A system by non-genetic means, there are two distinct modes through which an APC may present a provided aAM as an aAPX:aAM complex. In the first scenario the aAM is provided in a form that may directly bind to the aAPX and forms an aAPX:aAM complex at the cells surface. An example of such an aAM would be a peptide antigen for an HLA complex. In the second scenario, the aAM is provided is in a form that may be taken up by the analyte APC and processed such that it is loaded as cargo in the aAPX and forms an aAPX:aAM complex at the cells surface.

A method to select and identify an **aAM** cargo or a **CM** cargo, wherein the cargo is a metabolite and/or a peptide, that is loaded in an **aAPX** of an APC selected and obtained by as a primary output of the multicomponent system, comprises
i. isolating an **aAPX:aAM** or an **aAPX:CM** or the **cargo aM** or the **cargo CM** and
ii. identifying the loaded cargo
wherein the identified loaded cargo (CM or aM) represent terminal outputs of the two-part device.

There are generally two modes through which a cargo molecule may be identified from a selected APC. First, a forced release of the cargo from the aAPX:aAM or aAPX:CM results in isolation of the aAM or CM that is available for subsequent identification. An example of this is acid-washing of the APC to liberate peptide aAM from HLA complexes. Secondly, the capture of the aAPX:aAM or aAPX:CM, for example, by liberation of the complex and immunoaffinity isolation methods, results in isolation of the aAPX:aAM or aAPX:CM compelxes, such that aAM or CM can be identified.

Methods for identifying isolated aAM and/or CM directly, or from the isolated **aAPX:aAM** or an **aAPX:CM** complexes, can comprise
i. Mass spectrometry analysis
ii. Peptide sequencing analysis
wherein the contain aAM and/or CM identities are terminal outputs from the two-part device.

### Determining the Affinity of the TCRsp for analyte antigen using the two-part device as an eTPC:A system

Subsequent to the above-described methods of obtaining primary outputs, wherein primary outputs are selected analyte eTPC-t cells that are selected on the basis of a measured signal response, the eTPC-t primary outputs may be subjected to an affinity analysis to determine the affinity of the **TCRsp** to a cognate analyte antigen wherein the analyte antigen is any of the following
i. aAPX and/or
ii. aAM and/or
iii. aAPX:aAM and/or
iv. CM and/or
v. aAPX:CM
and wherein the analyte antigen is either provided as a soluble reagent or presented by an analyte APC, or analyte NCBP such that the affinity of the analyte TCRsp is determined according to the following method
i. Labelling the selected analyte eTPC-t with the analyte antigen at range of concentrations
ii. Conducting FACS analysis on the stained analyte eTPC-t of step a
iii. Determining the intensity of fluorescent labelling of the analyte eTPC-t over the range of concentrations of analyte antigen
iv. Calculating the affinity of the TCRsp to the analyte antigen

The affinity of the analyte TCRsp may also be determined by the previously described method but wherein a labelled reference may also be included, such that the affinity is calculated using the ratio of the analyte antigen fluorescence intensity to the reference fluorescence intensity wherein the labelled reference is selected from
i. The analyte eTPC-t labelled with an affinity reagent to one of the analyte TCR chains or to both analyte TCR chains
ii. The analyte eTPC-t labelled with an affinity reagent to one or more of the CD3 proteins
iii. a cell or particle presenting a labelled reference single TCR chain or labelled reference pair of TCR chains

### Use of eTPC-t as assay standards

The present invention relates to the provision of an engineered multi-component system, the components of which are used to prepare one or more analyte eTPC-t, and the use of the prepared eTPC-t in an eTPC:A analytical system, which is used to determine the analyte antigen specificity of TCRsp presented by analyte eTPC-t and if desired, the selection of the eTPC-t with or without affinity towards the analyte antigen.

This system is ideally suited to rapidly generate TCR-presenting standards for use in the control and calibration of assays detecting the presence of TCR-bearing cells (see Example 9), or for the development and/or control in manufacturing affinity reagents and/or NCBP aimed at detecting TCR pairs of selected specificities.

For the past two decades HLA multimer reagents have been a key part of T-cell research, being used to label T-cells (and other cell types) presenting particular TCRs, as is employed throughout the above examples. Originally devised as tetramerised reagents on the basis of biotinylation of the HLA molecule and multimerisation with strep-tavidin, these reagents now take various forms at differing degrees of multimerisation. The multimerisation is generally considered key to reliable function of the HLA multimer reagents, as the TCR-antigen-HLA interaction is relatively weak, and thus the multimeric state of the HLA-antigen molecule stabilises interaction with cognate TCR. A typical assay entails, contact of the HLA multimer reagent with prepared peripheral blood specimens, and detection of labelled T-cells within the specimen by use of flow cytometry.

Considering the relatively complex nature of these reagents, unlike the robust production and use of antibody reagents, there has been little uptake of their use into clinical diagnostics. A key challenge to integration into routine clinical diagnostics is a means to control for false positives, and false negative signal detection, in the context of a low-frequency T-cell analyte in complex biological specimens, using relatively complex and unstable reagents. False positives are often observed in the case of degraded HLA-multimer reagents, which may result in higher non-specific binding to T-cells. Reagent degradation can also abrogate specific TCR binding, leading to false negative readouts. A related challenge is the issue that any given antigen-specific T-cell population is likely to represent just a tiny fraction of cells in a patient specimen, and thus HLA multimer labelling often approaches the practical limit if detection fro flow cytometric assays. Therefore, it is often unclear whether the lack of observed T-cell labelling in a given specimen represents a true negative or a false negative result. This ambiguity is further amplified by the relatively unstable nature of the reagents themselves, and their sensitivity to correct specimen preparation.

To overcome the unreliable nature of HLA multimer reagent assays, a robust and highly defined assay standard is required. It is possible to produce recombinant TCs in various formats that could be functionalised on flow cytometry compatible microbeads. However, such production is unreliable, and it is often impractical to produce certain pairs in significant quantities. Rather than a recombinant protein TCR standard reagent, the eTPC-t represents a recombinant cell standard reagent that can express any human TCR, and can be rapidly mass-produced in simple mammalian cell culture systems. Moreover, the TCR in an eTPC-t is presented in the native CD3 complex context at the cell surface, thus reliably reflects the same conformation of TCR within the analyte primary T-cell population (or other cell population). One key aspect in the use of eTPC-t for reliable assay standards is the stability and practical distribution of the eTPC-t reagent, since viable cell cultures are highly impractical for clinical diagnostic use, or even routine research use.

An eTPC:A system as described above may thus be used to select and characterise one or more eTPC-t bearing TCRsp of defined specificity and staining intensity by desired antigen bearing affinity reagent, NCBP or other detection reagent.

Selected eTPC-t, or eTPC-t generated with independently selected TCR pairs, may be used as standard reagents for control of antigen bearing affinity reagents, NCBP or other detection reagents in assays designed to detect the presence of specific TCR pairs. Such an assay may comprise,
a) A cytometric assay for determination of the presence of T-cell of selected TCR specificity in a clinical specimen;
b) A cytometric assay for determination of the presence of cells bearing specific TCR in the manufacture of TCR-expressing primary T-cell cellular therapeutic products;
c) A cytometric assay for determination of the presence of cells bearing specific TCR in the manufacture of TCR-expressing engineered T-cell cellular therapeutic products;
d) An assay for determination of specificity and/or quality of detection of a given affinity reagent, NCBP or other reagent designed to detect a specific TCR pair;
wherein; such an assay may represent a flow cytometric assay or static binding using labelled or unlabelled reagents.

An eTPC-t for use in such assays may be prepared as
a) A viable cell
b) A cryopreserved cell
c) A chemically fixed cell
d) An irradiated cell
wherein; a) and b) may be combined; b) and c) may be combined; b) and d) may be combined.

### Legends to figures

The invention is illustrated in the following non-limiting figures.

### Figure 1 - Description of the components of a single integration couple Multi-component System

An example of a multicomponent cellular system (MCS) that is not claimed comprising four components. The first component **A** is the **eTPC** line itself with all required engineered features of that cell. The **eTPC A** contains a second component, **B,** which is a genomic integration site for integration of a pair of complementary analyte TCR chain ORFs. A third component included in the **eTPC, A,** is a synthetic reporter construct that is induced upon TCR ligation, **F**. One additional independent component, **C**, represents a genetic donor vectors for site-directed integration of ORFs into site **B**, where arrow indicates coupled specificity.

### Figure 2 - Description of the components of a dual integration couple Multicomponent System

An example of a **multicomponent cellular** system (MCS) comprising six components. The first component **A** is the **eTPC** line itself with all required engineered features of that cell. The **eTPC A** contains three further components, two of which are **B and D**, which are genomic integration sites for integration of an analyte TCR chain pair. A third component included in the **eTPC, A,** is a synthetic reporter construct that is induced upon TCR ligation, **F**. Two additional independent components, **C** and **E**, represent genetic donor vectors for site-directed integration of ORFs into sites **B** and **D**, respectively, where arrows indicate coupled specificity. Each paired integration site / donor vector couple may be formatted to integrate a single ORF or a pair of ORFs to introduce analyte TCR chain pair expression by different means.

### Figure 3 - Compilation of different analyte TCRsp presenting eTPC

The operation of the multicomponent cellular system (MCS) to prepare analyte **eTPC** populations begins with the **eTPC** and uses a donor vector(s) to create cells expressing analyte **TCRsp,** or single analyte TCR chains. An **eTPC** presenting **TCRsp** is termed **eTPC-t,** and may be created by introduction of **two complimentary TCR chain** encoding ORFs to the **eTPC (step i).** An **eTPC** expressing **a single analyte TCR chain** alone is termed an **eTPC-x,** and may be created by introduction of **a single TCR chain** encoding ORF(s) to the **eTPC (step ii).** A **eTPC-t** may alternatively be created from an eTPC-x, wherein **a second complimentary TCR chain** encoding ORF is introduced to an existing **eTPC-x (step iii).** In some instances, **an eTPC-x** may be created **from an eTPC-t** by removing a single analyte TCR chain **(step iv)**

### Figure 4 - Operation of the genetic donor vector and genomic receiver site integration couple

A genetic donor vector and genomic receiver site form an integration couple, wherein one or more ORFs encoded within the genetic donor vector can integrated specifically to its coupled genomic receiver site. **Step 1** in operation of the integration couple is to introduce one or more target ORFs to the donor vector. The initial donor vector is denoted **X**, and is modified to a primed donor vector **X'**, by introduction of target ORF(s). **Step 2** entails combination of the primed donor vector, **X',** with a cell harbouring a genomic receiver site, Y. Introduction of the ORF encoded by the primed donor vector into the receiver site results in the creation of a cell harbouring an integrated site, **Y'.**

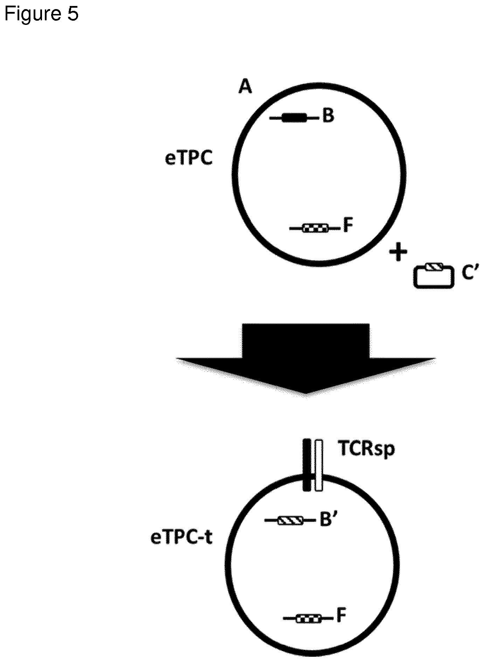

### Figure 5 - Non-claimed Compilation of an eTPC system with a one-step and one-vector format

**eTPC A** contains distinct genomic receiver site. The genetic donor vectors **C** is coupled to **D**. Donor vector **C** encodes a **TCR chain pair.** The **eTPC A** further contains a **TCR signal response element F.** The **eTPC A** is combined with donor vector **C**. The resulting cell has insert **C** exchanged to the **B** genomic receiver site to create site **C'** and deliver the two ORFs for a **TCR chain pair.** This cell is capable of presenting a **TCRsp** at the surface, and is thus designated an **eTPC-t.**

### Figure 6 - Compilation of an eTPC-t in one step with one vector and an unused receiver site

**eTPC A** contains distinct genomic receiver sites **B and D**. The genetic donor vectors **C'** is coupled to **D**. Donor vector **C'** encodes a **TCR chain pair.** The **eTPC A** further contains a **TCR signal response element F.** The **eTPC A** is combined with donor vector **C'**. The resulting cell has insert **C'** exchanged to the **B** genomic receiver site to create site **B'** and deliver the two ORFs for a **TCR chain pair.** Genomic receiver site **D** remains unused. This cell is capable of presenting a **TCRsp** at the surface, and thus designated a **eTPC-t.**

### Figure 7 - Compilation of an eTPC-t in one step with two vectors and two integration couples

**eTPC A** contains distinct genomic receiver sites **B and D.** The **eTPC A** further contains a **TCR signal response element F.** Distinct genetic donor vectors **C'** and **E'** are independently coupled to **B** and **D**, respectively. Donor vector **C'** encodes a single **TCR chain,** and donor vector **E'** encodes a second reciprocal **TCR chain.** The **eTPC A** is combined with donor vectors **C'** and **E'.** The resulting cell has insert **C** exchanged to the **B** genomic receiver site to create site **B'** and deliver an ORF for a first **TCR chain.** In addition, the resulting cell line has insert **E'** exchanged to the **D** genomic receiver site to create site **D'** and deliver an ORF for a second **TCR chain.** This cell is capable of presenting a **TCRsp** at the surface, and is thus designated an **eTPC-t.**

### Figure 8 - Compilation of an eTPC-x in one step with one vector and an unused receiver site

**eTPC A** contains distinct genomic receiver sites **B** and **D**. The genetic donor vectors **C'** is coupled to **D.** Donor vector **C'** encodes a single **TCR chain.** The **eTPC** A further contains a **TCR signal response element F.** The **eTPC A** is combined with donor vector **C'.** The resulting cell has insert **C'** exchanged to the **B** genomic receiver site to create site **B'** and deliver a single TCR chain ORF. Genomic receiver site **D** remains unused. This cell expresses only a **single TCR chain** and is thus designated an **eTPC-x.**

### Figure 9 - Compilation of an eTPC-t in two steps with two vectors

**eTPC A** contains distinct genomic receiver sites **B** and **D.** The **eTPC** A further contains a **TCR signal response element F.** Distinct genetic donor vectors **C'** and **E'** are independently coupled to **B** and **D**, respectively. Donor vector **C'** encodes a single **TCR chain,** and donor vector **E'** encodes a second reciprocal **TCR chain.** In **STEP 1** a **eTPC A** is combined with donor vector **C'.** The resulting cell has insert **C'** exchanged to the **B** genomic receiver site to create site **B'** and deliver an ORF for a first **TCR chain.** This cell expresses only a **single TCR chain** and is thus designated an **eTPC-x.** Genomic receiver site **D** remains unused. In **STEP 2,** the **eTPC-x** is combined with donor vector **E'.** The resulting cell has insert **E'** exchanged to the **D** genomic receiver site to create site **D'** and deliver an ORF for a second complementary **TCR chain.** This cell is capable of presenting a **TCRsp** at the surface, and is thus designated an **eTPC-t.**

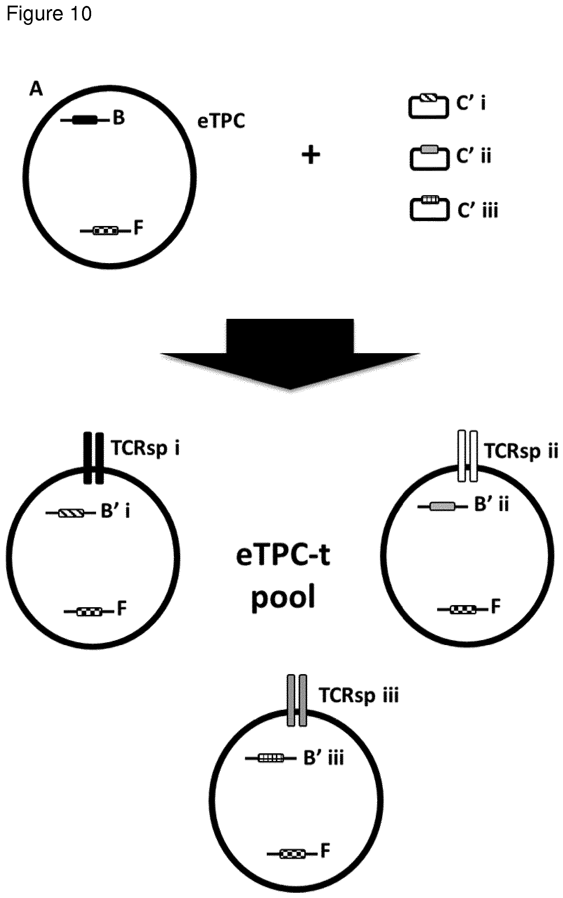

### Figure 10 - Shotgun compilation of an eTPC-t pool from an eTPC with paired ana-Iyte TCR chains in single replicate vector to express discrete TCR chain pairs from a selected TCR chain pair library

**eTPC A** contains a genomic receiver site **B.** The genetic donor vectors **C'** is coupled to **B.** Donor vector **C' i, C' ii** and **C' iii** encode a distinct **TCR chain pair** and constitutes a mixed vector library of discrete **TCR chain pairs.** The **eTPC A** further contains a **TCR signal response element F.** The **eTPC A** is combined with donor vectors **C' i, C' ii** and **C' iii** simultaneously. The resulting cell pool has insert **C** exchanged to the **B** genomic receiver site multiple independent instances to create site **B' i, B' ii** and **B' iii** delivering two ORFs for each discrete **TCR chain pair** contained in the initial vector library. This **eTPC-t** cell pool comprises a mixed population of three distinct cell clones each expressing a distinct **TCR chain pairs,** denoted **TCRsp i, ii** and **iii,** forming an **eTPC-t** pooled library.

### Figure 11 - Shotgun compilation of an eTPC-t pool from an eTPC with paired analyte TCR chains in single replicate vector to express discrete TCR chain pairs from a selected TCR chain pair library and an unused receiver site

**eTPC A** contains distinct genomic receiver sites **B and D.** The genetic donor vectors **C'** is coupled to **B.** Donor vector **C' i, C' ii** and **C' iii** encode a distinct **TCR chain pair** and constitutes a mixed vector library of discrete **TCR chain pairs.** The **eTPC A** further contains a **TCR signal response element F.** The **eTPC A** is combined with donor vectors **C' i, C' ii** and **C' iii** simultaneously. The resulting cell pool has insert **C** exchanged to the **B** genomic receiver site multiple independent instances to create site **B' i, B' ii** and **B' iii** delivering two ORFs for each discrete **TCR chain pair** contained in the initial vector library. This **eTPC-t** cell pool comprises a mixed population of three distinct cell clones each expressing a distinct **TCR chain pairs,** denoted **TCRsp i, ii** and **iii,** forming an **eTPC-t** pooled library. Genomic receiver site **D** remains unused.

### Figure 12 - Shotgun compilation of an eTPC-t pool from an eTPC with two vectors to express random combinations of paired TCR chain pairs from a selected single TCR chain library

**eTPC A** contains distinct genomic receiver sites **B and D.** Distinct genetic donor vectors **C'** and **E'** are independently coupled to **B and D**, respectively. Donor vectors **C'** i and **C' ii** each encode a single **TCR chain,** and donor vectors **E'** i and **E' ii** each encode a reciprocal single **TCR chain.** The **eTPC A** further contains a **TCR signal response element F.** The **eTPC A** is combined with donor vectors **C' i, C' ii, E' i** and **E' ii** simultaneously. The resulting cell pool has insert **C' i** or **C' ii** exchanged to the **B** genomic receiver site multiple independent instances to create sites **B' i** and **B' ii**, each delivering a single ORF for a **TCR chain.** The resulting cell pool further has insert **E i** or **E ii** exchanged to the **D** genomic receiver site multiple independent instances to create sites **E' i** and **E' ii,** each delivering a single ORF for a **TCR chain** reciprocal to those at sites **C' i** and **C' ii.** The resulting **eTPC-t** cell pool comprises a mixed population of four distinct cell cohorts each expressing a discrete randomised **TCRsp** at the surface comprised of one of each reciprocal **TCR chain** contained in the initial vector library.

### Figure 13 - Shotgun compilation of an eTPC-t pool from an eTPC-x with unpaired analyte TCR chains to express random combinations of paired TCR chain pairs from a selected single TCR chain library

**eTPC-x** contains the exchanged genomic receiver site **B'** expressing a single **TCR chain** and the distinct genomic receiver site **D**. Distinct genetic donor vectors **E' i** and **E' ii** are coupled to **D**, respectively. Donor vectors **E' i** and **E' ii** each encode a single **TCR chain.** The **eTPC-x** further contains a **TCR signal response element F.** The **eTPC-x** is combined with donor vectors **E'** i and **E' ii** simultaneously. The resulting cell pool has insert **E' i** or **E' ii** exchanged to the **D** genomic receiver site multiple independent instances to create sites **E i** and **E' ii,** each delivering a single ORF for a **TCR chain.** The resulting **eTPC-t** cell pool comprises a mixed population of 2 distinct cell cohorts expressing a discrete **TCRsp** at the surface comprised of the **TCR chain** expressed from **B'** paired with one of each **TCR chain** contained in the initial vector library.

### Figure 14 - Reversion of an eTPC-t to an eTPC-x

The cell depicted in the upper panel is capable of presenting a **TCRsp** at the surface, and is thus designated a **eTPC-t.** This eTPC-t has genomic receiver sites **B** and **D** occupied by TCR ORFs, rendering them in the **B'** and **D'** forms. Genetic donor vectors harbouring genomic receiver site marker(s), and coupled to sites **B'** or **D'**, designated **Y** and **Z.** Addition of **Y** or **Z** to the eTPC-t will exchange the genomic receiver site marker for the TCR chain encoded by either **B'** or **D'.** The resulting cell expresses only a single TCR chain, and thus is designated **eTPC-x.**

### Figure 15 - Operation of a combined analyte eTPC:A system showing possible analyte affinity reagent- or NCBP-bound eTPC-t output states

The analyte **eTPC-t** contains sites **B'** and **D'** each integrated with one ORF encoding a reciprocal **TCRsp** at the surface. When analyte **eTPC-t** and **analyte affinity reagent or NCBP** are contacted, different **eTPC-t** labelling states can be achieved; in this example one negative and three positive. The negative state is the resting state of the input **eTPC-t,** with no detectable binding of the analyte affinity reagent or NCBP, denoting failure of the analyte affinity reagent or NCBP to form a stable complex with the **eTPC-t-**presented **TCRsp.** Three positive states show hypothetical range of the degree of binding of the analyte affinity reagent or NCBP, as denoted by darker shading of the cells. This indicates a graded binding of analyte **affinity reagent or NCBP** analyte to the **TCRsp** expressed by **eTPC-t** population.

### Figure 16 - Operation of a combined analyte eTPC:A system showing possible signal-reported eTPC-t-output states in response to analyte affinity reagent or NCBP

The analyte **eTPC-t** contains sites **B'** and **D'** each integrated with one ORF encoding a reciprocal **TCRsp** at the surface. The **eTPC-t** further contains a **TCR signal response element F.** When analyte **eTPC-t** and **analyte affinity reagent or NCBP** are contacted, different **eTPC-t** response states can be achieved, in this example one negative and three positive. The negative state is the resting state of the **eTPC-t,** with no signal strength at the **F** element, denoting failure of the analyte **affinity reagent or NCBP** to form a complex and stimulate the **eTPC-t** presented **TCRsp.** Three positive states show increasing signal strength from the **F.** States **F'+, F'++** and **F'+++** denote low, medium and high signal strength, respectively. The gene product of **F** denoted as hexagons accumulates to report signal strength of each cell state, as denoted by darker shading of the cells. This indicates a graded response of analyte **TCRsp** expressed by **eTPC-t** population towards analyte affinity reagent or NCBP resulting in signal transduction to the F element.

### Figure 17 - Operation of a combined analyte eTPC:A system showing possible signal-reported eTPC-t-output states in response to analyte APC

The analyte **eTPC-t** contains sites **B'** and **D'** each integrated with one ORF encoding a reciprocal **TCRsp** at the surface. The **eTPC-t** further contains a **TCR signal response element F.** When analyte **eTPC-t** and **analyte APC** populations are contacted, different **eTPC-t** response states can be achieved, in this example one negative and three positive. The negative state is the resting state of the **eTPC-t,** with no signal strength at the **F** element, denoting failure of the analyte **APC-presented aAPX:aAM/CM** or **aAM** to stimulate the **eTPC-t** presented **TCRsp.** Three positive states show increasing signal strength from the **F.** States **F'+, F'++** and **F'+++** denote low, medium and high signal strength, respectively. The gene product of **F** denoted as hexagons accumulates to report signal strength of each cell state, as denoted by darker shading of the cells. This indicates a graded response of analyte **TCRsp** expressed by **eTPC-t** population towards analyte **aAPX:aAM/CM** or **aAM** presented by the analyte **APC.**

### Figure 18 - Operation of a combined analyte eTPC:A system showing possible analyte APC output states

The analyte **eTPC-t** contains sites **B'** and **D'** each integrated with one ORF encoding a reciprocal **TCRsp** at the surface. The **eTPC-t** further contains a **TCR signal response element F.** When analyte **eTPC-t** and analyte **APC** populations are contacted, different **APC** response states can be achieved, in this example one negative and three positive. The negative state is the resting state of the analyte **APC,** denoting failure of the **TCRsp** chain pair to stimulate the **aAPX:aAM/CM or aAM** complex presented by the analyte **APC.** Three positive states show increasing signal strength from the contacted **aAPX:aAM/CM or aAM.** The reported signal strength of each cell state, is denoted by *, **, ***, and also denoted by darker shading of the cells. This indicates a graded response of analyte **aAPX:aAM/CM or aAM** towards the analyte **TCRsp** chain pair presented by the analyte **eTPC-t.**

### Figure 19 - Operation of a combined eTPC:A to identify TCRsp chain pairs binding with analyte affinity reagent or NCBP from a pool of eTPC-t

The **eTPC-t** pool contains cells harbouring sites **B' i, ii** or **iii** and **D' i, ii** or **iii,** wherein each eTPC-t is integrated with a pair of ORFs encoding a pair of complementary TCR chains, and thus each cell cohort in the population expresses a discrete **TCRsp** at the surface. An analyte affinity reagent or NCBP is contacted with the analyte **eTPC-t** pool. In the present example, only the pair of TCR chains expressed from **B' i/D' i (TCRsp i)** is specific for the **analyte affinity reagent or NCBP** such that, only the cell cohort of the **eTPC-t** that bears **TCRsp i (etTPC-t*)** is able to detectably bind the analyte antigen or NCBP (*). The **eTPC-t*** bound to analyte affinity reagent- or NCBP- may be selected from the pool on the basis of the affinity reagent- or NCBP- labelling. Subsequently the analyte TCRsp-encoding ORFs of the selected and isolated **eTPC-t*** can be identified by sequencing of **B'** and **D'** DNA directly or indirectly through reverse-transcriptase PCR of the expressed transcripts of **B'** and **D'.**

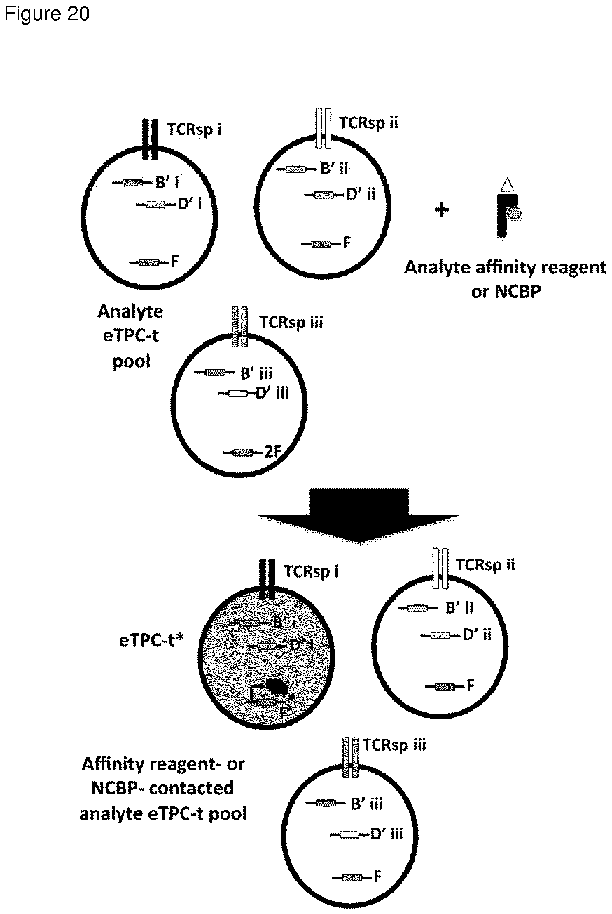

### Figure 20 - Operation of a combined eTPC:A system to identify TCRsp chain pairs from a pool of eTPC-t via induction of a signal-report response through stimulation with analyte affinity reagent or NCBP

The **eTPC-t** pool contains cells harbouring sites **B' i, ii** or **iii** and **D' i, ii** or **iii,** wherein each **eTPC-t** is integrated with a pair of ORFs encoding a complementary Pair of TCR chains, and thus each cell cohort in the population expresses a discrete **TCRsp** at the surface. The **eTPC-t** further contains a **TCR signal response element F.** An analyte antigen or NCBP is contacted with the analyte **eTPC-t** pool. In the present example, only the Pair of TCR chains expressed from **B' i/D' i (TCRsp i)** is specific for the **analyte affinity reagent or NCBP** such that, only the cell cohort of the **eTPC-t** that bears **TCRsp i (eTPC-t*)** is able to induce a signal report response via **element F** (*). The **eTPC-t*** bound to analyte affinity reagent- or NCBP- may be selected from the pool of **eTPC-t** on the basis of the affinity reagent- or NCBP- labelling. Subsequently, the analyte TCRsp-encoding ORFs of the selected and isolated **eTPC-t*** can be identified by sequencing of **B'** and **D'** DNA directly or indirectly through reverse-transcriptase PCR of the expressed transcripts of **B'** and **D'.**

### Figure 21 - Operation of a combined eTPC:A system to identify TCRsp chain pairs from an eTPC-t pool via induction of a signal-report response through stimulation with analyte APC

The **eTPC-t** pool contains cells harboring sites **B' i, ii** or **iii** and **D' i, ii** or **iii,** wherein each eTPC-t is integrated with a pair of ORFs encoding a reciprocal TCR chain pair, and thus each cell cohort in the population expresses a discrete **TCRsp** at the surface. The **eTPC-t** further contains a **TCR signal response element F.** The analyte **APC** express on the surface an **aAPX:aAM/CM** or **aAM.** In the present example, only the **TRC chain pair** expressed from **B' i/D' i (TCRsp i)** is specific for the **aAPX:aAM/CM** or **aAM** presented by the **analyte APC,** such that when **eTPC-t** pool and **analyte APC** population are contacted, only the cell cohort of the **eTPC-t** that bears **TCRsp i (eTPC-t*)** reports **TCRsp** engagement through state **F'.** The **eTPC-t*** stimulated by the analyte APC may be selected from the pool on the basis of signal-report response. Subsequently, the analyte TCRsp-encoding ORFs of the selected and isolated **eTPC-t*** can be identified by sequencing of **B'** and **D'** DNA directly or indirectly through reverse-transcriptase PCR of the expressed transcripts of **B'** and **D'.**

### Figure 22 - Operation of a combined eTPC:A system to identify analyte antigens presented by analyte APC, via induction of an APC-centric signal-report response

The analyte **APC** pool contains cells expressing varied **aAPX:aAM/CM or aAM** on their surface. The analyte **eTPC-t** contain the exchanged genomic receiver site **B' and D'** driving the expression of a **TCRsp** at the surface. In the present example, only the complex **aAPX:aAM/CM or aAM i** is specific for the **TCRsp** presented by the analyte **eTPC-t,** such that when analyte **APC pool** and analyte **eTPC-t** population are contacted, only the cell cohort expressing **aAPX:aAM/CM i** responds **(*).** This response may be an intrinsic signal response to eTPC-t engagement, such as a change in surface phenotype, transcript abundance or cell death. The responding analyte APC may be selected to determine aAPX:aAM/CM or aAM that has been contacted by the analyte TCRsp presented bet the analyte eTPC-t.

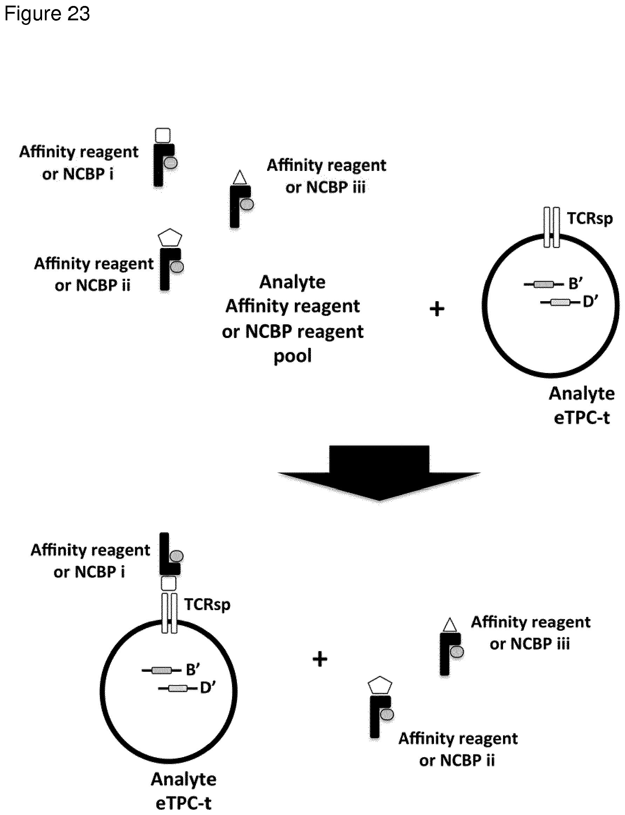

### Figure 23 - Operation of a combined eTPC:A system to identify affinity reagent or NCBP from a pool of such entities via capture of the affinity reagent or NCBP reagent by an eTPC-t

The analyte **eTPC-t** contain the exchanged genomic receiver site **B' and D'** driving the expression of a **TCRsp** at the surface. An **affinity reagent** or **NCBP** pool is contacted with analyte **eTPC-t,** which permits the binding of analyte affinity reagent or NCBP specific for **TCRsp** presented by the analyte eTPC-t. In the present depiction, the **TCRsp** specifically binds only **affinity reagent or NCBP i,** and thus the analyte **eTPC-t** is labelled with only **affinity reagent or NCBP i.** An **affinity reagent or NCBP** may thus be selected from the pool via association with the **eTPC-t,** to identify those affinity reagents or NCBP specific for the analyte **TCRsp** presented by the analyte **eTPC-t.**

### Figure 24 - Operation of the multicomponent system for preparing eTPC-t for assembly of a combined eTPC:A system

The overall system in which the engineered multicomponent cellular system (MCS) comprises contacting prepared analyte engineered TCR-presenting cells (eTPC) with various analyte antigens into combined **eTPC:A system.** It is from the combined eTPC:A system that primary outputs are derived, and from these primary outputs that terminal outputs are derived. Operation of the overall system comprises two phases, the preparation phase, and the analytical phase.

In one aspect of **Phase 1,** analyte antigens are prepared. Such analyte antigens express antigens in various forms of antigenic moiety; analyte antigen-presenting complexes **(aAPX);** analyte antigenic molecules **(aAM); aAPX** with loaded **aAM** cargo **(aAPX:aAM);** a cargo molecule **(CM);** an **aAPX** loaded with **CM (aAPX:CM);** wherein the analyte antigens represent those to be tested for affinity or signal induction against the analyte eTPC-t **(step i)** and wherein the analyte antigens may take the form of at least one of the following, soluble reagents, immobilized reagents, presented by non-cell based particles **(NCBP)** or presented on the surface of cells (APC). In another aspect of **Phase1, the multicomponent** system is used to prepare cells expressing analyte TCR chain pairs **(TCRsp)** at the cell surface **(step ii).** An **eTPC** presenting a **TCRsp** at the cell surface is termed an **eTPC-t,** wherein the eTPC-t present TCRsp to analyte antigens to test affinity or signal induction against the analyte antigens. The contact of eTPC-t and analyte antigens results from the assembly of a combined **eTPC:A system (step iii).**

**Phase 2** of the overall system is the contacting of eTPC-t and analyte antigens prepared in **Phase 1,** resulting in the assembly of a combined **eTPC:A system (step iii).** Contacted analyte **antigens** present analyte moieties to the **analyte eTPC-t** and **potentially bind eTPC-t** to form a stable complex with the presented **TCRsp.** Within the combined **eTPC:A system,** outputs of the analyte antigens, or analyte eTPC-t may change their signal state or the stable complex may be directly identified (denoted with *, **and the darker shading)** such that those responding or stably complexed species may be identified **(step iv).** Based on altered signal states within the **eTPC:A system, specific** analyte antigens may be selected on their ability to induce a response in an eTPC-t, or form a stable complex with the TCRsp, or the ability of an eTPC-t to induce a response in analyte antigen, if applicable. Similarly, an analyte eTPC-t may be selected on the ability to induce a response in the contacted analyte antigens, or for those analytes to induce a signal response in the eTPC-t, or for the analyte antigen to form a stable complex with the TCRsp. Selection based on this responsiveness or stable complex yields the primary outputs of the combined eTPC:A system **(step v).** By obtaining the analyte cells and/or analyte antigens from **step v,** the presented analyte **aAPX, aAM, aAPX:aAM, CM, aAPX:CM** and/or **TCRsp** , may be identified as the **terminal output** of the system operation **(step vi).**

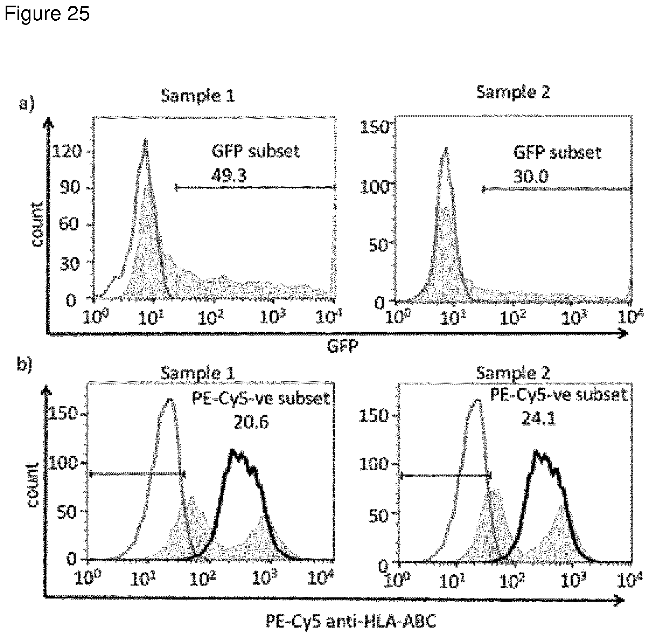

### Figure 25 - Selection of cells with targeted mutagenesis of the HLA-A, HLA-B and HLA-C loci in HEK239 cell line

a) GFP fluorescence signal in two independent cell populations 48 hours after transfection with plasmids encoding Cas9-P2A-GFP and gRNAs targeting the HLA-A, HLA-B and HLA-C loci (grey histogram) compared to HEK293 control cells (dashed lined histogram). Cells that had a GFP signal within the GFP subset gate were sorted as a polyclonal population. **b)** Cell surface HLA-ABC signal observed on the two sorted polyclonal populations when labelled with a PE-Cy5 anti-HLA-ABC conjugated antibody (grey histogram). Single cells that showed a low PE-Cy5 anti-HLA-ABC signal and were displayed within the sort gate were sorted to establish monoclones. Non-labelled HEK293 cells (dashed line histogram) and PE-Cy5 anti-HLA-ABC labelled HEK293 cells (full black lined histogram) served as controls.

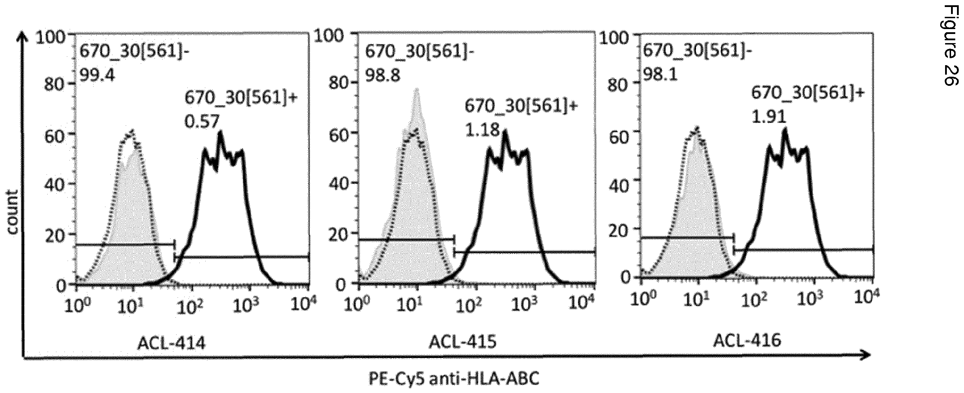

**Figure 26** - **Phenotypic analysis of HLA-ABC^{null} monoclones:** Monoclone populations were stained with the PE-Cy5 anti-HLA-ABC conjugated antibody, and were analysed by flow cytometry (grey histogram). Non-labelled HEK293 cells (dashed lined histogram) and PE-Cy5 anti-HLA-ABC labelled HEK293 cells (full black lined histogram) served as controls. All three monoclone lines showed a fluorescent signal matching to non-labelled controls demonstrating that each line lacked HLA-ABC surface expression.

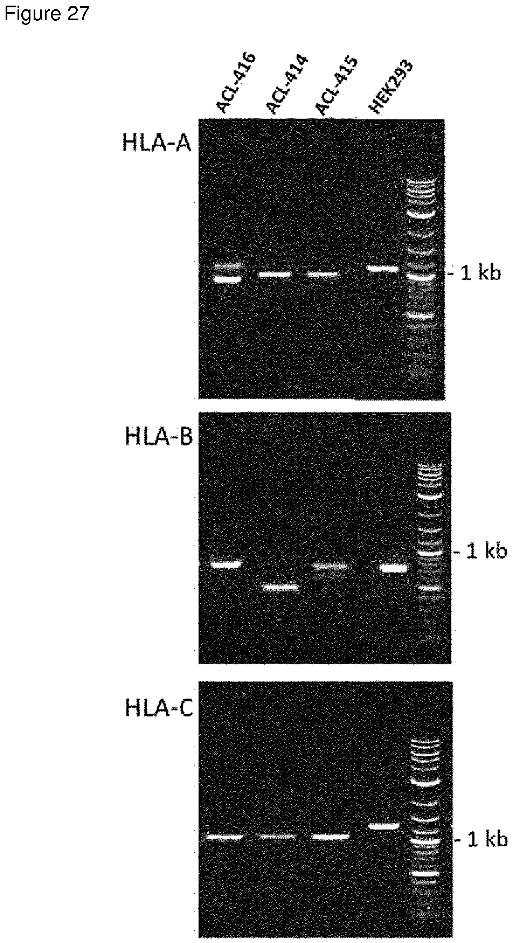

**Figure 27** - **Genetic characterization of a selection of monoclones lacking surface HLA-ABC expression demonstrating a genomic deletion in the targeted HLA alleles.** PCR amplicons were generated with primers that spanned the gRNA genomic target sites of a specific HLA alleles and their size determined by electrophoresis. The expected size of the wild type HLA-A amplicon is 1067 bp, HLA-B amplicon is 717 bp and HLA-C amplicon is 1221 bp.

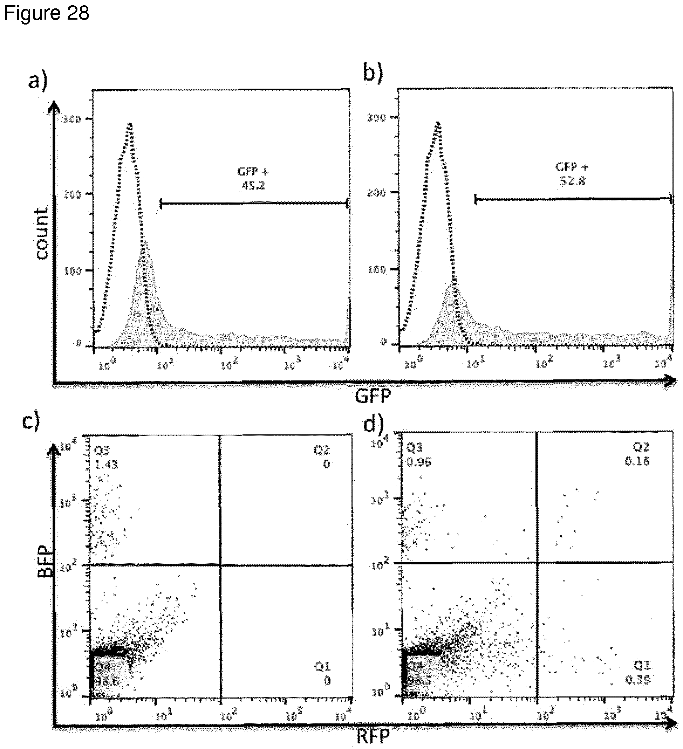

### Figure 28 - Selection of cells with targeted genomic integration of synthetic Component B with or without synthetic Component D

**a)** GFP fluorescence signal 48 hours after transfection with plasmids encoding Cas9-P2A-GFP, gRNAs targeting the AAVS1 locus and component B genetic elements flanked by AAVS1 left and right homology arms (grey histogram). HEK293 cells server as a GFP negative control (dashed line histogram). Cells that had a GFP signal within the GFP+ gate were sorted as a polyclonal population. **b)** GFP fluorescence signal 48 hours after transfection with plasmids encoding Cas9-P2A-GFP, gRNAs targeting the AAVS1 locus and component B and D, both flanked by AAVS1 left and right homology arms (grey histogram). HEK293 cells server as a GFP negative control (dashed line histogram). Cells that had a GFP signal within the GFP+ gate were sorted as a polyclonal population **c)** Maintained BFP but no detectable RFP signal observed in the D1 sorted polyclonal population. Single cells that showed high BFP signal in quadrant Q3 were sorted to establish eTPC containing synthetic component B monoclones. **d)** Maintained BFP and RFP signal observed in the D2 sorted polyclonal population. Single cells that showed high BFP and RFP signals in quadrant Q2 were sorted to establish eTPC monoclones containing synthetic component B and synthetic component D.

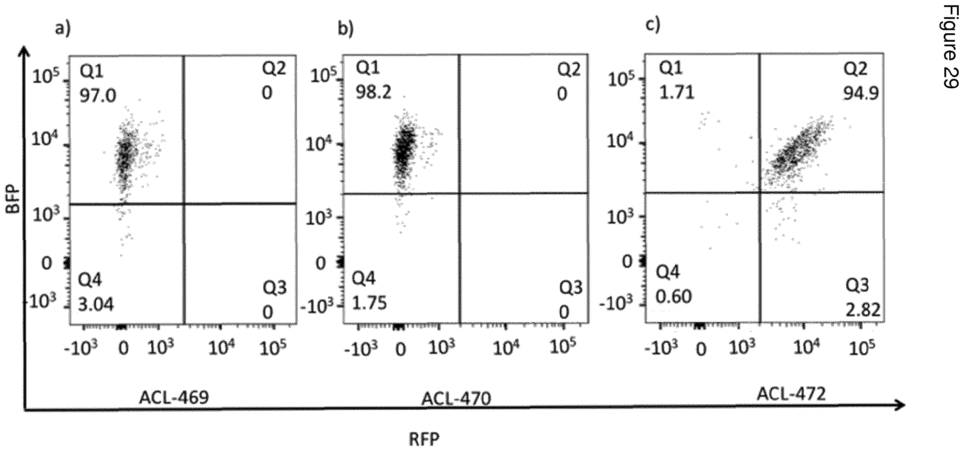

**Figure 29** - **Phenotypic analysis of eTPC monoclones: a and b)** Monoclone populations that display maintained BFP expression suggest the integration of synthetic component B. **c)** Monoclone populations that display maintained BFP and RFP expression suggest the integration of both synthetic component B and synthetic component D.

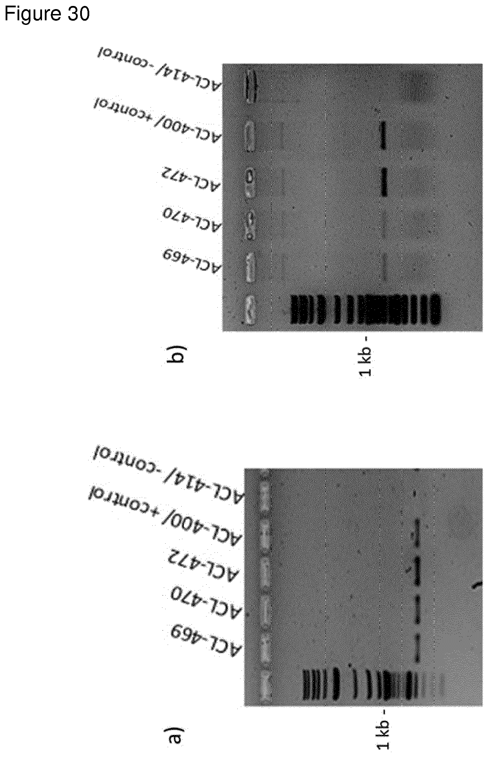

**Figure 30** - **Genetic characterization of a selection of monoclones for integration of Component B or Component B and D in the AAVS1 locus. a)** PCR amplicons were generated with primers that prime within component B and/or D and size determined by electrophoresis. The expected size of a positive amplicon is 380bp indicating stable integration of component B and/or D. **b)** PCR amplicons were generated with primers that prime on AAVS1 genomic sequence distal to region encoded by the homologous arms and the SV40 pA terminator encoded by component B and/or D and size determined by electrophoresis. The expected size of a positive amplicon is 660 bp indicating integration of component B and/or D occurred in the AAVS1 site.

### Figures 31 illustrates identification of target base cell and methods according to the invention to obtain an eTPC or variant thereof

A method to engineer and obtain an **eTPC** and/or an **eTPC+F** from a Target Base Cell wherein the method comprises:
- Step a is selecting of a **Target Base Cell**
- Step b is identifying and selecting a **TCRsp Competent Base Cell,** defined as a **Target Base Cell** that is competent at expressing on the surface of the cell a pair of complementary TCR chains as proteins in complex with CD3 (TCRsp)
- Step c is identifying and selecting a **Confirmed Base Cell,** defined as a **TCRsp Competent Base Cell** that contains a genomically encoded CD3, either natively or is genetically engineered, but is conditionally expressed on the surface when the cell is provided with nucleic acids encoding a complementary pair of TCR chains
- Step d is engineering and selecting an **Putative eTPC,** defined as a **Confirmed Base Cell** that has been engineered to contain a genomically integrated component B and/or component D, but wherein the exact number of copies and location have not been determined
- Step e is confirming and selecting an **eTPC,** defined as an **Putative eTPC** that has been confirmed to have only a single copy of each component B and/or D. Optionally, the component B and/or component D are tested and capable of integrating at least one ORF encoded within component C' and/or E'
- Step f is engineering and selecting an **Putative eTPC+F,** defined as an **eTPC** that has been engineered to contain a genomically integrated component F, but wherein the cell has not been tested to confirm that the cell can elicit a signal response via component F
- Step g is confirming and selecting an **eTPC+F,** defined as an **Putative eTPC+F** that has been tested a confirmed capable of eliciting a signal response via component F by stimulation with a cognate antigen of a complementary pair of TCR chains. Optionally, the genomic location and copy number of component F is determined

It is possible to create an eTPC+F directly from the Target Base Cell without the need to create an eTPC prior, wherein the method comprises Step a to c, and further comprises
- Step h is engineering and selecting an **Putative Base Cell+F,** defined as an **Confirmed Base Cell** that has been engineered to contain a genomically integrated component F, but wherein the cell has not been tested to confirm that the cell can elicit a signal response via component F
- Step i is confirming and selecting an **A Base Cell+F,** defined as a **Putative Base Cell+F** that has been tested a confirmed capable of eliciting a signal response via component F by stimulation with a cognate antigen of a complementary pair of TCR chains. Optionally, the genomic location and copy number of component F is determined
- Step j is engineering and selecting an an **Putative eTPC+F,** defined as a **Base Cell+F** that has been engineered to contain a genomically integrated component B and/or component D, but wherein the exact number of copies and location have not been determined
- Step g is confirming and selecting an **eTPC+F,** defined as an **Putative eTPC+F** that has been confirmed to have only a single copy of each component B and/or D. Optionally, the component B and/or component D are tested and capable of integrating at least one ORF encoded within component C' and/or E'.

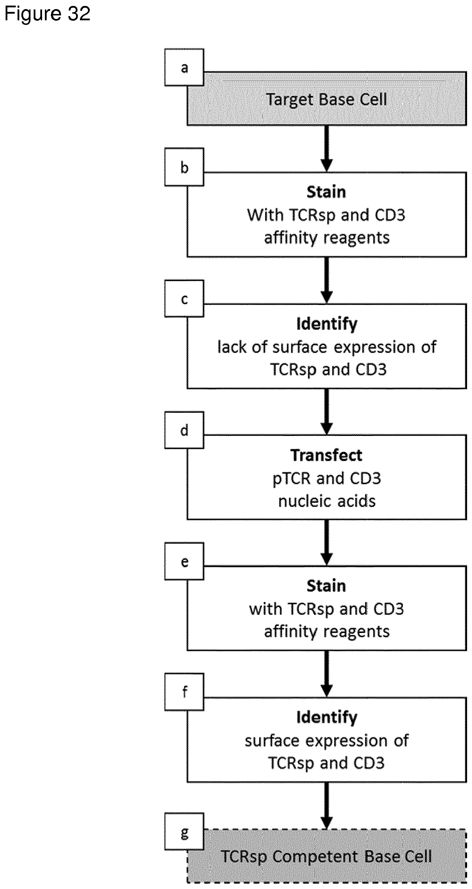

### Figure 32 - A method to identify and select a TCRsp Competent Base Cell

A method to identify and select a Target Base Cell that is competent at expressing a pair of TCR chains in complex with CD3 (TCRsp) on the surface of the cell, designated a TCRsp Competent Base Cell, as the first process in engineering an eTPC
- Step a is selecting a Target Base Cell
- Step b is staining with TCRsp and CD3 specific affinity reagents
- Step c is identifying if the cell lacks surface expression of TCRsp and CD3
- Step d is transfecting of the cell with nucleic acids encoding expression of a pair of complementary TCR chains (pTCR) and CD3 proteins
- Step e is staining of the cell with TCRsp and CD3 specific affinity reagents
- Step f is identifying the cell with surface expression of TCRsp and CD3 to confirm the cell is competent of TCRsp expression
- Step g is selecting the identified cell in step f as a **TCRsp Competent Base Cell**

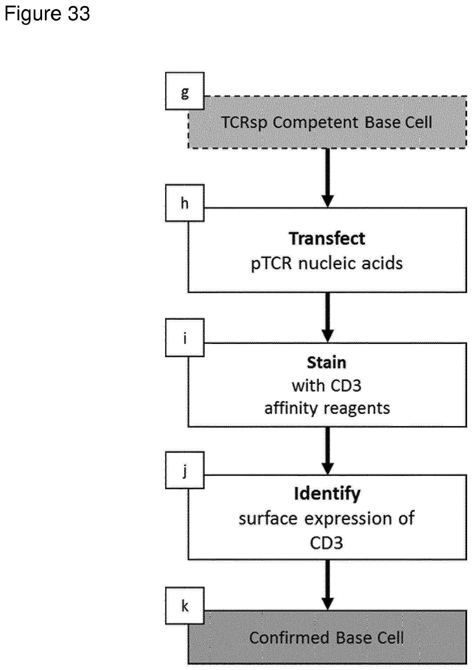

### Figure 33 - A First Method to Identify and Select a Confirmed Base Cell from a TCRsp Competent Base Cell

A first method by which a **TCRsp Competent Base Cell** can be identified and selected as a **Confirmed Base Cell** wherein a **Confirmed Base Cell** is a cell capable of TCRsp and also genomically encodes expression of CD3.

Wherein the method comprises
- Step g is selecting the **TCRsp Competent Base Cell** (from Figure 31)
- Step h is transfection of the cell with nucleic acids encoding expression of a pair of complementary TCR chains (pTCR)
- Step i is staining of the cell with CD3 specific affinity reagents
- Step j is identifying the cell with surface expression of CD3, confirming the cell is capable of expressing CD3 when provided with nucleic acids encoding a TCPsp
- Step k is selecting the identified cell as defined in step j, as a **Confirmed Base Cell**

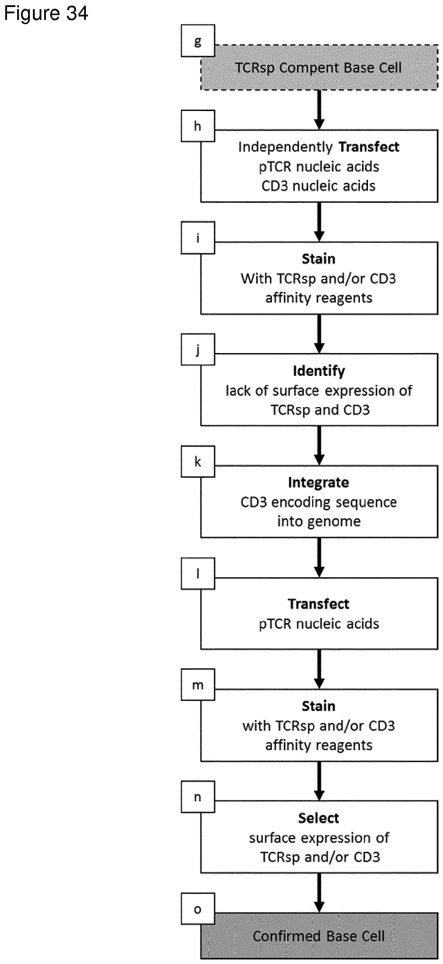

### Figure 34 - A Second method to identify, engineer and select a Confirmed Base Cell from a TCRsp Competent Base Cell

A second method by which a TCRsp Competent Base Cell can be identified, engineered and selected as a Confirmed Base Cell wherein a Confirmed Base Cell is a cell capable of TCRsp and also genomically encodes expression of CD3.

Wherein the method comprises
- Step g is selecting the TCRsp Competent Base Cell (from Figure 31)
- Step h is transfection of the cell with nucleic acids encoding expression of a pair of complementary TCR chains (pTCR) and independently also transfecting with nucleic acids encoding expression of CD3
- Step i is staining of the cell with TCRsp and/or CD3 specific affinity reagents
- Step j is identifying the cell to lack TCRsp and/or CD3 expression, in both independent transfections
- Step k is integrating nucleic acids encoding expression of CD3 into the genome of the cell
- Step l is transfecting of the cell with nucleic acids encoding expression of a pair of complementary TCR chains (pTCR)
- Step m is staining of the cell with TCRsp and/or CD3 specific affinity reagents
- Step n is selecting the cell that is expressing TCRsp and CD3 on the surface of the cell
- Step o is selecting the engineered cell of step n as a **Confirmed Base Cell**

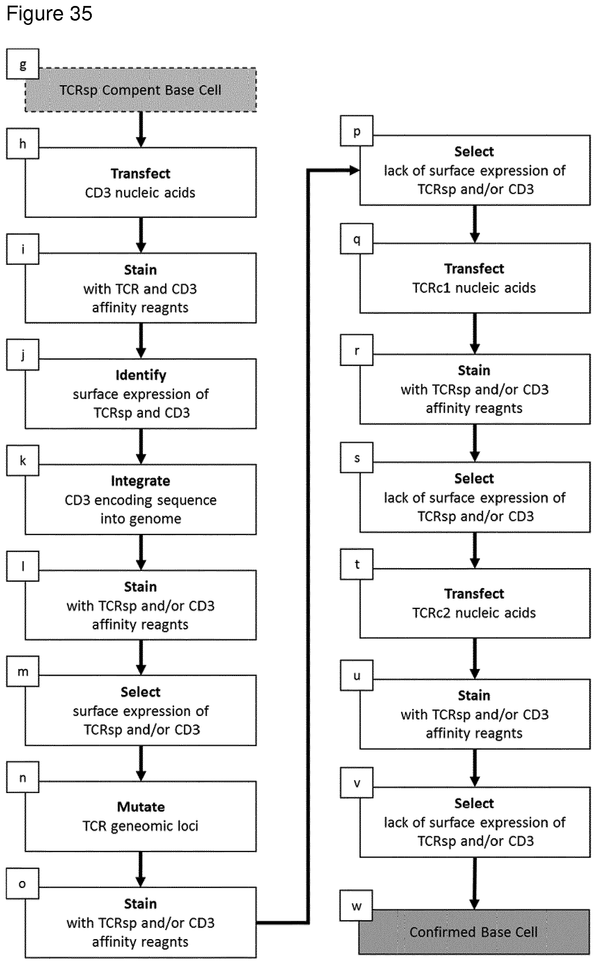

### Figure 35 - A third method to identify, engineer and select a Confirmed Base Cell from a TCRsp Competent Base Cell

A third method by which a TCRsp Competent Base Cell can be identified, engineered and selected as a Confirmed Base Cell wherein a Confirmed Base Cell is a cell capable of TCRsp and also genomically encodes expression of CD3.

Wherein the method comprises
- Step g is selecting the TCRsp Competent Base Cell (from Figure 31)
- Step h is transfection of the cell with nucleic acids encoding expression of CD3
- Step i is staining of the cell with TCRsp and CD3 specific affinity reagents
- Step j is identifying the cell with surface expression of TCRsp and CD3
- Step k is integrating nucleic acids encoding expression of CD3 into the genomic of the cell
- Step l is staining of the cell with TCRsp and/or CD3 specific affinity reagents
- Step m is selecting the cell that is expressing TCRsp and/or CD3 on the surface of the cell
- Step n is targeted mutation of the TCR genomic loci
- Step o is staining of the cell with TCRsp and/or CD3 specific affinity reagents
- Step p is selecting the cell that lacks expression of TCRsp and/or CD3 on the surface of the cell
- Step q is transfection of the cell with nucleic acids encoding expression of a single chain of a complementary pair of TCR chains (TCRc1)
- Step r is staining of the cell with TCRsp and/or CD3 specific affinity reagents
- Step s is selecting the cell that lacks expression of TCRsp and/or CD3 on the surface of the cell
- Step t is transfection of the cell with nucleic acids encoding expression of a single chain of a complementary pair of TCR chains (TCRc2) , which is the cognate pair of TCRc1 in step q
- Step u is staining of the cell with TCRsp and/or CD3 specific affinity reagents
- Step v is selecting the cell that lacks expression of TCRsp and/or CD3 on the surface of the cell
- Step w is selecting the engineered cell of step v as a Confirmed Base Cell

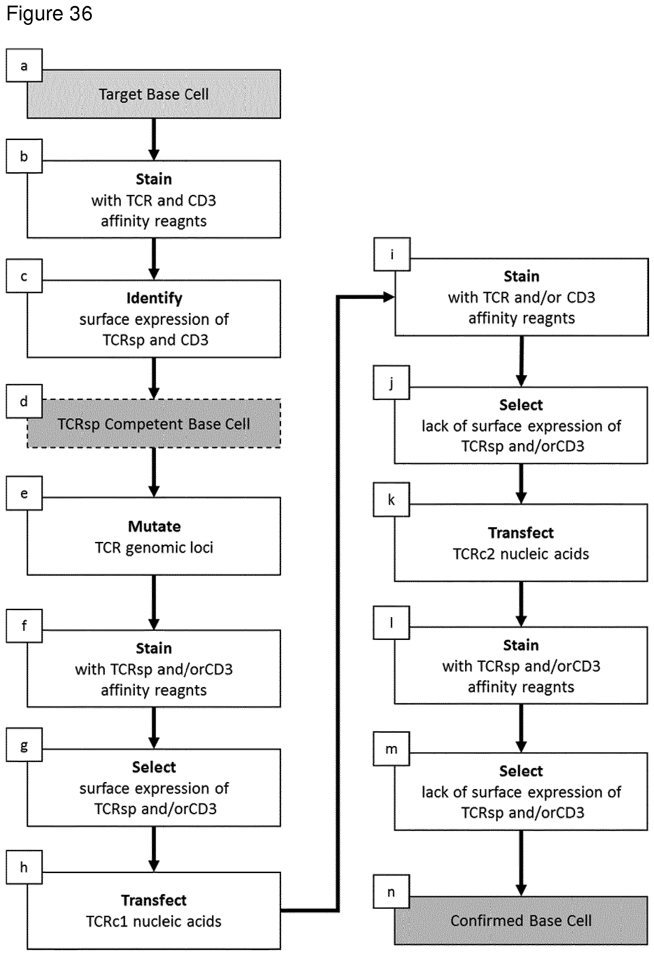

### Figure 36 - A method to Identify, Engineer, and Select a Confirmed Base cell from a Target Base Cell

A method by which a Target Base Cell can be identified, engineered and selected as a Confirmed Base Cell, and wherein the Confirmed Base Cell is a cell capable of TCRsp and also genomically encodes expression of CD3. Selection of a Confirmed Base Cell is the second process in engineering an eTPC.

The method comprises
- Step a is selecting a Target Base Cell
- Step b is staining with TCRsp and CD3 specific affinity reagents
- Step c is identifying the surface expression of TCRsp and CD3
- Step d is selecting the TCRsp Competent Base Cell as determined in Step c
- Step e is targeted mutation of the TCR genomic loci
- Step f is staining of the cell with TCRsp and CD3 specific affinity reagents
- Step g is selecting the cell that lacks expression of TCRsp and CD3 on the surface of the cell
- Step h is transfection of the cell with nucleic acids encoding expression of a single chain of a complementary pair of TCR chains (TCRc1)
- Step i is staining of the cell with TCRsp and CD3 specific affinity reagents
- Step j is selecting the cell that lacks expression of TCRsp and CD3 on the surface of the cell
- Step k is transfection of the cell with nucleic acids encoding expression of a single chain of a complementary pair of TCR chains (TCRc2) , which is the cognate pair of TCRc1 in step q
- Step l is staining of the cell with TCRsp and CD3 specific affinity reagents
- Step m is selecting the cell that lacks expression of TCRsp and CD3 on the surface of the cell
- Step n is selecting the engineered cell of step v as a Confirmed Base Cell

### Figure 37 - A method to engineer and select an eTPC or an eTPC+F from a Confirmed Base Cell

A method to engineer and select an eTPC, wherein a **Confirmed Base Cell** is engineered to contain a genomically integrated component B and/or component D to obtain an **Putative eTPC,** and wherein the **Putative eTPC** is then confirmed to have only a single copy of each component B and/or D. Optionally, the component B and/or component D are tested and found capable of integrating at least one ORF encoded within component C' and/or E'

The method comprises
- Step a is selecting a Confirmed Base Cell, using any of methods defined in Figures 32 to 36
- Step b is integrating component B and/or component B into the genome of the cell
- Step c is selecting a cell with integrated component B and/or C by selecting for expression of component B and/or component D selection marker(s)
- Step d is obtaining a Putative eTPC according to step c
- Step e is confirming component B and/or D the location within the genome and that only a single copy of each is present within the genome
- Step f is optionally transfecting the cell with component C' and/or E' in conjunction with integration factors
- Step g is optionally staining of the cell with TCRsp and/or CD3 specific affinity reagents
- Step h is optionally identifying the cell with surface expression of TCRsp and/or CD3
- Step i is optionally identifying the loss of component B and/or D selection marker(s)
- Step j is optionally identifying the gain of component C' and/or component E' selection marker of integration
- Step k is confirmation of the Putative eTPC as an eTPC, wherein the confirmation is determined by step e, and/or any of step f, i, j and/or g and h

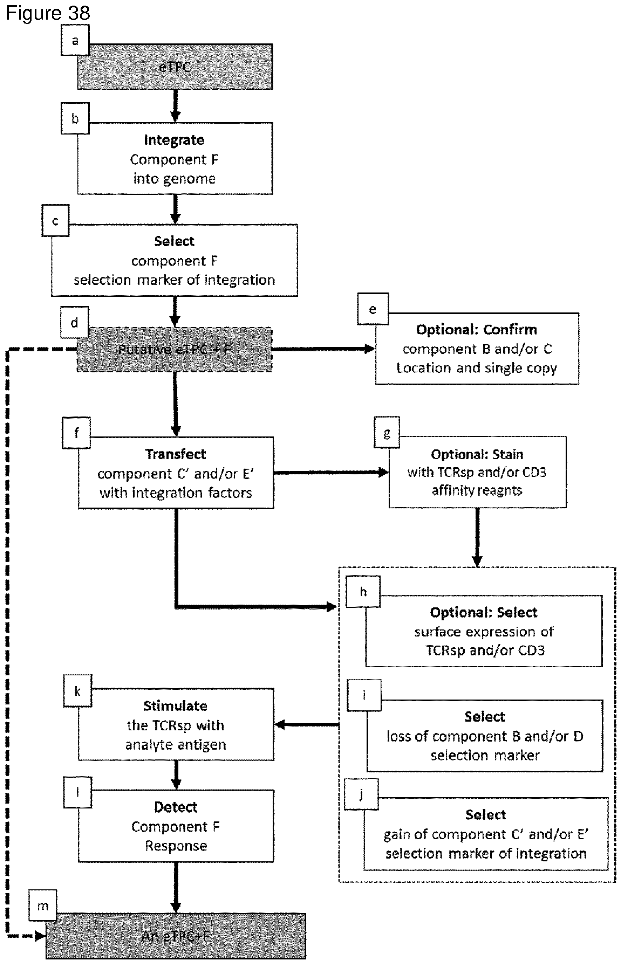

The dashed arrow from step d to step k indicates that the Putative eTPC is confirmed to be an eTPC by at the minimum confirmation as defined in step e. Dashed arrow from step e to f, indicates that this process is optional, and the dotted box outlining steps h, i, and j indicate at least one of h, i, or j must be conducted. The dashed line from the dotted box to step k, indicates further confirmation and characterisation of the eTPC, as determined in steps h, i, and/or j The method may also substitute step a, step d and step k with the following to obtain an **eTPC+F**
- Step a is selecting a **A Base Cell+F,** using the substituted method defined in Figures 38
- Step d is obtaining a **Putative eTPC+F** according to step c
- Step k is confirmation of the Putative **eTPC+F** as an **eTPC+F,** wherein the confirmation is determined by step e, and/or any of step f, i, j and/or g and h

### Figure 38 - A method engineer and select an eTPC+F or eTPC from a Confirmed Base Cell

A method to engineer and select an **eTPC+F,** wherein a **eTPC** is engineered to contain a genomically integrated component F to obtain an **Putative eTPC+F,** and wherein the **Putative eTPC+F** is then tested to confirm that cell and component F is capable of eliciting a signal response via component F by stimulation with a cognate antigen of a complementary pair of TCR chains. Optionally, the genomic location and copy number of component F is determined wherein
The method comprises
- Step a is selecting a **eTPC,** using the method defined in Figures 37
- Step b is integrating component F into the genome of the cell
- Step c is selecting a cell with integrated component F by selecting for expression of component F selection marker of integration
- Step d is obtaining a **Putative eTPC+F** according to step c
- Step e is optionally confirming component F location within the genome and number of copies present within the genome of the cell
- Step f is transfecting the cell with component C' and/or E' in conjunction with integration factors
- Step g is optionally staining of the cell with TCRsp and/or CD3 specific affinity reagents
- Step h is optionally selecting the cell with surface expression of TCRsp and/or CD3
- Step i is selecting the loss of component B and/or D selection marker(s)
- Step j is selecting the gain of component C' and/or component E' selection marker of integration
- Step k is stimulating the TCRsp of the selected cell, as selected by at least one of step h, i, and/or j, with the cognate antigen to the TCRsp
- Step l detecting a signal response induced by step k stimulation, wherein the response is an reported by component F
- Step m is confirmation of the **Putative eTPC+F** as an **eTPC+F,** wherein the confirmation is determined by step l, and optionally step e

The dashed arrow from step d to step m indicates that the **Putative eTPC+F** is confirmed to be an **eTPC+F** by at the minimum confirmation as defined in step l. The dotted box outlining steps h, i, and j indicates at least one of h, i, orj must be conducted in order to select a cell for step k

The method may also **substitute** step a, d, f, and m with the following
- Step a is selecting a **Confirmed Base Cell,** using any of methods defined in Figures 32 to 36
- Step d is obtaining a **A Putative Base Cell+F** according to step c
- Step f is transiently transfecting nucleic acids encoding expression of a complementary pair of TCR chains
- Step m is confirmation of the **Putative Base Cell+F** as a **Base Cell+F,** wherein the confirmation is determined by step l, and optionally step e

### Figure 39 - FACS analysis of Target Base Cell, wherein the cells had been stained with anti-TCR-alpha-beta and anti-CD3.

The plots clearly demonstrate that the Target Base Cell lacks surface expression of CD3 and TCRsp. There is no-significant difference in fluorescence intensity between the stained (Black Line, grey in-fill) and unstained (Dashed Line, unfilled).

### Figure 40 - FACS analysis of Target Base Cell

Cellswere transfected with plasmids encoding transient expression of CD3 and pTCR, and subsequently stained with anti-TCR-alpha-beta and anti-CD3 and dexA2-NLV-PE, a the soluble antigen (an aAPX:aM). Two different pTCR were used, the first pTCR (A) is known to be incapable of binding to dexA2-NLV-PE, whereas the second pTCR (B) is known to bind dexA2-NLV-PE as a cognate antigen. As a control, the Target Base Cell had been transfected with a plasmid that encoded no expression, designated an empty vector control (C). All plots have the anti-CD3 fluorescence intensity as the y-axis, and the left-hand panels the x-axis as anti-TCR-alpha-beta fluorescence intensity, and the right-hand panels the x-axis as dexA2-NLV-PE fluorescence intensity. The value inside each plot, in the upper-right corner is the percentage of cells positive for CD3+TCRsp+ or CD3+dexA2-NLV-PE+.

The plots clearly demonstrate that the Target Base Cell is capable of expressing CD3 and TCRsp when provided with the nucleic acids encoding the expression of CD3 and TCRsp. In addition, the TCRsp encoded by the pTCR with known binding to dexA2-NLV-PE (B) exhibits clear binding to the soluble antigen, indicating the TCRsp retains its biological function. In contrast, the TCRsp (B) that is known incapable of binding dexA2-NLV-PE, exhibited no binding.

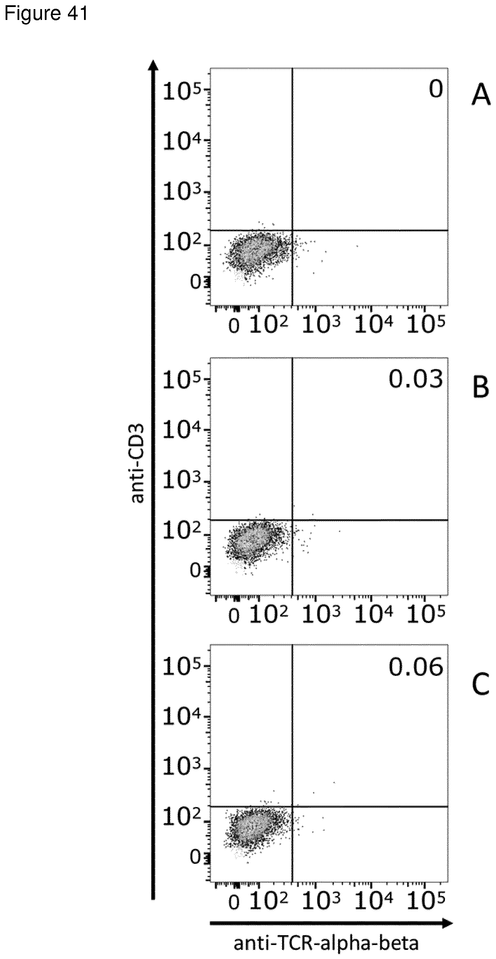

### Figure 41 - Identification of lack of surface expression of CD3 and TCRsp of a TCRsp Competent Base Cell

Identification of lack of surface expression of CD3 and TCRsp of a TCRsp Competent Base Cell that had been transfected with plasmids encoding expression of either TCR-alpha (A), TCR-beta (B) or empty vector control (C) . No significant difference was observed in the fluorescence intensity for the cells that had been transfected with TCRalpha or TCRbeta plasmids compared to the empty vector control, indicating a lack of endogenous expression of any complementary TCR chain. In addition, no surface expression of CD3 was exhibited, further confirming that the cell did not express endogenous CD3 or any TCR chains.

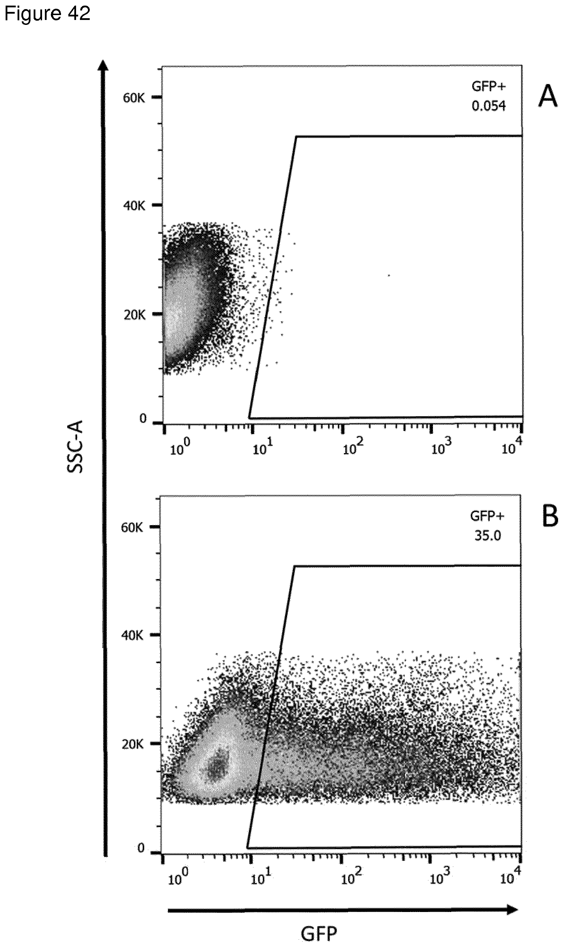

### Figure 42 - Transient transfection of the TCRsp Competent Base Cell to integrate CD3

Transient transfection of the TCRsp Competent Base Cell to integrate CD3 into the genome. The TCRsp Competent Base Cell had been transfected with two plasmids that encoded expression of the two integration factors, Cas9-2A-GFP and guideRNA, in addition to a third plasmid which encoded the CD3 in an site directed homologous recombination plasmid, targeted to the HLA-A loci. A control transfection had been conducted wherein empty vector that encoded no expression, as a comparison. Cells exhibiting GFP expression, and subsequently contained the integrated cell population, were selected by cell sorting.

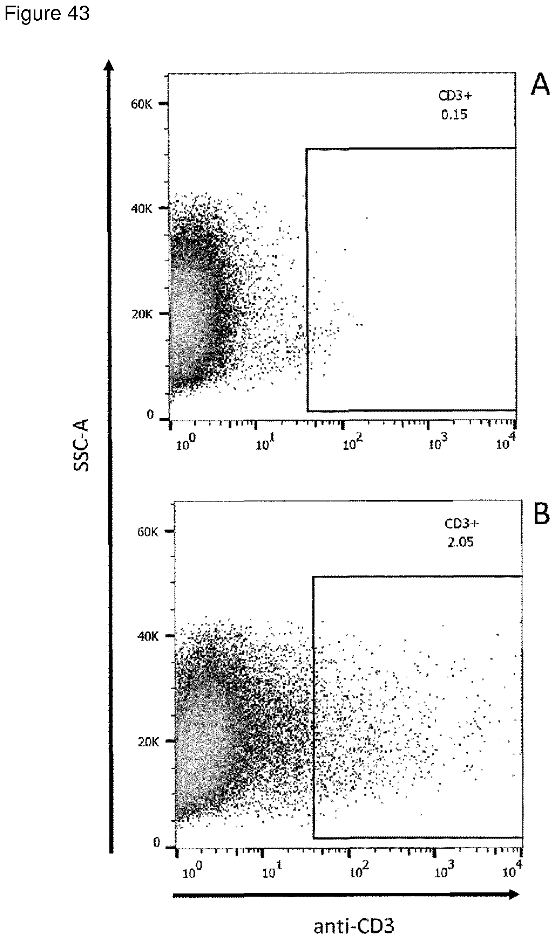

### Figure 43 - Transient transfection of the sorted cells for first selection

Transient transfection of the sorted cells from Figure 42. The sorted cells had been transfected (B) with with three plasmids, the first encoded expression of GFP and was used as a transfection control. The second and third plasmids, each encoded either a TCR-alpha (V3.C.3) chain or a TCR-beta chain. A control transfection had been conducted wherein the cell was not provided with TCRsp encoding plasmids (A). Cells were stained with anti-CD3, with cells exhibiting CD3 surface expression selected and sorted.

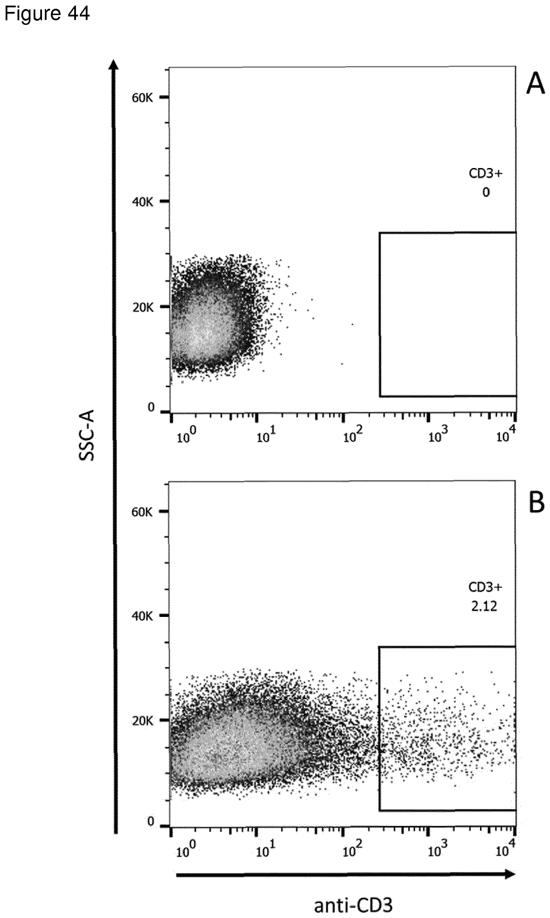

### Figure 44 - Transient transfection of the sorted cells for second selection

Transient transfection of the sorted cells from Figure 43. The sorted cells had been transfected (B) with with three plasmids, the first encoded expression of GFP and was used as a transfection control. The second and third plasmids, each encoded either a TCR-alpha (V3.C.3) chain or a TCR-beta chain. A control transfection had been conducted wherein the cell was not provided with TCRsp encoding plasmids (A). Cells were stained with anti-CD3, with cells exhibiting CD3 surface expression selected and sorted.

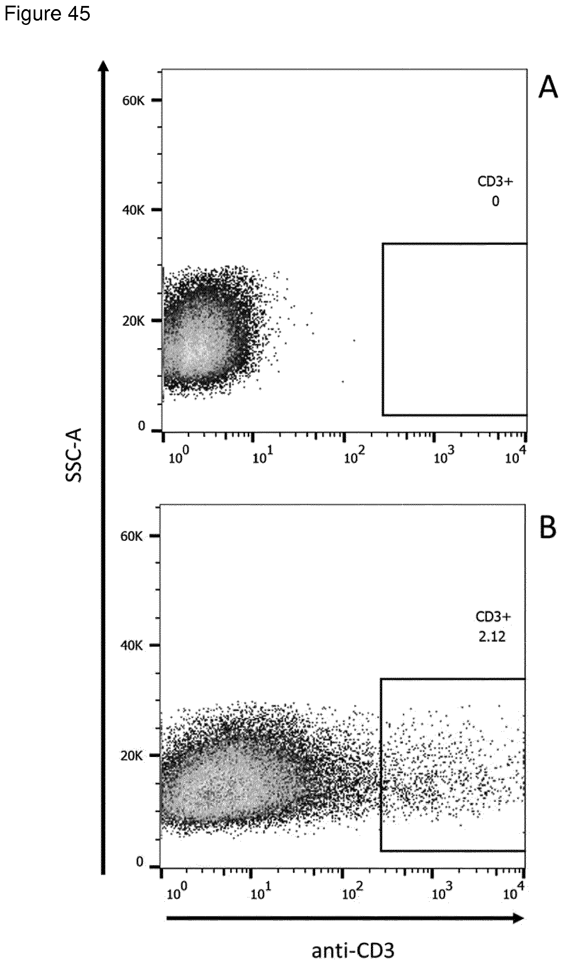

### Figure 45 - Transient transfection of the sorted cells for third selection

Transient transfection of the sorted cells from Figure 44. The sorted cells had been transfected (B) with with three plasmids, the first encoded expression of GFP and was used as a transfection control. The second and third plasmids, each encoded either a TCR-alpha (V3.C.3) chain or a TCR-beta chain. A control transfection had been conducted wherein the cell was not provided with TCRsp encoding plasmids (A). Cells were stained with anti-CD3, with 96 cells exhibiting high CD3 surface expression selected and sorted for monocloning (CD3 Monolcone selection box), with the remaining CD3+ cells sorted as a pool for cryopreservation.

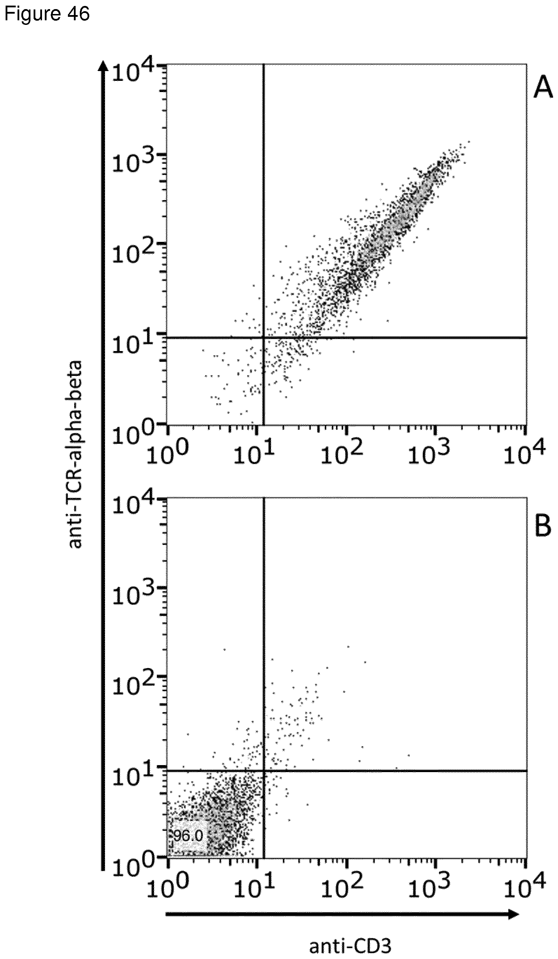

### Figure 46 - eTPC characterisation

Identification and selection of a monoclone from Figure 45 that had exhibited CD3 and TCRsp surface expression. The cell had been transiently transfected with two plasmids which encoded a complementary TCRsp and a third that encoded GFP expression (A). A control transfection had been conducted (B) wherein the cells were transfected using only the GFP plasmid. FAC analysis of the two transfections, gated for GFP+ cells, clearly indicated that the monoclone exhibited CD3 expression conditionally on provision of genetic material that encoded expression of a TCRsp. The monoclone was subsequently selected as a Confirmed Base Cell.

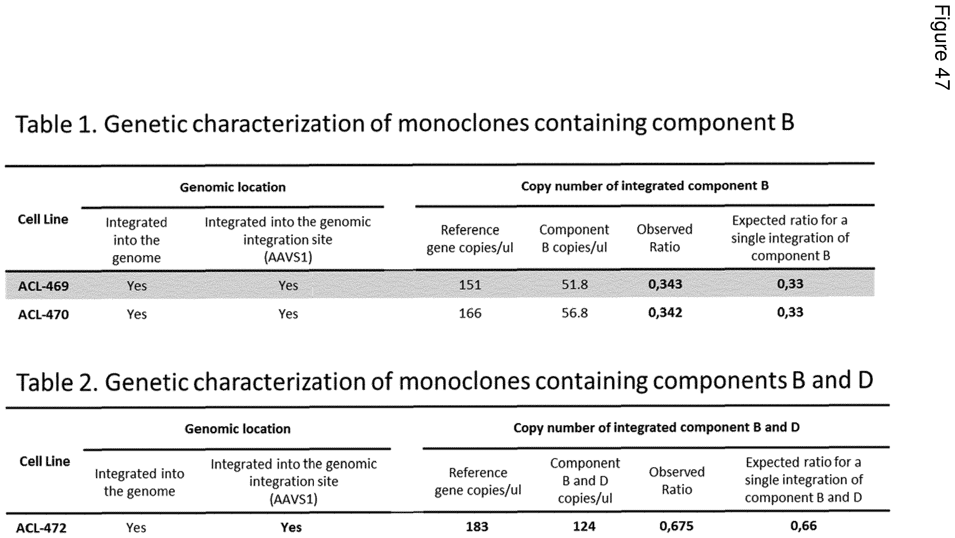

### Figure 47 - Genetic characterisation of monoclones containing components D and/or B

Two tables are presented summarising the genetic characterisation of cells generated in examples 2 and 3, to contain Component B, or Component B and D, respectively.

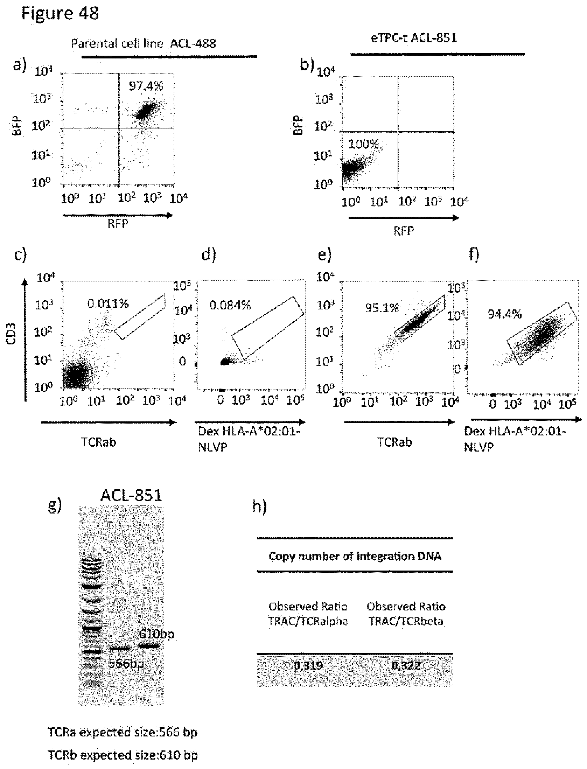

### Figure 48: Demonstration of eTPC-t generation from parental eTPC

A model TCR alpha/beta pair (JG9-TCR), which has a known specificity for a HCMV antigen presented in HLA-A*02:01 was selected for integration to an eTPC parental line. The JG9-TCR-alpha ORF was cloned in a Component E' context, and JG9-TCR-beta in a C' context. An eTPC-t was created through RMCE by transfection of component C' and E' plasmids and a construct encoding flp recombinase into the eTPC line ACL-488, which harbours two genomic integration sites, B and D, encoding reporters BFP and RFP, respectively. 10 days after transfection, individual cells diminished for the BFP and RFP signals, encoded by **Components B and D** selection markers, were sorted as single cells. Resulting monoclonal eTPC-t ACL-851 were analysed in parallel with the parental eTPC, and a single example presented.
a) and b) Parental eTPC cell line ACL-488 and an example monoclonal was analysed by flow cytometry for BFP and RFP signals. The plot displays live single cells as BFP versus RFP, showing the eTPC cell line is positive for selection markers present in component B and D (a), and resulting monoclone has lost these markers as expected for integration couple events between B/C and D/E (b). Percentage values represent the percentage of double positive cells in a) and double negative cells in b).
c) to f) eTPC ACL-488 and monoclone eTPC-t ACL-851 were stained with antibodies for CD3 and TCR alpha/beta (TCRab) and HLA multimer reagent specific for the JG9-TCR (Dex HLA-A*02:01-NLVP) and analysed by flow cytometry and gated for live single cells. The parental eTPC line showed no positive staining for CD3 or TCR on the cell surface (c), and was also negative for staining with HLA multimer reagent (d). In contrast, the resulting monoclone showed positive staining for both CD3 and TCR on the cell surface (e) and showed positive staining with the multimer reagent specific for the expressed JG9-TCR. Percentage values represent the percentage of CD3/TCRab double positive cells in c) and e), and CD3/HLA-multimer double positive cells in d) and f).
g) Genomic DNA was prepared from monoclonal eTPC-t ACL-851 and subjected to PCR with primers specific for the JG9-TCR-alpha chain encoded by component D', or the JG9-TCR-beta chain encoded by component B'. PCR products were resolved by agarose gel and observed as expected band size. h) Genomic DNA was prepared from monoclonal eTPC-t ACL-851 and subjected to digital drop PCR with primers and probes specific for the JG9-TCR-alpha chain encoded by component D', or the JG9-TCR-beta chain encoded by component B'. A reference amplicon primer/probe pair for an intron of the TCR alpha constant (TRAC) was included. The table presents ratios of reference to TCR alpha and TCR beta. A ratio of close to 0.33 indicates that a single copy of each TCR alpha and beta chain is present in the eTPC-t line ACL-851, which is a triploid line.

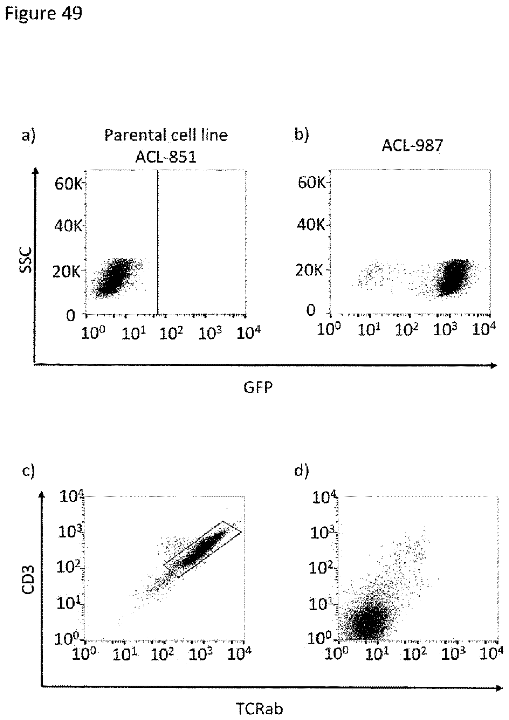

### Figure 49 - Demonstration of eTPC-x reversion from eTPC-t

A parental eTPC-t cell line ACL-851, expressing a TCR alpha and beta chain at site D' and B', respectively was reverted to a eTPC-x line by exchanging component D' with a donor vector encoding GFP (Component Z). **Component Z** contained recombinase heterospecific F14/ F15 sites flanking the GFP ORF, and was thus compatible with **Component D'.** eTPC-t line ACL-851 was transfected with Component Z along with a construct encoding flp recombinase. 7 days after transfection, individual cells positive for GFP signals were sorted and grown as monoclones. Resulting monoclonal eTPC-x lines were analysed by flow cytometry in parallel with the parental eTPC-t, and a single example presented. a) and b) The monolcone eTPC-x ACL-987 derived from parental eTPC-t ACL-851 was analysed by flow cytometry for GFP expression along with the parental line. Plots display SSC versus GFP parameters of gated live single cells. The parental cell line has no GFP expression (a), while the monoclone ACL-987 has gained GFP as expected (b), indicating exchange of the TCR alpha ORF for a GPF ORF. c) and d) The monolcone eTPC-x ACL-987 derived from parental ACL-851 along with the parental eTPC-t ACL-851 were stained with antibodies for CD3 and TCRab and analysed by flow cytometry. Plots display CD3 versus TCRab parameters gated on live single cells. The parental cell showed positive staining for both CD3 and TCRab (c), while the derived monoclone showed negative staining for both (d); confirming loss of TCR alpha ORF in the derived eTPC-x line.

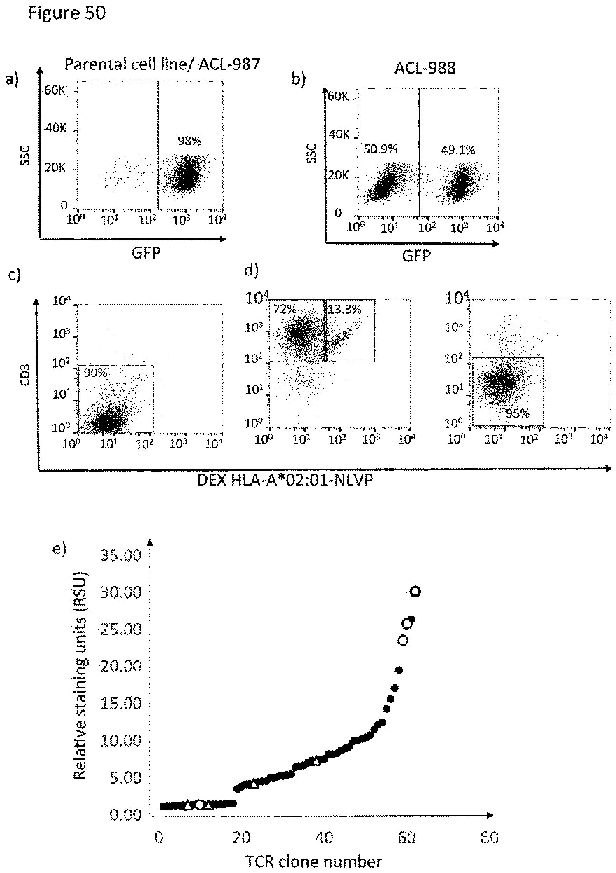

### Figure 50 - Demonstration of shotgun integration into eTPC-x to create pool of eTPC-t

An eTPC-t pool was created from an eTPC-x parental line expressing a single TCR beta chain in Component B'. The eTPC-x line expressed GFP as the reporter at available site D. A pool of 64 variant TCR alpha chains, including the parental chain, were constructed. The parental TCR chain pair represents the JG9-TCR with known specificity for a HCMV antigen presented in HLA-A*02:01. The Component E pool was transfected into the parental eTPC-x ACL-987 along with a construct encoding flp recombinase. A polyclonal line was selected by sorting for GFP positive cells 10 days after transfection. The resulting ACL-988 polyclonal eTPC-t was subsequently sorted on the basis of negative staining for GFP and positive or negative staining for HLA multimer reagent (DEX HLA-A*02:01-NLVP). Recovered single cells were sequenced to identify the encoded TCR-alpha chains and compared to a parallel analysis of each of the TCR-alpha chain variants paired with the native TCR-beta chain in terms of staining with an HLA multimer reagent specific for the parental TCR chain pair.
a) and b) Parental eTPC-x ACL-987 line and resulting polyclone eTPC-t ACL-988 line were analysed by flow cytometry for GFP expression. Plots display SSC versus GFP parameters of live single cells. Parental cell line shows positive signal for GFP, indicating intact component D (a). Derived polyclonal line shows half positive and half negative for GFP (b), indicating that half of the cells in the polyclonal population have potentially exchanged the GFP ORF at D for TCR alpha ORF to form component D'.
c) and d) Parental eTPC-x ACL-987 line and resulting polyclone eTPC-t ACL-988 line were stained with and CD3 antibody and HLA multimer with specificity for the parental JG9-TCR (DEX HLA-A*02:01-NLVP), and analysed by flow cytometry. Plots display CD3 versus HLA multimer parameters of live single cells. The parental cell line is negative for both CD3 and HLA multimer staining (c). The left hand panel of d) displays gated GFP-negative events, and the right hand GFP-positive events. Only GFP-negative events, where the component D is converted to D', shows CD3 positive staining, of which a subset shows positive staining for HLA multimer. Single cells from the gated HLA multimer negative and positive gate were sorted and the integrated ORF at component D' sequenced to determine identity of TCR alpha ORF. e) All 64 JG9-TCR-alpha variants were cloned into an expression construct that permitted each to be independently transfected to parental eTPC-x (ACL-987). Relative staining units (RSU) against the HLA-A*02:01-NLVP tetramer reagent was determined for each. RSU is calculated as the ratio of the mean fluorescence intensity (MFI) of HLA-A*02:01-NLVP tetramer signal for the CD3 positive population over the CD3 negative population, and is indicative of the binding strength of each TCR chain pair variant to the HLA multimer reagent. Each point plotted in Figure e) represents the observed RSU for each 64 variants. Open circles correlate to the sequenced cells recovered from the GFP-negative/HLA multimer-positive gate. Open triangles correlate to the sequenced cells recovered from the GFP-negative/HLA multimer-negative gate.

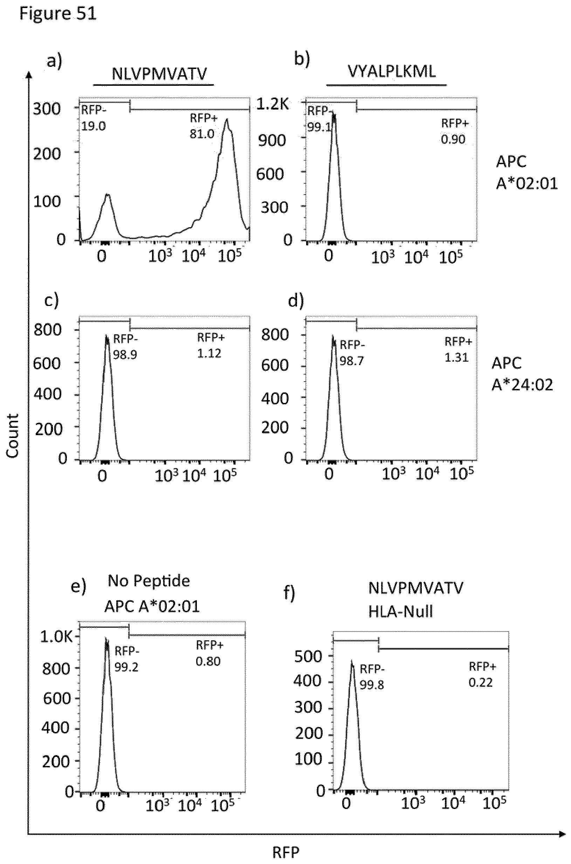

### Figure 51 - Functional demonstration of component F

The eTPC-t cell line carrying a component F (ACL-1277), wherein the TCR chains at Component B' and D' encode a TCR pair that is specific for HMCV antigenic peptide NLVPMVATV presented in HLA-A*02:01. The component F reporter was RFP. This eTPC-t was contacted for 24 hours with various APC lines of differing HLA characteristics in the presence and absence of model peptide antigens, and the contact cultures analysed by flow cytometry. Flow cytometry histogram plots show event counts against RFP signal of viable single T-cells identified by antibody staining for a specific surface marker that was not presented by the APCs. a) and b) APC cells expressing only HLA-A*02:01 (ACL-209) were pulsed with NLVPMVATV (a) or VYALPLKML (b) peptides and subsequently co-cultured with eTPC-t for 24 hrs. c) and d) APC cells expressing only HLA-A*24:02 (ACL-963) were pulsed with NLVPMVATV (c) or VYALPLKML (d) peptides and subsequently co-cultured with eTPC-t for 24 hrs. e) APC cells expressing only HLA-A*02:01 (ACL-209) were left without peptide pulsing and subsequently co-cultured with eTPC-t for 24 hrs. f) APC cells that express no HLA on the cell surface (ACL-128) were pulsed with NLVPMVATV and subsequently co-cultured with eTPC-t for 24 hrs. RFP signal was significantly increased in the eTPC-t ACL-1277 only in the presence of HLA-A*02:01 expressing cells pulsed with NLVPMVATV, representing the known target of the expressed TCR. Histogram gates and values reflect percentage of events in the RFP positive and RFP negative gates. This indicates the specific response of Component F to engagement of eTPC-t expressed TCRsp with cognate HLA/antigen (aAPX:aAM).

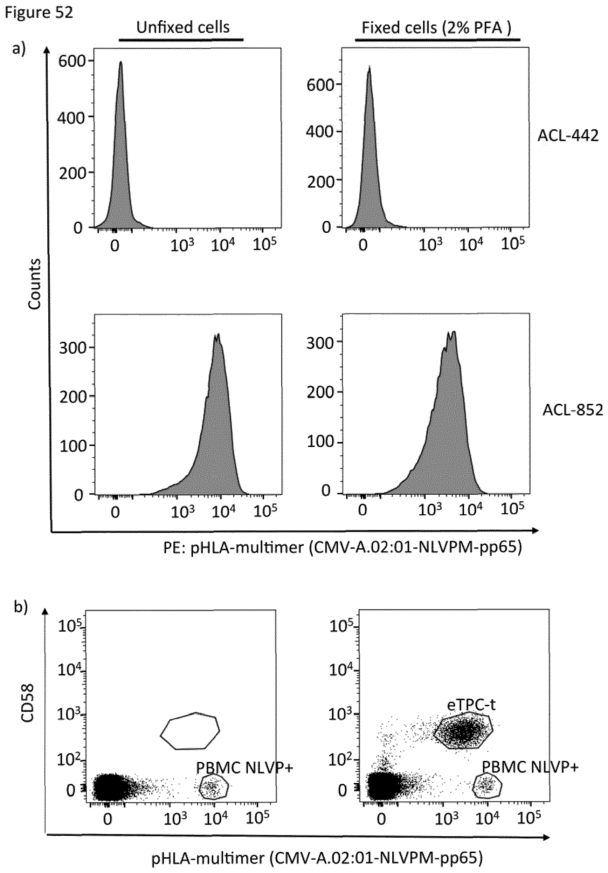

### Figure 52 - The use of eTPCs as a TCR standard

The eTPC-t cell line expressing TCR, specific to the NLVPMVATV peptide from the CMV presented in HLA-A*02:01 (ACL-852 ) was compared to an eTPC lacking TCR expression (ACL-442). Cells were harvested and left unfixed, or fixed with PFA. a) eTPC-t and eTPC cells were stained with HLA multimer reagent specific for the TCR presented by eTPC-t (ACL-852) and analysed by flow cytometry. Gated single cells are displayed as histogram plots. ACL-442 is displayed in the upper panels, whereas ACL-852 in the lower panels. Only ACL-852 cells showed staining with HLA multimer reagent, where unfixed (left panel) showed equivalent staining to fixed (right panel) cells. b) eTPC-t cells were stained with an antibody against CD58, then added to isolated PBMC. A PBMC pool without eTPC-t, and a PBMC pool with spiked in eTPC-t, were then stained with a HLA multimer reagent specific for the TCR presented by eTPC-t (ACL-852, CMV-HLA-A*02:01-NLVPM-pp65). Left panel displays PBMC alone, and right panel PBMC and eTPC-t, gated as single cells and CD58 versus HLA multimer parameters. PBMC with specific labelling (CD8 T-cells) can be seen as multimer-positive cells in both specimens, where as the eTPC-t can easily be visualised as CD58/HLA multimer double positive cells (right panel).

### Materials and methods

### Electroporation of ARH-77 cells

Per reaction, 4×10⁶ cells were electroporated in 500 ul RPMI 1640 with Glutamax-I (Life Technologies) using the Gene Pulser Xcell^{™} (Bio-Rad) with the following setting Square Wave 285V, pulse length 12.5 ms and 2 pulses with 1s interval.

The DNA concentration used for the Cas9 plasmid V1.A.8 was 10 ug/ml and 7.5 ug/ml for the gRNA targeting the integration site (V2.L10 and V2.J.1 for integration in HLA endogenous locus and V2.J.6 to target the AAVS1 site) **(Table 1).** The integration vectors were electroporated at a concentration of 7.5 µg/µl.

For HDR integration in the HLA locus, HLA class I V1.C.6 and V1.C.9 plasmids were used. For HDR integration in AAVS1 locus, HLA class I V1.F.8 and V1.F.10 and HLA class II V1.1.5 and V1.1.7.

Variants of pp65 ORF were integrated into previously created HLA monoallelic lines. Plasmids V1.G.9 and V1.H.1 containing a form of pp65 linked with a GFP marker were used for this purpose.

To generate an ARH-77 HLA-null line with one RMCE site, plasmids with heterospecific recombinase sites flanking a marker were used, V4.B.2 for RFP and V4.B.3 for BFP. The same plasmids were co-electroporated to produce a stable line containing two RMCE sites.

A monoallelic HLA line was also created using RMCE, where vector V4.D.2 was electroporated into a cell containing one RMCE site.

After electroporation, cells were incubated in culture medium RPMI 1640 with Glutamax-I + 10% FBS (37°C, 5% CO₂) for two days, before analysis.

### Transfection of HEK293 cells

One day prior to transfection, cells were seeded at a density of 1.2-1.4 ×10⁶ cells/60mm dish in 90% DMEM + 2mML-glutamine + 10% HI-FBS (Life Technologies).

The following day, cells with 65% confluency were transfected with a total amount of 5ug DNA and jetPEI ^{®} (Polyplus transfection reagent, Life Technologies) at a N/P ratio of 6. The medium was replaced before transfection.

For deletion of HLA class I genes, cells were transfected with 0.42 µg of DNA vectors encoding the Cas9_GFP (V1.A.8), gRNAs targeting HLA-A, B and C (V2.A.1, V2.A.7 and V2.B.3 respectively) and an empty vector (V1.C.2).

For integration of RMCE sites in the AAVS1 locus, cells were transfected with 0.5 µg of V1.A.8; 0.625 µg of gRNA V2.J.6 and 0.75 µg of plasmids encoding two markers flanked by RMCE sites (V4.B.2 for RFP and V4.B.3 for BFP), empty vector V1.C.2 was used to complete 5 µg of DNA.

Stock solutions of DNA and jetPEI ^{®} were diluted in sterile 1M NaCl and 150mM NaCl respectively. The final volume of each solution was equivalent to 50% of the total mix volume. The PEI solution was then added to the diluted DNA and the mixture was incubated at room temperature for 15min. Finally the DNA/PEI mixtures were added to the 60-mm dishes, being careful not to disrupt the cell film. The cells were incubated for 48 hours at (37 °C, 5% CO2, 95% relative humidity) prior to GFP expression analysis.

### RMCE between a paired integration couple

For RMCE integration, cells were transfected with 0.6 µg of DNA vectors encoding FLP, (V4.L8), 2 µg of Component C/Y, 2 µg of Component E/Z, 0.4 µg of DNA encoding a marker to track DNA delivery. 2 days after transfection cell positive for the DNA delivery marker, either GFP or RFP positive, were sorted by FACS. 4-10 days after transfection, individual cells displaying diminished fluorescent protein signal, encoded by Components D and B selection markers were sorted by FACS. The exception being for generating ACL-987 where individual cells displaying GFP positivity were sorted by FACS.

**Table 1: Vectors**

| **ID** | **Name** |
|---|---|
| V1.A.4 | pcDNA3.1_GFP |
| V1.A.8 | SpCas9-2A-GFP |
| V1.C.2 | pMA-SV40pA |
| V1.C.6 | HLA-A 02:01 6xHis + Exon2/3-HA-L+R |
| V1.C.9 | HLA-B 35:01 6xHis + Exon2/3-HA-L+R |
| V1.F.8 | AAVS1-S_A24_6xH |
| V1.F.10 | AAVS1-L_B07_6xH |
| V1.G.10 | AAVS1-1_GFP_HCMVpp65 |
| V1.H.1 | AAVS1-I_GFP_HCMVpp65 AIN |
| V1.1.5 | AAVS1_DRA_Flag-DRB1_6xHis |
| V1.1.7 | AAVS1_DPA1_Flag-DPB1_6xHis |
| V2.A.1 | HLA-A-sg-sp-opti1 |
| V2.A.7 | HLA-B-sg-sp-3 |
| V2.B.3 | HLA-C-sg-sp-4 |
| V2.I.10 | HLA-A-ex2-3_sg-sp-opti_1 |
| V2.J.1 | HLA-A-ex2-3_sg-sp-opti_2 |
| V2.J.6 | AAVSI_sg-sp-opti_3 |
| V4.B.2 | AAVS_Efla-intron_F14_RFPnls_F15 |
| V4.B.3 | AAVS_Efla-intron_FRT_BFPnls_F3 |
| V4.D.2 | pMA_FRT_HLA-A*02:01-6xHis_F3 |
| V4.I.8 | CMVpro-Flpo-sv40pA-V2 |
| V1.A.4 | pcDNA3.1_GFP |
| V1.A.6 | pcDNA3.1_RFP |
| V3.C.5 | pMA-CS-JG9-TCRbeta |
| V4.H9 | pMA-F14-GFP-F15 |
| V7.A.3 | pMA-F14-TCR-JG9-alpha-F15 |
| V7.A.4 | pMA-FRT-TCR-JG9-beta-F3 |
| V8.F.8 | F14-TCRaF15 CDR3degen.64mix |
| VP.7751.RC1 (A1->H8 | 64 individual vectors, JG9-TRA CDR3 64 variants set |

### Transient expression of TCR chain pairs to characterize their relative staining unit (RSU)

For transient expression, cells were transfected with DNA vectors encoding FLP, (V4.I.8), JG9-TCR-alpha variant (VP.7751.RC1.A1 to VP.7751.RC1.H8), JG9-TCR-beta WT chain (V3.C.5), and DNA vector vehicle (V1.C.2) . 2 days after transfection, all cells were stained with HLA-A*02:01-NLVP tetramer and anti-CD3 antibodies. RSU were calculated as the ratio of the mean fluorescence intensity (MFI) of HLA-A*02:01-NLVP tetramer signal for the CD3 positive population over the CD3 negative population, and was indicative of the binding strength of each TCR chain pair variant.

### Fluorescence activated cell sorting (FACS)

Cells electroporated or transfected with Cas9-P2A-GFP (V1.A.8) or with a plasmid encoding a GFP selection marker (V1.A.4) were sorted for transient GFP expression, using the FACSJAzz^{™} cell sorter (BD Biosciences).

HEK 293 cells were harvested with TrypLE^{™} Express Trypsin (ThermoFisher Scientific) and resuspended in a suitable volume of DPBS 1X (Life Technologies) prior to cell sorting, in DMEM 1X medium containing 20% HI-FBS and Anti-Anti 100X (Life Technologies).

ARH-77 cells were washed and resuspended in an adequate volume of DPBS before sorting in RPMI 1640 with Glutamax-I with 20% HI-FBS and Anti-Anti 100X (Life Technologies).

### Genomic DNA extraction for genetic characterization

DNA was extracted from 5×10⁶ cells using the QIAamp DNA Minikit (Qiagen). DNA was stored in 1xTE (10mM Tris pH8.0 and 0.1mM EDTA).

### Confirmation of integration in correct genomic location

Monoclones with desired phenotypic characteristics were screened and assessed at a molecular level, this was done by PCR using Q5^{®} Hot Start High-Fidelity DNA Polymerase (NBE), in 20 µl reactions, using the components and volumes recommended by the manufacturer.

To determine whether HLA I ORFs were integrated in the HLA locus, primers 9.C.4 and 9.D.6 were used; correct right homologous arm recombination was indicated by 1 kb amplicons **(table 2).**

For HLA integration in the AAVS1 locus, four sets of primers were used: 9.C.3 and 9.C.8 to assess correct left homologous arm recombination (1.1 kb), 9.C.4 and 9.D. 1 to assess right homologous arm recombination (660 bp), 1.C.5 and 9.C.5 to amplify the CMV promoter of the internal construct (810 bp), 1.C.2 and 9.C.10 to obtain an amplicon for the SV40pA terminator of the internal construct (380 bp). Assessment of RMCE site integration in HEK293 and ARH-77 HLA-null lines was done using primer sets 2 and 4.

To confirm HLA class I deletion in HEK293 cells, specific HLA primers were used as follows: 4.A.3 and 4.A.4 targeting HLA-A, 4.A.7 and 4.B.1 for HLA-B and 4.B.5 and 8.A.1 for HLA-C. Initially, a PCR Master Mix was prepared with all components (Q5^{®} Reaction Buffer, dNTPs, Hot-Start Q5^{®} DNA polymerase, primers Fwd and Rev, 100 ng of DNA template and H₂0). PCR reactions were run using C1000 Touch^{™} Thermal Cycler (Bio-Rad). PCR products were run on a 1% Agarose gel in 1XTAE buffer, using the PowerPac Basic (Bio-Rad), stained with 10,000 dilution of sybersafe and analyzed with Fusion SL (Vilber Lourmat).

**Table 2: Primers**

| **ID** | **Name** | **Sequence** |
|---|---|---|
| 1.C.2 | pMA-sv40_OE_F1 | CCTGATCATAATCAAGCCATATCAC |
| 1.C.3 | pMA-sv40_OE_R1 | GTGATATGGCTTGATTATGATCAGG |
| 1.C.5 | pMA-CMV_OE_R1 | GTACCTGATCTGACGGTTCAC |
| 4.A.3 | HLA-A-GT-Rg3 | TCCCGTTCTCCAGGTATCTG |
| 4.A.4 | HLA-A-GT-Fg2 | GTGTCGGGTTTCCAGAGAAG |
| 4.A.7 | HLA-B-GT-Fg2 | GGGTCCCAGTTCTAAAGTCC |
| 4.B.1 | HLA-B-GT-Rg2 | GGGGATTTTGGCCTCAACTG |
| 4.B.5 | HLA-C-GT-Fg2 | TCTTCCTGAATACTCATGACG |
| 4.I.9 | HLA-A-02_GT_Rg4 | GGAGATCTACAGGCGATCAG |
| 6.I.9 | HLA-A-Exon3_HA-RE-BgIII_F1 | GGTTAGATCTGGGAAGGAGACGCTGCAG |
| 8.A.1 | HLA-C-04-GT-Rg1 | GATCCCATTTTCCTCCCCTC |
| 8.B.2 | CMV-pA-HLA-Ex3_Probe_F1 | ATGTCTGGATCTGCGGATCAGCGCACG |
| 9.C.3 | CMV-pro_GT_R1 | ATGGGCTATGAACTAATGACC |
| 9.C.4 | sv40pA_GT_F1 | CATTCTAGTTGTGGTTTGTCC |
| 9.C.5 | AAVS1_GT_F1 | CTTACCTCTCTAGTCTGTGC |
| 9.C.7 | AAVS1_GT_F3 | CCATTGTCACTTTGCGCTG |
| 9.C.8 | AAVS1_GT_F4 | TCCTGGACTTTGTCTCCTTC |
| 9.C.10 | AAVS1_GT_R2 | AGAGATGGCTCCAGGAAATG |
| 9.D.1 | AAVS1_GT_R3 | AAGAGAAAGGGAGTAGAGGC |
| 9.D.2 | AAVS1_GT_R4 | CCCGAAGAGTGAGTTTGC |
| 9.D.6 | HLA-A-intron4_GT_R1 | GCTAAAGGTCAGAGAGGCTC |
| 9.D.7 | sv40pA-GT-F2 | CTGCATTCTAGTTGTGGTTTGTC |
| 9.D.9 | AAVS1_GT_R6 | TAGGAAGGAGGAGGCCTAAG |
| 9.J.2 | sv40pA-AAVS1-probe-FAM-F1 | TGCGGATCAGGATTGGTGACAGAA |
| 10.A.9 | TRAC_TCRA-ex1_R1 | GACTTGTCACTGGATTTAGAGTCTCT |
| 10.A.10 | TRAC_TCRA-promoter_F1 | CTGATCCTCTTGTCCCACAGATA |
| 10.B.6 | TRAC_probe (HEX) | ATCCAGAACCCTGACCCTGCCG |
| 1.F.7 | TRAC-GT-F1 | ATGTGCAAACGCCTTCAAC |
| 15.H.2 | sv40pA GT-R1 | CCTCTACAGATGTGATATGGCTTG |
| 1.F.9 | TRBC2-GT-F1 | GCTGTCAAGTCCAGTTCTACG |
| 3.J.5 | tGFP-Cterm-Out_SQ_F1 | GAAGAGGACCACAGCAACAC |

### Sorting polyclonal and monoclonal cells with stable expression of component of interest

To obtain a population of cells constitutively expressing the integrated protein or marker, cells were sorted 7 to 15 days after the first GFP+ selection.

For cells expected to express a surface protein, antibody staining was performed prior to sorting. For HLA class I genes, PE-Cy^{™}5 Mouse Anti-Human HLA-ABC antibody (BD Biosciences) was used. Staining of HLA-DR and HLA-DP was done with Alexa FluorO 647 Mouse Anti-Human HLA-DR, DP, DQ (BD Biosciences).

In the case of HEK 293 derived cell lines, cells were harvested with TrypLE^{™} Express Trypsin (ThermoFisher Scientific) and washed in a suitable volume of DPBS 1X (Life Technologies) prior to cell sorting in DMEM 1X medium containing 20% HI-FBS and Anti-Anti 100X (Life Technologies).

ARH-77 derived cell lines were washed in an adequate volume of DPBS before sorting in RPMI 1640 with Glutamax-I with 20% HI-FBS and Anti-Anti 100X (Life Technologies).

For HLA knockout or integration, selection of cells was done based on loss or gain of HLA expression, respectively. Cells with integrated RMCE sites were sorted based on the expression of BFP and RFP markers, and HLA monoclones with integrated pp65 mutants were sorted for GFP expression **(Table 3).**

Monoclonal sorting of cells expressing the gene of interest was done in 96-well plates, containing 200 µl of growth medium. One to two plates was sorted per sample. Polyclonal sorting of the remaining cells was done immediately after, in FACS tubes, using the Two-way sorting setting in the cell sorter Influx^{™} (BD Biosciences).

### Phenotypic screening of monoclonal populations

A 20,000 cells of the out grown monoclones population was transferred into microtiter plates for analysis, cells were resuspended in 250 µl of DPBS 1X (Life Technologies) and analyzed on the LRSFortessa^{™} (BD Biosciences).

BFP and RFP expression was detected using the PMTs for BV421 and PE-Texas Red fluorochromes, respectively.

**Table 3: FACSJazz and Influx filters**

| **Protein** | **Fluorochrome** | **Filter** |
|---|---|---|
| Cas9/GFP | GFP | 488-513/17 |
| Cas9/GFP | GFP | 488-530/40 |
| HLA-A, B, C | PE-Cy5 | 561-670/30 |
| HLA-DR,DP,DQ | Alexa 647 | 640-670/30 |
| BFP | BFP | 405-460/50 |
| RFP | RFP | 561-585/29 |
| TCRab (R63) | APC | 640-670/30 |
| CD3 (R78) | APC-H7 | 640-750LP |
| CD3 (R71) | APC | 640-760/30 |
| DEX HLA-A*02:01-NLVP | PE | 561-585/29 |
| Anti-CD8 | BV510 | 405-525/50 |
| Anti-CD3 | FITC Alexa fluor | 488-530/30 |
| Anti-CD58 | 647 | 640-670/30 |
| CMV-A.02:01-NLVPM-pp65 | PE | 561-585/15 |

For proteins with surface expression, cells were first stained using PE-Cy^{™}5 Mouse Anti-Human HLA-ABC antibody (BD Biosciences) or Alexa Fluor^{®} 647 Mouse Anti-Human HLA-DR, DP, DQ (BD Biosciences). Staining solution was prepared using the recommended antibody volume diluted in 100 µl of staining buffer (DPBS +2% FBS). Cells were incubated for 1 hour at 4°C and then washed twice with 500 µl of staining buffer, prior to analysis.

Selected monoclones were maintained in normal growth medium. HEK239 cells grow in DMEM+ 2mML-glutamine+10% HI-FBS (Life Technologies) and ARH-7 cells grow in RPMI 1640 with Glutamax-I + 10% HI-FBS.

The confluence of cells was monitored every day, until they reached 10-12×10⁶. DNA was extracted from 5×10⁶ cells using the QIAamp DNA Minikit (Qiagen) The remaining cells were further expanded and cryopreserved at a density of 3×10⁶ cells/ml, in 70% growth medium + 20% HI-FBS + 10% DMSO.

### Sorting of eTPC-t cells

Single cell sorting or polyclone sorting was achieved through standard cell sorting methodologies using a *BDInflux* instrument. Briefly, ACL cells were harvested with Try-pLE^{™} Express Trypsin (ThermoFisher Scientific) and resuspended in a suitable volume of DPBS 1X (Life Technologies) prior to cell sorting, in DMEM 1X medium containing 20% HI-FBS and Anti-Anti 100X (Life Technologies).

Cells were stained with HLA-multimer reagent on ice for 10 mins, then with CD3 and/or TCRab antibodies. Detection of specific cell fluorescent properties by the *BDInflux* instrument are defined in table 3.

Sorting of single cells for monoclonal generation, the cells displaying the phenotype interest were deposited into 96-well plates, containing 200 ul of growth medium. One to two plates were sorted per sample. Polyclonal cell sorts were directed into FACS tubes, containing media, using the Two-way sorting setting in the cell sorter Influx^{™} (BD Biosciences).

Single cells sorts for molecular characterization of their JG9-TCR-alpha variant were sorted to PCR plate pre-loaded with 5 µL of nuclease-free water. Specimens were snap-frozen until subsequent processing.

### Genomic DNA extraction for genetic characterization

DNA was extracted from 5×10⁶ cells using the QIAamp DNA Minikit (Qiagen). DNA was stored in 1xTE (10mM Tris pH8.0 and 0.1mM EDTA).

### PCR reactions to assess the RMCE- integration of the TRA-ORF and TRB-ORF into component B or D.

Primers used to assess integration of the TRA-ORF, annealed to the TRA-C segment (forward primer 1.F.7) and the sv40pA terminator (Reverse primer 15.H.2) that is a preexisting part of the genomic receiving sites. Expected size 566bp Primers used to assess integration of the TRB-ORF, annealed to the TRB-C segment (forward primer 1.F.9) and the sv40pA terminator (Reverse primer 15.H.2) that is a pre-existing part of the genomic receiving sites. Expected size 610bp PCR products were run on a 1% Agarose gel in 1XTAE buffer, using the PowerPac Basic (Bio-Rad), stained with 10,000 dilution of sybersafe and analyzed with Fusion SL (Vilber Lourmat).

**Table 4: PCR reagents for assess integration of ORF encoding TCR-alpha and TCR-beta**

| **Reaction Component (TCR-alpha)** | **Volume per reaction** |
|---|---|
| 5xPhusion buffer | 4 µl |
| DNTPs | 0,2 µl |
| Phusion DNA polymerase | 0,15 µl |
| 1.F.7: TRAC-GT-F1 | 0,5 µl |
| 15.H.2: sv40pA-GT-R1 | 0,5 ul |
| H20 | up to 20 µl |
| DNA (100ng) | 1 ul (100 ng/µl) |

| **Reaction Component (TCR Beta)** | **Volume per reaction** |
|---|---|
| 5xPhusion buffer | 4 µl |
| DNTPs | 0,2 µl |
| Phusion DNA polymerase | 0,15 µl |
| 1.F.9: TRBC2-GT-F1 | 0,5 µl |
| 15.H.2: sv40pA-GT-R1 | 0,5 µl |
| H20 | up to 20 µl |
| DNA (100 ng) | 1 ul (100 ng/µl) |

**Table 5: PCR cycle conditions**

| **Step** | **Temperature** | **Time** |
|---|---|---|
| Initial Denaturation | 98°C | 30 sec |
| 30 cycles | 98°C | 10 sec |
| | 60°C | 10 sec |
| | 72°C | 15 sec |
| Final extension | 72°C | 10 min |

### ddPCR reactions to assess the copy number of TRA-ORF and TRB-ORF in the genome after DNA delivery.

DNA of selected ACL-851 monoclones was analysed by using specific primers and probed targeting the TCR_ORF C segment of interest
Primers and probe used to assess TCR-alpha ORF copy number, annealed to the TRAC segment (forward primer 1.F.7, Reverse primer 1.F.8 and probe 1.G.1)
Primers and probe used to assess TCR-beta ORF copy number, annealed to the TRBC segment (forward primer 1.F.9, Reverse primer 1.F.10 and probe 1.G.2)

In all cases, a reference gene (TRAC) was simultaneously screened to chromosome determine copy numbers, using primers 10.A.9 and 10.A.10 together with the fluorescent probe 10.B.6 conjugated with HEX. Integration copy number considered that HEK293 cells are triploid for reference gene (TRAC).

Prior to Droplet Digital PCR, DNA was digested with Mfel (NEB) to separate tandem integrations. The reaction setup and cycling conditions were followed according to the protocol for ddPCR^{™} Supermix for Probes (No dUTP) (Bio-Rad), using the QX200^{™} Droplet Reader and Droplet Generator and the C1000 Touch^{™} deep-well Thermal cycler (Bio-Rad). Data was acquired using the QuantaSoft^{™} Software, using Ch1 to detect FAM and Ch2 for HEX.

**Table 6: ddPCR conditions**

| **Step** | **Temperature** | **Time** |
|---|---|---|
| Initial Denaturation | 95°C | 10 min |
| 40 cycles | 98°C | 30 sec |
| | 60°C | 60 sec |
| Final extension | 72°C | 10 min |
| (Option) Cooling | 8°C | ∞ |

**Table 7: ddPCR Primers and probes**

| **ID** | **Name** | **Sequence** |
|---|---|---|
| 1.F.7 | TRAC-GT-F1 | ATGTGCAAACGCCTTCAAC |
| 1.F.8 | TRAC-GT-R1 | TTCGGAACCCAATCACTGAC |
| 1.G.1 | TRAC-probe-FAM | TTTCTCGACCAGCTTGACATCACAGG |
| 1.F.9 | TRBC2-GT-F1 | GCTGTCAAGTCCAGTTCTACG |
| 1.F.10 | TRBC2-GT-R1 | CTTGCTGGTAAGACTCGGAG |
| 1.G.2 | TRBC2-probe-FAM | CAAACCCGTCACCCAGATCGTCA |
| 10.A.9 | TRAC-TCRA-ex1-F1 | CTGATCCTCTTGTCCCACAGATA |
| 10.A.10 | TRAC-TCRA-ex1-F1 | GACTTGTCACTGGATTTAGAGTCTCT |
| 10.B.6 | TRAC-probe(HEX) | ATCCAGAACCCTGACCCTGCCG |

**Table 8: ACL cell lines**

| **ID** | **Components** | **Sequence** |
|---|---|---|
| ACL-128 | | APC, HLA-ABC null |
| ACL-209 | | APC, expressing HLA-A*02:01 |
| ACL-488 | B, D | eTPC, |
| | | B encodes BFP, |
| | | D encodes RFP |
| ACL-851 | B', D' | eTPC-t, |
| | | B' encodes JG9-TC-beta, |
| | | D' encodes JG9-TCR-alpha |
| ACL-963 | | APC, expressing HLA-A*24:02 |
| ACL-987 | B', D | eTPC-x, |
| | | B' encodes JG9-TCR-beta, |
| | | D' encodes GFP |
| ACL-988 | B', D' | eTPC-t (pool), |
| | | B' encodes wtJG9-TCR-beta, |
| | | D' encodes 1 of 64x variants of JG9-TCR-alpha |
| ACL-1063 | B, D, F | eTPC with two-component responder element, |
| | | B and D encode selection markers |
| ACL-1277 | B', D', F | eTPC-t with two-component responder element, |
| | | B' encodes TCR-alpha |
| | | D' encodes TCR-beta |

### Sequencing of TCR alpha and beta chains from single T-cells

Individual FACS-sorted eTPC-t-cells were subjected to a two-step amplification process that entails a V-region specific primer collection for each TCR-alpha and TCR-beta, followed by paired nested PCR reactions that create TCR-alpha and TCR-beta amplicons for sequence analysis. This procedure is described previously (Han et. al. Nat Biotechnol. 2014 32(7): 684-692). The following materials were used in the described procedures:

### Table 9: Single cell RT-PCR and nested PCR reagents

**Table 9: Single cell RT-PCR and nested PCR reagents**

| **Product** | **Supplier** | **Supplier Number** |
|---|---|---|
| 2x Reaction Mix | Thermo Scientific | 12574035 |
| 5X Phusion HF Buffer | Thermo Fisher Scientific | F-549S |
| dNTPs | Thermo Fisher Scientific | 10297018 |
| Nuclease free water | Qiagen | 129114 |
| Phusion Hot Start II DNA Polymerase | Thermo Fisher Scientific | F-549S |
| SuperScript^{®} III One- Step RT-PCR System with Platinum@ Taq High Fidelity DNA Polymerase | Thermo Scientific | 12574035 |

### Demonstration of functional component F

eTPC-t and APC cells were routinely cultured in RPMI+10% heat-inactivated Fetal Calf Serum (complete media) between 0.2 × 10^6 - 1.5 × 10^6 cells/ml, at 37°C, 90% relative humidity and 5% CO2. Peptides NLVPMVATV and VYALPLKML were synthetized by Genescript, and received lyophilized. Peptide primary stocks were suspended in 10% DMSO and sorted at -80'C. Working stocks were prepared at the time of administration, at 50 µM in complete media (50x concentrated). The following APCs presenting HLA-A*02:01 (ACL-209) or HLA-A*24:02 (ACL-963) or HLA-null (ACL-128) were used. The eTPC-t cell line (ACL-1277, Component A) was engineered with two unique genomic receiver sites (Components B, D), engineered to be HLA Null, utilizing native CD3 expression, and harbouring a two-component, synthetic response element (Component F). In addition, ACL-1277 had Components B, D converted to B' / D' with the integration of TCR alpha/beta ORF encoding a TCRsp specific for pHLA: HLA-A*02:01-NLVPMVATV (See Example 8).

### Antigen pulsing procedure

Actively growing cultures of APC cells (0.5-1.0 × 10^6 cells/ml) were suspended, sample taken and counted to determine cell concentration. Subsequently, 1 million cells were harvested, washed once with Dulbecco's phosphate buffered saline (DPBS, Gibco) followed by suspension in complete media with 1 µM of peptide or no peptide at a cell concentration between 1 to 2 × 10^6 cells/ml. Cells were incubated for 2 h in standard culturing conditions, in a 24-well culture plate. After 2 h the cells were harvested, pelleted by centrifugation (400 rcf, 3 min), followed by 3 × 10 ml washes with DPBS. Cells were subsequently suspended at 0.2 × 10^6 cells/ml in complete media.

### eTPC-t harvesting

Actively growing cultures of eTPC-t cells (0.5-1.0 × 10^6 cells/ml) were suspended, sample taken and counted to determine cell concentration. Cells were harvested, washed once with PBS and then suspended at a concentration of 0.6 × 10^6 cells/ml in complete media.

### Contacting eTPC-t and APC in an eTPC:A system

To each well of a 96-well round-bottom plate, 50 µl of complete media, 50 µl of APC , followed by 50 µl of eTPC-t were added. This equated to approximately 10,000 APC and 30,000 eTPC-t for a ratio of 1:3, at a total cell concentration of approximately 0.27 × 10^6 cells/ml. The cell mixture was then incubated for approximately 24 hours at standard culturing conditions.

### Staining and analysis

After 24 hours incubation, the cells were harvested, and transplanted into 0.75 ml V-bottom Micronic tubes, washed once with 500 µl DPBS and subsequently stained with Dead Cell Marker (DCM-APC-H7) as follows; to each well 25 µl of staining solution was added, cells suspended by mixing and then incubated for 15-20 min. The staining solution comprised of 0.5 µl DCM-APC-H7 per 100 µl staining solution. After incubation, cells were washed twice with 500 µl DPBS+2%FCS (Wash Buffer). Cells were then stained for surface markers unique to the eTPC-t; to each well 30 µl of staining solution was added, cells suspended by mixing and then incubated for 30-45 min. The staining solution comprised of 2.5 µl anti-myc-AF647 per 100 µl staining solution (clone 9E10, Santa Cruz Biotech). After incubation, cells were washed twice with 500 µl Wash buffer, then suspended in 200 µl of Wash buffer and then analysed by FACS on a LSRFortessa (BD Biosciences).

### The use of eTPCs as standard

### Cell preparation

The eTPC cell lines with TCR, specific to the NLVP peptide from the CMV virus, were harvested and pooled into a 50ml tube. Cells were pelleted at 400g for 5min and washed once with PBS. The eTPCs were resuspended into 3 ml of PBS, and counted. Four million cells were distributed into each 15ml tubes for fixation.

### Fixation

The fixing solution was prepared freshly before use, by diluting the Cell fix (containing 10 % paraformaldehyde (PFA)) with phosphate buffered saline (PBS) to a final concentration of 2 % PFA. 1 ml of fixation solution was added to cell pellet and cells were mixed by pipetting several times. For the untreated control, 1 ml of PBS was added instead of fixation solution. The cells were incubated for 5min at room temperature, then washed by addition of 3ml of PBS and pelleted at 400g for 5min. The cells were then resuspended into 500ul of PBS then transferred into micronic tubes for staining.

### Staining

The cells were first stained by multimers for 10 min at room temperature, then anti-CD3 antibodies were added and the samples further incubated at 4C for additional 30min. the cells were acquired on LSRFortessa.

### Spike-in assay

The eTPC cell lines with TCR, specific to the NLVP peptide from the CMV virus, were harvested and pooled into a 50ml tube. The cells were washed once with PBS, and pelleted down at 400g for 5min. The cells were then resuspended into 5 ml of PBS, then sorted on flow cytometer influx, as single cells into a 5ml FACS tubes containing 300ul staining buffer.

Sorting was based solely on the scatter of the cells. Gating: SSC vs FSC -> gating of live cells from the scatter plot; live cells were plotted FSC-W vs FSC -> gating of single cells that were sorted. The cells were pelleted at 400g for 5min, counted, stained with Alexa 647-CD58 for 30min at 4C, washed, then used in spike-in assay.

Frozen PBMCs from healthy blood donor, previously isolated using Ficoll gradient separation on 50 ml tubes, and positive for the CMV peptide (NLVP), were thawed, counted then used for the spike in experiment.

For the spike in assay, 2×10⁶ PBMCs were mixed in micronic tubes with either no eTPCs or with 50 000 eTPCs. The cells were first stained by multimers for 10min at room temperature, then anti- CD3 and anti-CD8 antibodies were added and the samples incubated at 4°C for additional 30min. The cells were acquired on Fortessa.

### Examples

### Example 1: Deletion of an APX gene family by targeted mutagenesis

Herein describes how targeted mutagenesis of a family of antigen-presenting complex **(APX)** encoding genes was achieved to produce a trait of an engineered TCR-presenting cell **(eTPC).** The said trait is the lack of surface expression of at least one member of the APX family.

In this example, the targeted APX comprised the three members of the major HLA class I family, HLA-A, HLA-B and HLA-C in the HEK293 cell line. HEK293 cells were derived from human embryonic kidney cells that showed endogenous surface expression of HLA-ABC. Cytogenetic analysis demonstrated that the cell line has a near triploid karyotype, therefore the HEK293 cells encoded three alleles of each HLA-A, HLA-B and HLA-C gene.

Targeted mutagenesis of the HLA-A, HLA-B and HLA-C genes was performed using an engineered CRISPR/Cas9 system, in which, Cas9 nuclease activity was targeted to the HLA-A, HLA-B and HLA-C loci by synthetic guide RNAs (gRNAs). 4 to 5 unique gRNAs were designed to target conserved nucleotide sequences for each HLA gene locus and the targeted sites were biased towards the start of the gene coding sequence as this was more likely to generate a null allele. The gRNAs efficiency to induce a mutation at their targeted loci was determined and the most efficient gRNAs were selected to generate the HLA-A, HLA-B and HLA-C null (HLA-ABC^{null}) HEK293 cell line.

Plasmid that encoded the optimal gRNAs targeting the HLA-A, HLA-B and HLA-C loci, together with a plasmid that encoded Cas9-P2A-GFP were transfected into HEK293 cells as described in the methods. Cells positive for Cas9-P2A-GFP plasmid uptake were FAC sorted based on GFP fluorescence, 2 days after transfection **(****figure 25a****).** The GFP sorted cells were further expanded for more than 5 days to allow sufficient time for gene editing events to occur, and in the case of a detrimental mutation, to lose of expression of the residual endogenous HLAI protein. After this growth period, the cells were stained with a pan-HLA-ABC antibody, resulting in the identification of cells with reduced expressed HLA-ABC on their surface **(****figure 25b****).** The absence of pan-HLA-ABC antibody staining implied that each HLA-A, HLA-B and HLA-C allele was mutated. Individual HLA-ABC negative cells were sorted and expanded to represent a collection of monoclones.

**HLA-ABC^{null}** monoclones were confirmed by lack of surface expression of HLA-ABC. It was demonstrated that a subset of monoclones lacked surface expression of HLA-ABC, of which three example monoclones, ACL-414, ACL-415 and ACL-416 are depicted in **figure 26****.** Further genetic characterization of the monoclones that lacked HLAI surface expression was performed by determining that the cell lines possessed an underlying genetic mutation in all alleles of the HLA-A, HLA-B and HLA-C genes **(****figure 27****).** Genetic characterization was performed by PCR with primers that spanned the gRNA genomic target sites, for detection of amplicon size changes and/or were used as a template for sequencing. **Figure 27** shows a selection of HLA-ABC^{null} monoclones that contained genetic deletion in the alleles of HLA-A, HLA-B and HLA-C genes detected by a shorter PCR amplicon compared to the amplicon size of the founding cell line (e.g. ACL-414).

In conclusion, the genetically modified HEK293 cell lines, including, ACL-414, ACL-415 and ACL-416, were demonstrated to lack surface expression of the HLA-ABC and therefore possessed a trait of an engineered TCR-presenting cell **(eTPC).**

### Example 2: Generation of an HLA-ABC^{null} containing Component B

Herein describes how **Component B** was stably integrated into the **HLA-ABC^{null}** monoclone line ACL-414 to produce the second trait of an **eTPC.** The said second trait contained at least one genomic receiver site for integration of at least one ORF, wherein the genomic receiver site was a synthetic construct designed for recombinase mediated cassette exchange (RMCE).

In this example, the genomic integration site, component B, comprised of selected genetic elements. Two unique heterospecific recombinase sites, FRT and F3, which flanked a ORF that encoded the selection marker, blue fluorescent protein (BFP). Encoded 5' of the FRT site, was an EF1a promoter and 3' of the F3 site was a SV40 polyadenylation signal terminator. The benefit of positioning the non-coding cis-regulatory elements on the outside of the heterospecific recombinase sites was so they are not required in the matched genetic donor vector, component C. Therefore, after cellular delivery of the genetic donor vector, no transient expression of the encoded ORF would be observed. This makde the selection of successful RMCE more reliable as the cellular expression of the ORF from the genetic donor vector would mostly likely occur only after correct integration into component B as it now contained the appropriate cis-regulator elements (see example 6).

To promote the stable genomic integration of component B into the genomic safe harbour locus, AAVS1, a plasmid was constructed, wherein, the DNA elements of component B were flanked with AAVS1 left and right homology arms. Each arm comprised of >500 bp of sequence homologous to the AAVS1 genomic locus.

Stable integration of component B was achieved through the process of homology directed recombination (HDR) at the genomic safe harbour locus, AAVS1. The ACL-414 cell line was transfected with plasmid that encoded the optimal gRNAs targeting the AAVS1 locus, plasmid that encoded Cas9-P2A-GFP and the plasmid that encoded component B genetic elements flanked by AAVS1 left and right homology arms. Cells positive for Cas9-P2A-GFP plasmid uptake were FAC sorted based on GFP fluorescence, 2 days after transfection **(****figure 28a****).** The GFP sorted cells were further expanded for greater than 7 days allowing sufficient time for HDR to occur and to lose transient expression of the selection marker, BFP. After this growth period, the cells were analysed on a FACS machine and individual BFP positive cells were sorted and expanded to represent a collection of monoclones **(****figure 28c****).**

Individual monoclone lines were selected on the basis of their maintained BFP expression and for a single integration of component B into the desired AAVS1 genomic location. Cell lines ACL-469 and ACL-470 representated monoclones with maintained BFP expression **(****figure 29a** **and b).** Genetic characterization was performed on DNA extracted from monoclones ACL-469 and ACL-470 and demonstrated that their genomes integrated component B, and that component B has been integrated into the AAVS1 site **(****figure 30****).** Confirmation of genomic integration was determined by the detection of a PCR amplicon of the expected size that utilized primers specific for the Component B **(****figure 30a****).** Confirmation that component B integrated into the AAVS1 site was determined by the detection of a PCR amplicon of the expected size that utilized primers designed against the AAVS1 genomic sequence distal to the region encoded by the homologous arms and a primer that is unique to the SV40 pA terminator encoded by component B **(****Figure 30b****).** The copy-number of component B was determined by digital drop PCR, in which the number of component B and reference gene DNA molecules were measured and the ratio calculated **(table 1).** Monoclones ACL-469 and ACL-470 contained a ratio of 1 component B molecule to 3 reference gene molecules. When factored in that the founding HEK293 cell line has a near triploid karyotype, this demonstrated a single integration of component B in ACL-469 and ACL-470 cell lines.

In conclusion, the genetically modified ACL-469 and ACL-470 cell lines, were HLA-ABC^{null} and contained a single copy of a synthetic genomic receiver site designed for RMCE and therefore demonstrated the creation of cell lines comprising of two traits that can be further modified into an **eTPC.**

### Example 3: Generation of an HLA-ABC^{null} cell containing Component B and Component D

Herein describes how **Component B** and **Component D** were stably integrated into the HLA-ABC^{null} monoclone line ACL-414 to produce the second trait of an **eTPC.** The said second trait, contains two genomic receiver site for integration of at least one ORF, wherein the genomic receiver site was a synthetic construct designed for recombinase mediated cassette exchange (RMCE).

This example uses the same methods and components as described in example 2 but with the addition of a second genomic receiver site, Component D. Component D genetic elements comprised of two unique heterospecific recombinase sites, F14 and F15, which were different to component B. These sites flanked the ORF that encoded the selection marker, the red fluorescent protein (RFP). Encoded 5' of the F14 site was an EF1a promoter and 3' of the F15 site was a SV40 polyadenylation signal terminator. As in example 2, component D genetic elements were flanked with AAVS1 left and right homology arms, each comprised of >500 bp of sequence homologous to the AAVS1 genomic locus.

Component B and component D were integrated into the AAVS1 as described in example 2 but with the addition of the plasmid that encoded component D elements, to the transfection mix. Cells positive for Cas9-P2A-GFP plasmid uptake were FAC sorted based on GFP fluorescence, 2 days after transfection **(****figure 28b****).** The GFP sorted cells were further expanded for greater than 7 days, after which, the cells were analysed on a FACS machine and individual BFP and RFP positive cells were sorted and expanded to represents a collection of monoclones **(****figure 28d****).**

Individual monoclone lines were selected on the basis of their maintained BFP and RFP expression and for a single integration of component B and a single integration of component D into different AAVS1 alleles. Cell line ACL-472 was a representative monoclone with maintained BFP and RFP expression **(****figure 29c****).** As described in example 2, genetic characterization was performed on DNA extracted from monoclone ACL-472 and demonstrated that their genomes integrated component B and component D, and that both components integrated into the AAVS1 site **(****figure 30****).** The copy-number of both component B and D was determined by digital drop PCR, in which the number of component B, D and reference gene DNA molecules was measured and the ratio calculated. The monoclone ACL-472 contained a ratio of 2 component B andD molecules to 3 reference gene molecules **(Table 2).** When factored in that the founding HEK293 cell line has a near triploid karyotype, this demonstrated a single integration of component B and a single integration of component D into the ACL-472 cell line.

In conclusion, the genetically modified ACL-472 cell line, was HLA-ABC^{null} and contained a single copies of the synthetic genomic receiver site component B and component D, designed for RMCE and therefore demonstrated the creation of a cell line comprising of multiple traits that can be further modified into an **eTPC.**

### Example 4: Integration of CD3 into an aAPX- cell that lacks TCR and CD3

Herein is presented an exemplar of the workflows of Figures 32 and Figure 34, wherein first a Target Base Cell is identified as a suitable TCRsp Competent Base Cell, followed by engineering of the TCRsp Competent Base Cell by integrating into the genome genetic material encoding expression of CD3 as a single ORF encoding all four proteins of CD3, being CD3 gamma, delta, epsilon and zeta. The four encoded proteins are linked via a self-cleaving viral P2A peptide, such that during translation of the transcript the action of the each P2A peptide cause separation and release of the 4 individual proteins. Once translation and separation occurs the CD3 proteins are subsequently free to associate and assemble into the CD3 complex. However, assembly and export to the surface of the cell only occurs in the presence of a translated pair of complementary TCR chains.

FAC analysis was conducted using a BD LSRFortessa instrument, with a high throughput sampler. FAC sorting was conducted using either a BD Influx cell sorter or a BD FACSJazz sorter. Transfection of ACL-5 and derivative cells was conducted using chemical transfection via the PEI method, with an NP ratio of 6, final DNA concentration of 200 ng/µl per PEI reaction, and 3.33 µg of total DNA per 1 million cells. Cells were stained using standard procedures known to those skilled in the art. Fluorescently labelled antibodies with affinity towards CD3 and TCR-alpha-beta were purchased from BD, with anti-CD3 conjugated to either FITC or PE-CF5486, and anti-TCR-alpha-beta conjugated to BV786 or APC. The aAPX:aAM soluble reagent was purchased from Immudex, specifically the dextramer HLA-A*0201, loaded with NLVPMVATV as cargo, conjugated to PE fluorophore, designated A2-NLV-PE. Plasmids used were obtained using standard methods known to those skilled in the art.

### Identification and Selection of a TCRsp Competent Base Cell

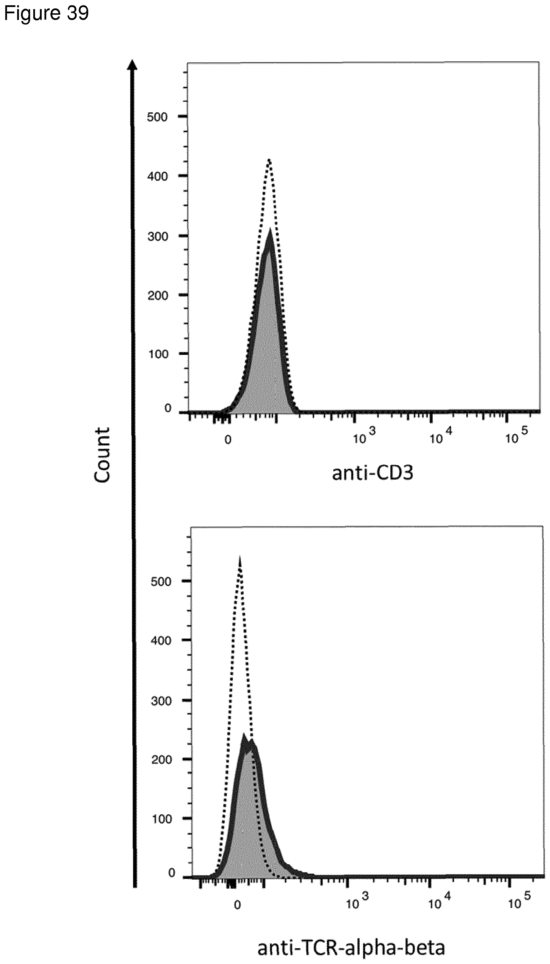

A HEK293-derived cells, ACL-5, was selected as a Target Base Cell (Figure 32, step a) and stained with anti-TCR-alpha-beta and anti-CD3, followed by FACS analysis to identify the lack of surface expression of TCRsp and CD3 (Figure 32, step b). As a comparison control cells staining were also analysed. No surface expression was detected (Figure 32, step c) as evident in Figure 39. In addition, the lack of CD3 expression also indicated that the Target Base Cell was also lacking in TCR-gamma-delta expression by virtue that CD3 cannot be expressed on the surface in the absence of expression of a complementary pair of TCR chains.

The Target Base Cells were subsequently chemically transfected with three transient expression plasmids, the first encoded all four CD3 proteins as a single ORF linked by P2A viral peptides (V3.C.6). The second and third plasmids encoded a complementary pair of TCR chains (pTCR), wherein each plasmid encoded transient expression of either a TCRalpha chain or a TCRbeta chain (Figure 32, step d). Two different TCRalpha-beta pairs were transfected, one pair encoded a TCRsp specific for HLA-A*02:01 loaded with NLVPMVATV peptide (V3.C.3, V3.C.5, A2-NLV^{pos}), and the other pair encoded a TCRsp that was not specific (V3.C.2, V3.C.4, A2-NLV^{neg}).

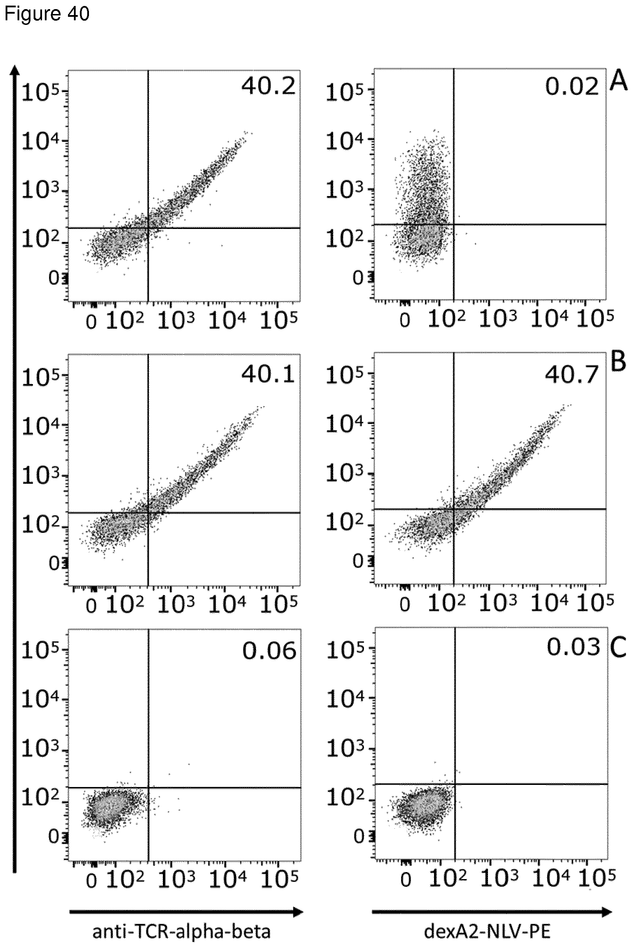

After 2 days outgrowth the transfected cells were stained with anti-TCR-alpha-beta and anti-CD3 (Figure 32, step e), in addition, a soluble aAPX:aM (dexA2-NLV-PE) was also used to stain the cells, which was the cognate antigen of A2-NLV^{pos} TCRab pair. The stained cells were subsequently analysed by FACS and shown to be competent for surface expression of CD3 and TCRsp (Figure 40). In addition, the cells transfected with the A2-NLV^{pos} exhibited binding to dexA2-NLV-PE, whereas cells transfected with A2-NLV^{neg} exhibited no binding to dexA2-NLV-PE (Figure 40). This indicated that the cells were competent at expressing TCRsp (Figure 32, step f) and subsequently the Target Base Cell was select as a suitable TCRsp Competent Base Cell.

The TCRsp Competent Base Cell was then subject to targeted mutation of the HLA class I family of aAPX, to render the cell lacking expression of the HLA class I family of aAPX. The mutated cell was obtained using the procedure in exemplar of Example 1. In addition, the TCRsp Competent Base Cell also naturally lacked expression of the HLA class II family of aAPX, thus, the TCRsp Competent Base Cell obtained lacked expression of two families of aAPX.

### Identification, Engineering and Selection of a Confirmed Base Cell

The TCRsp Competent Base Cell (lacking expression of two aAPX families) was chemically transfected with two transient expression plasmids, the first encoding CD3 as previous described (V3.C.6) and either TCRalpha (V3.C.2, V3.C.3) or TCRbeta (V3.C.4, V3.C.5), which was a modified version of Figure 34, step h. After 2 days outgrowth the transfected cells were stained with anti-TCR-alpha-beta and anti-CD3 (Figure 34, step i) and were subsequently analysed by FACS (Figure 41) and identified to lack surface expression of TCRsp and CD3 (Figure 34, step i) confirming that neither CD3 nor TCR alpha or TCR beta was endogenously expressed.

The CD3 was integrated in to the TCRsp Competent Base Cell (Figure 34, step k). In order to achieve integration the cell was transiently transfected three plasmids, the first encoding expression of the first integration factor, Cas9-2A-GFP (V1.A.8) and the second encoding the expression of the second integration factor, a guideRNA targeted to the HLA-A loci (V2.A.2). The third plasmid was for site directed homologous recombination, targeted to the HLA-A loci and encoded expression of CD3 proteins via the human EF1a-Long promoter and SV40pA terminator, as a single ORF linked by P2A viral peptides (V4.L7).

At 2 days post-transfection, cells were harvested and sorted based on GFP expression to enrich for cells expressing Cas9 of which a proportion had the integrated the CD3 encoding sequence at the HLA-A loci (Figure 42).

The sorted cells were out-grown for 3 days, whereupon cells were co-transfected with three plasmids, the first encoded expression of GFP and was used as a transfection control. The second and third plasmids, each encoded either a TCR-alpha (V3.C.3) chain or a TCR-beta chain (V3.C.5) of a pTCR (A2-NLV^{pos}) (Figure 34, step I). The transfected cells were outgrown 2 days and were subsequently stained with anti- anti-CD3 (Figure 34, step m). Cells were then selected for surface expression of CD3 (Figure 34, step n), wherein selection was conducted via FAC sorting as a polyclonal population (Figure 43).

The sorted cells were out grown for 11 days and then subsequently subjected to another round of transfection (Figure 34, step I), staining (Figure 34, step m) and selection (Figure 34, step n) as described previously (Figure 44). The sorted cells were out-grown and subsequently transfected and stained a third time. Stained cells were then selected for TCRsp expression and 96 single cells were selected by single cell sorting for monocloning (Figure 45). The remaining stained cells were selected accordingly, polyclonal sorted and cryopreserved at -80'C.

The single-cell clones were outgrown until sufficient numbers were available for cryopreservation, genomic and phenotypic analysis via PCR, ddPCR and FACS analysis. Of the 96 single cell sorts, 20 survived for analysis, and 8 of 20 clones were positive for correct genomic integration and were also positive by digital drop analysis. The 8 positive monoclones were transfected with two plasmids, that encoded either a TCR-alpha (V3.C.2) chain or a TCR-beta chain (V3.C.4) of a pTCR (A2-NLV^{neg}) and a further third plasmid that encoded GFP expression as a transfection control (V1.A.4). The cells were then stained with anti-CD3 and anti-TCR-alpha-beta and analysed for surface expression of TCRsp and CD3 (Figure 34, step n). Two of the 8 lines were positive for surface expression, with one line showing high percentage of expression (Figure 46) which was subsequently selected as the Confirmed Base Cell.

### Example 5: Demonstration of eTPC-t generation in one step

The present example describes the generation of eTPC-t in a standardised manner, wherein the parental eTPC contains distinct synthetic genomic receiver sites **Components B and D.** The genetic donor vectors **Components C' and E'** comprised a single chain of a TCR pair (JG9-TCR) known to engage with the antigenic peptide NLVPMVATV (NLVP) derived from Human Cytomegalovirus polypeptide 65 (HCMV pp65) when presented in HLA-A2 alleles. Components C' and E' are designed for RMCE, and derived from parental **Components C and E.**

This example uses a parental eTPC cell line ACL-488, which is TCR null, HLA null, CD4 null and CD8 null, and further containing **Component B** and **D. Component B** comprises two unique heterospecific recombinase sites, FRT and F3 that flank a Kozak sequence and ORF encoding the selection marker, blue fluorescent protein (BFP). Encoded 5' of the FRT site, is an EF1a promoter and 3' of the F3 site is a SV40 polyadenylation signal terminator. **Component D** comprises two unique heterospecific recombinase sites, F14 and F15, which were different to **Component B.** These sites flank a Kozak sequence and ORF that encodes the selection marker, the red fluorescent protein, (RFP). Encoded 5' of the F14 site is an EF1a promoter, and 3' of the F15 site is a SV40 polyadenylation signal terminator.

This example uses genetic donor vectors, **Component C' and Component E',** each comprising of two heterospecific recombinase sites, FRT/F3 **(C')** and F14/F15 **(E'),** thus being matched to **Component B** and **D,** respectively. **Component C'** further comprises, between the FRT/F3 sites, of a Kozak sequence, start codon and TCR ORF encoding JG9-TCR-beta chain. **Component E'** further comprises, between the F14/F15 sites, of a Kozak sequence, start codon and TCR ORF encoding JG9-TCR-alpha chain.

An eTPC-t was created through RMCE by electroporation ACL-488 (eTPC). Four to ten days after electroporation, individual cells displaying diminished fluorescent protein signal, BFP and RFP, encoded by **Components D** and **B** selection markers, were sorted by FACS. Individual monoclones were out grown and then phenotypically assessed. The resulting monoclone, ACL-851, was BFP and RFP negative (figure 48 a and b). ACL-851 also showed TCR and CD3 surface expression while the parental cell line did not (figure 48 c and e). Furthermore, the introduced JG9-TCR showed specific staining with the HLA-A*02:01- NLVP tetramer, indicating that it is a functional TCRsp on the surface of the eTPC-t (figure 48 d to f). ACL-851 was confirmed by PCR to contain the TCRsp encoded by **Component B'** and **Component D'** integrated into the genome (figure 48 g and h).

In summary, an eTPC was converted to an eTPC-t, by use of an RMCE based integration method to integrate TCR ORF delivered in **Component C'** and **E',** such that **Components B** and **D** were converted into **Component B'** and **D',** and where by this eTPC-t expressed a functional TCRsp on the surface of the cell. Furthermore, this example demonstrates operation of a simple eTPC:A system, where a binary composition of an eTPC-t and analyte antigen were combined and the eTPC-t selected based on a complex formation between the soluble analyte antigen (HLA multimer: HLA-A*02:01-NLVPMVATV).

### Example 6: Demonstration of conversion of eTPC-t to an eTPC-x

The present example describes conversion of an eTPC-t to an eTPC-x, wherein the eTPC-x has component B' encoding a TCR chain ORF and Component D is available for integration of complementary TCR chain ORF. Conversion of Component D' of the eTPC-t to Component D of the eTPC-x is achieved by use of a genetic donor vector (Component Z) matched to Component D'.

In this example, the parental eTPC-t cell line ACL-851 generated in example 5 was used. Component Z is a plasmid vector comprised of two heterospecific recombinase sites, F14/F15 matched to Component D', a Kozak sequence, start codon and an ORF encoding a green fluorescent protein (GFP) as a selection marker of integration. The eTPC-t was combined with **Component Z** and a vector encoding RMCE recombinase enzyme by electroporation, whereupon the cells were subsequently selected for loss of CD3 presentation and gain of the GFP selection marker of integration. The monolcone ACL-987 was phenotypically characterised by FACS, and it was observed that the ACL-987 has gained GFP and lost CD3 and TCRab (Figure 49b, d), indicating successful exchange of JG9-TCR-alpha with the GFP ORF and conversion of Component D' to Component D, and thus generation of an eTPC-x. In comparison the parental eTPC-t, ACL-851, is lacking GFP expression and has CD3 and TCRab surface expression (Figure 49 a to c).

In summary, this example demonstrates conversion of an eTPC-t to an eTPC-x, with removal of the JG9-TCR-alpha TCR ORF at Component D' in exchange for the GFP selection marker of integration thereby creating Component D, for further integration coupling events of alternative complementary TCR chain ORF. This conversion was conducted using the RMCE method for genomic integration.

### Example 7: Demonstration of shotgun integration into eTPC-x to create pool of eTPC-t

The present example describes how a pool of vectors encoding 64 single JG9-TCR-alpha variants (as **Component E')** were integrated as a single step into a parental eTPC-x cell line (described in example 6) to create a pooled eTPC-t library wherein each individual cell integrated a single random TCR ORF encoding alpha chain from the original pool of vectors, such that each eTPC-t expresses a single random pair of TCR ORF as TCRsp. Combined together the individual eTPC-t comprises a pooled a library of eTPC-t wherein the pool of cells potentially represents all possible combinations of the 64 TCR-alpha paired with constant original TCR beta chain (see figure 12 schematic). Such a method is now referred to as 'shotgun' integration. The 64 JG9-TCR-alpha variants have been created by modifying the CDR3 sequence which falls at the junction of the V and J fragments.

In this example, the parental eTPC-x cell line ACL-987 (see example 6), with non-surface expressed JG9-TCR-beta **(Component B')** and CD3 complex was used. **Component D** encodes GFP, a selection marker, as described in example 6. In this example, the 64 JG9-TCR-alpha variant fragments were cloned into the Component E donor vector, creating **Component E',** flanked by F14/ F15 sites, matching to Component D. The 64 vectors were subsequently combined into a single pool of vectors.

An eTPC-t pool was created via RMCE based genomic integration, wherein the eTPC-x (ACL-987) and 64 **Components E'** and RMCE recombinase vector were combined by electroporation. Polyclones were selected on the basis of the GFP expression. The resulting polyclone, ACL-988, comprised of both GFP positive and GFP negative cell populations, unlike the parental line which comprised of only GFP positive cells (figure 50 a and b). However, only GFP negative population showed consistently strong CD3 expression, indicating successful conversion of **Component D** into **Component D'** and therefore the eTPC-x has been converted into a pool of eTPC-t (figure 50 c and d). Furthermore, ACL-988 GFP negative populations showed two distinct intensities when stained with the JG9-TCR specific multimer reagent (DEX HLA-A*02:01-NLVP), suggesting that this population comprises of cells that express TCR variants with varying binding efficiency.

In parallel, all 64 JG9-TCR-alpha variants were cloned into an expression construct that permitted transient transfection to a parental eTPC-x (ACL-987) and relative staining units (RSU) against the HLA-A*02:01-NLVP multimer reagent to a reference for each TCR pair presented in the above-described pooled eTPC-t expressing variant JG9-TCR were determined. RSU were calculated as the ratio of the mean fluorescence intensity (MFI) of HLA multimer signal for the CD3 positive population over the CD3 negative population, and was indicative of the binding strength of each TCR chain pair variant. After the independent transfection of the parental ACL-987 line with each JG9-TCR-alpha variant, the cells were stained with antibodies against CD3 and with the HLA-multimer reagent and analysed by flow cytometry. Each point plotted in Figure 51e represent the observed RSU for each 64 variants.

Individual cells from the pool of ACL-988 were single cell sorted, from the HLA-multimer positive population and the HLA-multimer negative population. The Component D' encoding the variant JG9-TCR-alpha ORF for each single cell were amplified and sequenced and compared to the results of the transient expressions RSU units described above (Figure 50e). Indeed, individual ACL-988 cells that were HLA-multimer positive encoded JG9-TCR-alpha variants that predominantly showed high RSU results in the individually tested variants (Figure 51e, open circles). Moreover, individual ACL-988 cells that were pHLA-multimer negative encoded JG9-TCR-alpha variants that predominantly showed low RSU results (figure 51e open triangles).

In conclusion, this example demonstrates use of the multi-component system for conversion of an eTPC-x and a pooled library of vectors (component E') into a pooled library of eTPC-t containing multiple different TCRsp. This was achieved in a single step using shotgun integration. Moreover, this example demonstrated that the pool of eTPC-t were combined with an analyte antigen (as a soluble affinity reagent) into an eTPC:A system, wherein it was demonstrated that single cells of the pool could be selected on the basis of complex formation with the analyte antigen (HLA-multimer) and wherein the subsequent TCR chain ORF encoded in Component D' were extracted and DNA sequences obtained.

### Example 8: Functional demonstration of component F

Herein describes an eTPC cell line (ACL-1277, Component A) engineered with two unique genomic receiver sites (Components **B, D),** engineered to be HLA Null, utilizing native CD3 expression, and harbouring an engineered genomic two-component, synthetic response element (Component **F).**

The response elements comprised of a Driver-Activator component and an Amplifier-Report component, wherein both units utilized synthetic promoters. The Driver is a synthetic promoter that is responsive to the native TCR signalling pathways, encoding three sets of tandem transcription factor binding sites for NFAT-AP1-NFkB (3xNF-AP-NB). Upon transcriptional activation, the Driver induces expression of the Activator protein, a synthetic designed transcription factor derived by fusion of the Herpes VP16 activation domain, the GAL4 DNA binding domain and two nuclear localization signals at the N- and C-terminals (NV16G4N), to which the cognate DNA recognition sequence is present 6 times in tandem in the Amplifier promoter. Both the Driver and Amplifier promoters utilized the core promoter sequence (B recognition element (BRE), TATA Box, Initiator (INR) and transcriptional start site) from HCMV IE1 promoter, immediately 3' of the respective transcription factor binding sites. The Amplifier upon transcriptional activation drives expression of the reporter, red fluorescent protein (RFP).

In this example, the eTPC cell line was converted to an eTPC-t cell line as described previously in example 5, wherein the TCR chain ORF at Component B' and E' encode a TCR pair that is specific for HLA-peptide complex (HLA), HLA-A*02:01-NLVPMVATV.

The eTPC-t cell line was then challenged against APCs presenting HLA-A*02:01 (ACL-209) or HLA-A*24:02 (ACL-963) or was HLA-null (ACL-128). Wherein analyte APC were prepared by pulsing the APC with analyte antigen of either peptide NLVPMVATV or VYALPLKML or no peptide. Subsequently, an eTPC-t and analyte APC were compiled into an eTPC:A system consisting of 30,000 eTPC-t co-cultured with 10,000 APCs for 24h. After 24h the cells were harvested, washed, stained with markers specific for the eTPC-t and analyte APC in order to distinguish the populations, and analysed by flow cytometry using techniques known to person skilled in the art. Strong activation of the eTPC-t, Component F (RFP+ expression >80%) was only observed in eTPC-t challenged with analyte APC presenting the known cognate antigen pHLA complex, i.e. the APC with A*02:01-NLVPMATV (Figure 51a). In contrast only resting state RFP expression was observed in eTPC:A compilations comprised of non-specific analyte APC (Figure 51b, c, d) or control analyte APC lacking either HLA or peptide.

In conclusion, an eTPC cell line containing a functional component F was engineered, and subsequently used to create an eTPC-t. Upon interaction of the eTPC-t with analyte APC presenting its cognate target T-cell antigen, a response was measurable as an increase in RFP expression. Conversely, when contacted with analyte APC presenting a non-cognate T-cell antigen and HLA, or no HLA, no measurable increase in RFP expression above background was exhibited by the eTPC-t. Furthermore, this example demonstrates an eTPC:A system wherein analyte APC and analyte eTPC-t are compiled in discrete binary compositions and the eTPC-t response is used to identify both the analyte APC and eTPC-t wherein a co-operative complex between the TCRsp and analyte antigen occurs.

### Example 9: Use of eTPC-t as TCR-bearing flow cytometric standards

Herein describes the exemplification of an eTPC-t created and selected using the multi-component system and subsequent compiled eTPC:A system, for the use as an in vitro FACS based assay standard. The present example demonstrates chemical fixation and cryopreservation of a confirmed and selected eTPC-t (ACL-851, example 5) expressing a TCRsp specific for an antigen (APX:AM), comprised of an HLA-A*02:01 antigen presenting complex (APX) loaded with the antigenic molecule (AM), the peptide NLVPMVATV derived from HCMV-pp65. The TCRsp maintained pHLA-multimer (APX:AM) labelling when recalled from storage (Figure 52a). Moreover, these cells could be 'spiked in' to a human peripheral blood mononuclear cell (PBMC) preparation, and detected along with HCMV-specific primary T-cells within the same specimen by flow cytometry (Figure 52b). The eTPC-t were pre-stained with a CD58 antibody prior to being spiked into the PBMC mix and subsequent staining with tetramer reagent. The CD58 parameter was used to distinguish the eTPC-t from the primary T-cells specific for NLVPMVATV peptide presented in HLA-A*02:01. This demonstrates that eTPC-t expressing specific TCRsp chain pairs can be used as TCR-presenting control reagents in flow cytometric assays detecting antigen-specific T-cells.

### SEQUENCE LISTING

<110> Genovie AB
<120> An Engineered Multi-component System for Identification and Characterisation of T-cell receptors, T-cell antigens and their functional interaction
<130> ANO9
<160> 134
<170> BiSSAP 1.3
<210> 1 <223> HCMV Antigen
<210> 2 <223> Analyte Antigenic Peptide
<210> 3 <223> Analyte Antigenic Peptide
<210> 4 <223> pcDNA3.1_GFP vector V1.A.4
<210> 5 <223> pcDNA3.1_RFP vector V1.A.6
<210> 6 <223> SpCas9-2A-GFP vector V1.A.8
<210> 7 <223> pMA-SV40pA vector V1.C.2
<210> 8 <223> HLA-A 02:01 6xHis + Exon2/3-HA-L+R vector V1.C.6
<210> 9 <223> HLA-B 35:01 6xHis + Exon2/3-HA-L+R vector V1.C.9
<210> 10 <223> AAVS1-L_B07_6xH vector V1.F.10
<210> 11 <223> AAVS1-S_A24_6xH vector V1.F.8
<210> 12 <223> AAVS1-I_GFP_HCMVpp65 vector V1.G.10
<210> 13 <223> AAVS1-I_GFP_HCMVpp65 ANET vector V1.G.9
<210> 14 <223> AAVS1-I_GFP_HCMVpp65 AIN vector V1.H.1
<210> 15 <223> AAVS1_DRA_Flag-DRB1_6xHis vector V1.1.5
<210> 16 <223> AAVS1_DPA1_Flag-DPB1_6xHis vector V1.I.7
<210> 17 <223> HLA-A-sg-sp-opti1 vector V2.A.1
<210> 18 <223> HLA-B-sg-sp-3 vector V2.A.7
<210> 19 <223> HLA-C-sg-sp-4 vector V2.B.3
<210> 20 <223> HLA-A-ex2-3_sg-sp-opti_1 vector V2.I.10
<210> 21 <223> HLA-A-ex2-3_sg-sp-opti_2 vector V2.J.1
<210> 22 <223> AAVSI_sg-sp-opti_3 vector V2.J.6
<210> 23 <223> pMA-CS-JG9-TCRbeta vector V3.C.5
<210> 24 <223> AAVS_Efla-intron_F14_RFPnls_F15 vector V4.B.2
<210> 25 <223> AAVS_Efla-intron_FRT_BFPnls_F3 vector V4.B.3
<210> 26 <223> pMA_FRT_HLA-A*02:01-6xHis_F3 vector V4.D.2
<210> 27 <223> pMA-F14-GFP-F15 vector V4.H9
<210> 28 <223> CMVpro-Flp-sv40pA-V2 vector V4.I.8
<210> 29 <223> pMA-F14-TCR-JG9-alpha-F15 vector V7.A.3
<210> 30 <223> pMA-FRT-TCR-JG9-beta-F3 vector V7.A.4
<210> 31 <223> F14-TCRaF15 CDR3degen.64mix vector V8.F.8
<210> 32 <223> JG9-TRA CDR3 64 variants vectors backbone VP.7751.RC1-A1 to H8
<210> 33 <223> CDR3 sequence of a JG9-TRA 64 variant VP.7751.RC1_A1
<210> 34 <223> CDR3 sequence of a JG9-TRA 64 variant VP.7751.RC1_A2
<210> 35 <223> CDR3 sequence of a JG9-TRA 64 variant VP.7751.RC1_A3
<210> 36 <223> CDR3 sequence of a JG9-TRA 64 variant VP.7751.RC1_A4
<210> 37 <223> CDR3 sequence of a JG9-TRA 64 variant VP.7751.RC1_A5
<210> 38 <223> CDR3 sequence of a JG9-TRA 64 variant VP.7751.RC1_A6
<210> 39 <223> CDR3 sequence of a JG9-TRA 64 variant VP.7751.RC1_A7
<210> 40 <223> CDR3 sequence of a JG9-TRA 64 variant VP.7751.RC1_A8
<210> 41 <223> CDR3 sequence of a JG9-TRA 64 variant VP.7751.RC1_B1
<210> 42 <223> CDR3 sequence of a JG9-TRA 64 variant VP.7751.RC1_B2
<210> 43 <223> CDR3 sequence of a JG9-TRA 64 variant VP.7751.RC1_B3
<210> 44 <223> CDR3 sequence of a JG9-TRA 64 variant VP.7751.RC1_B4
<210> 45 <223> CDR3 sequence of a JG9-TRA 64 variant VP.7751.RC1_B5
<210> 46 <223> CDR3 sequence of a JG9-TRA 64 variant VP.7751.RC1_B6
<210> 47 <223> CDR3 sequence of a JG9-TRA 64 variant VP.7751.RC1_B7
<210> 48 <223> CDR3 sequence of a JG9-TRA 64 variant VP.7751.RC1_B8
<210> 49 <223> CDR3 sequence of a JG9-TRA 64 variant VP.7751.RC1_C1
<210> 50 <223> CDR3 sequence of a JG9-TRA 64 variant VP.7751.RC1_C2
<210> 51 <223> CDR3 sequence of a JG9-TRA 64 variant VP.7751.RC1_C3
<210> 52 <223> CDR3 sequence of a JG9-TRA 64 variant VP.7751.RC1_C4
<210> 53 <223> CDR3 sequence of a JG9-TRA 64 variant VP.7751.RC1_C5
<210> 54 <223> CDR3 sequence of a JG9-TRA 64 variant VP.7751.RC1_C6
<210> 55 <223> CDR3 sequence of a JG9-TRA 64 variant VP.7751.RC1_C7
<210> 56 <223> CDR3 sequence of a JG9-TRA 64 variant VP.7751.RC1_D1
<210> 57 <223> CDR3 sequence of a JG9-TRA 64 variant VP.7751.RC1_D2
<210> 58 <223> CDR3 sequence of a JG9-TRA 64 variant VP.7751.RC1_D3
<210> 59 <223> CDR3 sequence of a JG9-TRA 64 variant VP.7751.RC1_D4
<210> 60 <223> CDR3 sequence of a JG9-TRA 64 variant VP.7751.RC1_D5
<210> 61 <223> CDR3 sequence of a JG9-TRA 64 variant VP.7751.RC1_D6
<210> 62 <223> CDR3 sequence of a JG9-TRA 64 variant VP.7751.RC1_D7
<210> 63 <223> CDR3 sequence of a JG9-TRA 64 variant VP.7751.RC1_D8
<210> 64 <223> CDR3 sequence of a JG9-TRA 64 variant VP.7751.RC1_E1
<210> 65 <223> CDR3 sequence of a JG9-TRA 64 variant VP.7751.RC1_E2
<210> 66 <223> CDR3 sequence of a JG9-TRA 64 variant VP.7751.RC1_E3
<210> 67 <223> CDR3 sequence of a JG9-TRA 64 variant VP.7751.RC1_E4
<210> 68 <223> CDR3 sequence of a JG9-TRA 64 variant VP.7751.RC1_E5
<210> 69 <223> CDR3 sequence of a JG9-TRA 64 variant VP.7751.RC1_E6
<210> 70 <223> CDR3 sequence of a JG9-TRA 64 variant VP.7751.RC1_E7
<210> 71 <223> CDR3 sequence of a JG9-TRA 64 variant VP.7751.RC1_E8
<210> 72 <223> CDR3 sequence of a JG9-TRA 64 variant VP.7751.RC1_F1
<210> 73 <223> CDR3 sequence of a JG9-TRA 64 variant VP.7751.RC1_F2
<210> 74 <223> CDR3 sequence of a JG9-TRA 64 variant VP.7751.RC1_F3
<210> 75 <223> CDR3 sequence of a JG9-TRA 64 variant VP.7751.RC1_F4
<210> 76 <223> CDR3 sequence of a JG9-TRA 64 variant VP.7751.RC1_F5
<210> 77 <223> CDR3 sequence of a JG9-TRA 64 variant VP.7751.RC1_F6
<210> 78 <223> CDR3 sequence of a JG9-TRA 64 variant VP.7751.RC1_F7
<210> 79 <223> CDR3 sequence of a JG9-TRA 64 variant VP.7751.RC1_F8
<210> 80 <223> CDR3 sequence of a JG9-TRA 64 variant VP.7751.RC1_G1
<210> 81 <223> CDR3 sequence of a JG9-TRA 64 variant VP.7751.RC1_G2
<210> 82 <223> CDR3 sequence of a JG9-TRA 64 variant VP.7751.RC1_G3
<210> 83 <223> CDR3 sequence of a JG9-TRA 64 variant VP.7751.RC1_G4
<210> 84 <223> CDR3 sequence of a JG9-TRA 64 variant VP.7751.RC1_G5
<210> 85 <223> CDR3 sequence of a JG9-TRA 64 variant VP.7751.RC1_G6
<210> 86 <223> CDR3 sequence of a JG9-TRA 64 variant VP.7751.RC1_G7
<210> 87 <223> CDR3 sequence of a JG9-TRA 64 variant VP.7751.RC1_G8
<210> 88 <223> CDR3 sequence of a JG9-TRA 64 variant VP.7751.RC1_H1
<210> 89 <223> CDR3 sequence of a JG9-TRA 64 variant VP.7751.RC1_H2
<210> 90 <223> CDR3 sequence of a JG9-TRA 64 variant VP.7751.RC1_H3
<210> 91 <223> CDR3 sequence of a JG9-TRA 64 variant VP.7751.RC1_H4
<210> 92 <223> CDR3 sequence of a JG9-TRA 64 variant VP.7751.RC1_H5
<210> 93 <223> CDR3 sequence of a JG9-TRA 64 variant VP.7751.RC1_H6
<210> 94 <223> CDR3 sequence of a JG9-TRA 64 variant VP.7751.RC1_H7
<210> 95 <223> CDR3 sequence of a JG9-TRA 64 variant VP.7751.RC1_H8
<210> 96 <223> pMA-sv40_OE_F1 primer 1.C.2
<210> 97 <223> pMA-sv40_OE_R1 primer 1.C.3
<210> 98 <223> pMA-CMV OE_R1 primer 1.C.5
<210> 99 <223> HLA-A-GT-Rg3 primer 4.A.3
<210> 100 <223> HLA-A-GT-Fg2 primer 4.A.4
<210> 101 <223> HLA-B-GT-Fg2 primer 4.A.7
<210> 102 <223> HLA-B-GT-Rg2 primer 4.B.1
<210> 103 <223> HLA-C-GT-Fg2 primer 4.B.5
<210> 104 <223> HLA-A-02_GT_Rg4 primer 4.I.9
<210> 105 <223> HLA-A-Exon3_HA-RE-BgIII_F1 primer 6.I.9
<210> 106 <223> HLA-C-04-GT-Rg1 primer 8.A.1
<210> 107 <223> CMV-pA-HLA-Ex3_Probe_F1 primer 8.B.2
<210> 108 <223> CMV-pro_GT_R1 primer 9.C.3
<210> 109 <223> sv40pA_GT_F1 primer 9.C.4
<210> 110 <223> AAVS1_GT_F1 primer 9.C.5
<210> 111 <223> AAVS1_GT_F3 primer 9.C.7
<210> 112 <223> AAVS1_GT_F4 primer 9.C.8
<210> 113 <223> AAVS1_GT_R2 primer 9.C.10
<210> 114 <223> AAVS1_GT_R3 promer 9.D.1
<210> 115 <223> AAVS1_GT_R4 primer 9.D.2
<210> 116 <223> HLA-A-intron4_GT_R1 primer 9.D.6
<210> 117 <223> sv40pA-GT-F2 primer 9.D.7
<210> 118 <223> sv40pA-AAVS1-probe-FAM-F1 primer 9.J.2
<210> 119 <223> TRAC_TCRA-ex1_R1 primer 10.A.9
<210> 120 <223> TRAC_TCRA-promoter_F1 primer 10.A. 10
<210> 121 <223> TRAC_probe (HEX) primer 10.B.6
<210> 122 <223> TRAC-GT-F1 primer 1.F.7
<210> 123 <223> sv40pA GT-R1 primer 15.H.2
<210> 124 <223> TRBC2-GT-F1 primer 1.F.9
<210> 125 <223> tGFP-Cterm-Out_SQ_F1 primer 3.J.5
<210> 126 <223> TRAC-GT-F1 ddPCR primer/probe
<210> 127 <223> TRAC-GT-R1 ddPCR primer/probe 1.F.8
<210> 128 <223> TRAC-probe-FAM ddPCR primer/probe
<210> 129 <223> TRBC2-GT-F1 ddPCR primer/probe 1.F.9
<210> 130 <223> TRBC2-GT-R1 ddPCR primer/probe 1.F.10
<210> 131 <223> TRBC2-probe-FAM ddPCR primer/probe 1.G.2
<210> 132 <223> TRAC-TCRA-ex1-F1 ddPCR primer/probe 10.A.9
<210> 133 <223> TRAC-TCRA-ex1-F1 ddPCR primer/probe 10.A.10
<210> 134 <223> TRAC-probe(HEX) ddPCR primer/probe 10.B.6

### List of abbreviations

- **aAPX**: Analyte antigen-presenting complex
- **aAM**: Analyte antigenic molecule
- **APC**: Antigen-presenting cell
- **APX**: Antigen-presenting complex
- **BFP**: Blue fluorescent protein
- **CAR-T**: CAR T-cell
- **CM**: Cargo molecules
- **CRISPR**: Clustered Regularly Interspaced Short Palindromic Repeats
- **gRNA**: Cas9 guide RNA
- **CAR**: Chimeric antigen receptor
- **CDR**: Complementarity-determining regions
- **C-region**: Constant region
- **CMV**: Cytomegalovirus
- **DAMPS**: Danger associated molecular patterns
- **DC**: Dendritic cells
- **DNA**: Deoxyribonucleic acid
- **D-region**: Diversity region
- **eTPC**: Engineered TCR-presenting cell
- **eTPC:A**: eTPC:Antigen system, wherein analyte eTPC-t are combined with analyte antigen
- **eTPC-t**: Engineered TCR-presenting cell that present full-length TCR pairs
- **FACS**: Fluorescence-activated cell sorting
- **GEM T-cells**: Germ line-encoded mycolyl-reactive T-cells
- **GFP**: Green fluorescent protein
- **HLAI**: HLA class I
- **HLAII**: HLA class II
- **HDR**: Homology directed recombination
- **HLA**: Human leukocyte antigen
- **IgSF**: Immunoglobulin superfamily
- **IRES**: Internal ribosome entry site
- **iNK T-cells**: Invariant natural killer T-cells
- **J-region**: Joining region
- **MACS**: Magnetic-activated cell sorting
- **MAGE**: Melanoma associated antigen
- **MAIT**: Mucosal-associated invariant T
- **NCBP**: Non-cell based particles
- **ORF**: Open reading frame
- **PAMPS**: Pathogen-associated molecular patterns
- **PCR**: Polymerase chain reaction
- **RMCE**: Recombinase mediated cassette exchange
- **RFP**: Red fluorescent protein
- **DNA**: Ribonucleic acid
- **SH2**: Src homology 2
- **T-cells**: T lymphocytes
- **TCR**: T-cell Receptor
- **TRA**: TCR alpha
- **TRB**: TCR beta
- **TRD**: TCR delta
- **TCRsp**: TCR surface proteins in complex with CD3
- **TALEN**: Transcription activator-like effector nucleases
- **TRG**: TRC gamma
- **TAA**: Tumour-associated-antigens
- **V-region**: Variable region
- **β2M**: β2-microglobulin
- **ZAP-70**: ζ-chain-associated protein of 70 kDa

### Definitions

**A pair of complementary TCR chains:** two TCR chains wherein the translated proteins are capable of forming a TCRsp on the surface of a TCR presenting cell
**Affinity:** Kinetic or equilibrium parameter of an interaction between two or more molecules or proteins
**Affinity reagent:** Any reagent designed with specific affinity for an analyte. Often used in the context of affinity for TCRsp
**Allele:** Variant form of a given gene
**AM:** Analyte antigenic molecule. Generally, a protein but could also be a metabolite that is expressed by a cell from their genomic DNA and/or a specific introduced genetic sequence. The AM is expressed in the cell and a fragment can then be presented on the cell surface by an APX as cargo or on its own. Either as cargo or not, the AM can then be the target of T-cell receptor bearing cells or related affinity reagents.
**Amplicon:** a piece of DNA or RNA that is the source and/or product of artificial amplification using various methods including PCR.
**Analyte:** an entity that is of interest to be identified and/or measured and/or queried in the combined system
**Analyte antigen:** an antigen comprising one at least one of the following, AM, APX, APX:CM, APX:AM, provided in the form of a soluble reagent, immobilised reagent, presented by an NCBP, presented on the surface of a cell
**Analyte APC:** an analyte cell that presents an analyte antigen
**Antibody:** Affinity molecule that is expressed by specialized cells of the immune system called B-cells and that contains of two chains. B-cells express a very large and very diverse repertoire of antibodies that do generally not bind self proteins but can bind and neutralize pathogens or toxins that would threaten the host. Natural or artificially engineered antibodies are often used as affinity reagents.
**Antigen:** any molecule that may be engaged by a TCR and results in a signal being transduced within the T-cell, often presented by an antigen-presenting complext
**APC:** Antigen-presenting cell. A cell bearing on the surface of the cell an AM, APX, APX
**APX:** Antigen-presenting complex. A protein that is expressed and presented on the cell surface by nucleated cells from genes/ORF encoding genomic DNA and/or a specific introduced genetic sequence. The APX presents a cargo, being either a peptide or other metabolite molecules.
**C-Region:** Constant region. One of the gene segments that is used to assemble the T-cell receptor. The c-region is a distinct segment that rather than driving diversity of the TCR, defines its general function in the immune system.
**Cargo-loading machinery:** Cellular set of proteins that generate and load cargo molecules on APX from proteins or other presented molecules found in the cell.
CDR: complementarity-determining regions. Short sequences on the antigen-facing end of TCRs and antibodies that perform most of the target binding function. Each antibody and TCR contains six CDRs and they are generally the most variable part of the molecules allowing detection of a large number of diverse target molecules.
CM: Cargo molecules. peptide or metabolite that is presented by an antigen-presenting complex for example a HLA I or HLA II. The CM can be expressed by the cell intrinsically from the genomic DNA, introduced into the culture medium or expressed from a specifically introduced genetic sequence.
**Copy-number:** The whole number occurrence of a defined sequence encoded within the genome of a cell
**Cytogenetic:** The study of inheritance in relation to the structure and function of chromosomes, i.e. determine the karyotype of a cell
**Cytotoxic/Cytotoxicity:** Process in which a T-cells releases factors that directly and specifically damage a target cell.
**D-region:** Diversity region. One of the gene segments that is used to assemble the T-cell receptor. Each individual has a large number of different variations of these regions making it possible for each individual to arm T-cells with a very large variety of different TCR.
**DNA:** Desoxyribonucleic acid. Chemical name of the molecule that forms genetic material encoding genes and proteins
**Endogenous:** Substance that originated from within a cell
**Engineered Cell:** A cell whereby the genome has been engineered through genetic modification modified.
**Eukaryotic conditional regulatory element:** A DNA sequence that can influence the activity of a promoter, which may be induced or repressed under defined conditions
**Eukaryotic Promoter:** A DNA sequence that encodes a RNA polymerase biniding site and response elements The sequence of the promoter region controls the binding of the RNA polymerase and transcription factors, therefore promoters play a large role in determining where and when your gene of interest will be expressed.
**Eukaryotic terminator/Signal terminator:** A DNA sequence that are recognized by protein factors that are associated with the RNA polymerase II and which trigger the termination process of transcription. It also encodes the poly-A signal
**eTPC:Antigen system:** eTPC:A, the system in which analyte eTPC-t are combined with analyte antigen
**FACS/Flow Cytometry:** Fluorescence-activated cell sorting. Analytical technique by which individual cells can be analyzed for the expression of specific cell surface and intracellular markers. A variation of that technique, cell sorting, allows cells that carry a defined set of markers to be retrieved for further analysis.
**Family of APX:** A set of several similar genes that encode functionally related proteins, which constitute an antigen pressing complex
**Fluorescent (protein) marker:** Molecule that has specific extinction and emission characteristics and can be detected by Microscopy, FACS and related techniques.
**Genetic Donor vector:** A genetic based vector for delivery of genetic material to the genomic receiver site
**Genomic Receiver Site:** A site within the genome for targeted integration of donor genetic material encoded within a Genetic Donor Vector.
**Heterospecific recombinase sites:** A DNA sequence that is recognized by a recombinase enzyme to promote the crossover of two DNA molecules
**HLA I:** Human Leukocyte Antigen class I. A gene that is expressed in humans in all nucleated cells and exported to the cell surface where it presents as cargo short fragments, peptides, of internal proteins to T-cell receptors. As such it presents fragments of potential ongoing infections along with intrinsic proteins. The HLA I can additionally present as cargo peptides that are added to the culture medium, generated from proteins expressed form introduced genetic elements or generated from proteins that are taken up by the cell. HLA class I genes are polymorphic meaning that different individuals are likely to have variation in the same gene leading to a variation in presentation. Related to HLA class II.
**HLA II:** Human Leukocyte Antigen Class II. A gene that is expressed in humans in specific cells that are coordinating and helping the adaptive immune response for example dendritic cells. Related to HLA class I. HLA class II proteins are exported to the cell surface where they present as cargo short fragments, peptides, of external proteins to T-cell receptors. As such it presents fragments of potential ongoing infections along with intrinsic proteins. The HLA II can additionally present as cargo peptides that are added to the culture medium, generated from proteins expressed form introduced genetic elements or generated from proteins that are taken up by the cell. HLA class II genes are polymorphic meaning that different individuals are likely to have variation in the same gene leading to a variation in presentation.
**Homologous arms:** A stretch of DNA that has near identical sequence identity to a complement homologous arm and therefore promote the exchange of two DNA molecules by the cellular process, homology directed repair.
**Immune surveillance:** Process in which the immune system detects and becomes activated by infections, malignancies or other potentially pathogenic alterations.
**Insulator:** A DNA sequence that prevents a gene from being influenced by the activation or repression of nearby genes. Insulators also prevent the spread of heterochro-matin from a silenced gene to an actively transcribed gene.
**Integration:** The physical ligation of a DNA sequence into a chromosome of a cell
**Integration couple:** A paired genetic donor vector and genomic receiver site
**Internal ribosome entry site (IRES):** A DNA sequence that once transcribed encodes a RNA element that allows the initiation of translation in a cap-independent manner
**J-region:** Joining region. One of the gene segments that is used to assemble the T-cell receptor. Each individual has a large number of different variations of these regions making it possible for each individual to arm T-cells with a very large variety of different TCR.
**Karyotype:** The chromosome composition of a cell
**Kozak Sequence:** Short sequence required for the efficient initiation of translation
**Major HLA class I:** a Family of APX that comprise of the genes HLA-A, HLA-B and HLA-C
**Matched:** When two components encode genetic elements that direct and restrict the interaction between the complemented components
**Meganuclease recognition site:** A DNA sequence that is recognized by a endodeoxyribonuclease, commonly referred to as a meganuclease
**Metabolite:** A molecule created or altered through metabolic pathways of the cell
**Mobile genetic element:** A DNA sequence that can permit the integration of DNA with the activity of transposases enzymes
**Monoclone cell line:** A defined group of cells produced from a single ancestral cell by repeated cellular replication
**Native:** a entity that is naturally occuring to the cell
**Non-coding gene:** A non protein coding DNA sequence that is transcribed into functional non-coding RNA molecules
**ORF:** Open reading frame. Stretch of genetic material that encodes a translation frame for synthesis of a protein (polypeptide) by the ribosome
**Paracrine:** Signalling through soluble factors that directly act on neighboring cells.
**PCR:** Polymerase chain reaction in which a specific target DNA molecule is exponentially amplified
**Peptide:** short string of amino acids between 6 - 30 amino acids in length
**Phenotypic analysis:** Analysis of the observable characteristics of a cell.
**Polymorphic:** Present in different forms in individuals of the same species through the presence of different alleles of the same gene.
**Polypeptide:** Protein consisting of a stretch of peptides, forming a three-dimensional structure.
**Primer:** Short DNA sequence that allows specific recognition of a target DNA sequence for example during a PCR.
**Primary Outputs:** eETP-t cells, analyte APC cells, NCBP or other analyte antigen forms from which the terminal outputs can be derived and/or determined from
**Promoter:** Regulatory DNA element for the controlled initiation of gene expression
**Selectable marker:** A DNA sequence that confers a trait suitable for artificial selection methods
**Shotgun Integration:** The process whereby a library of vectors is introduced to a population of cells, whereby only a single copy of any given vector insert may be integrated to the genome of each single cell. Used to refer to pooled vector integration to a given cell population via an integration couple
**Slice acceptor site:** A DNA sequence at the 3' end of the intron AM, APX CM or affinity reagent for interaction with cells with TCRsp on the surface, or TCRsp based reagents
**Slice donor site:** A DNA sequence at the 5' end of the intron
**Synthetic:** an entity that is artificially generated and introduced to a cell
**T-cell:** T lymphocyte. White blood cell that expresses a T-cell receptor on its surface. Selected by the immune system to not react with the own body but have the potential to recognize infections and malignancies as well as reject grafts from most members of the same species.
**TCR:** T-cell Receptor. Affinity molecule expressed by a subgroup of lymphocytes called T-lymphocytes. In humans the TCR recognizes cargo presented by APX CM or APX AM, including fragments from virus or bacterial infections or cancerous cells. Therefore, the TCR recognition is an integral part of the adaptive immune system. The TCR consists of two chains that are paired on the cell surface. The TCR expressed on the surface of each cells is assembled at random from a large pool of varied genes (the v,d,j and c regions) and thus each individual has a pool of T-cells expressing a very large and diverse repertoire of different TCRs.
**TCRsp:** A pair of complementary TCR chains that express as surface proteins in complex with CD3 or a pair of complementary TCR chains expressed as proteins in the form of a soluble reagent, an immobilised reagent or present by NCBP.
**Terminal Outputs:** analyte antigen and TCR sequences, in the form of AM, APX, APX:CM, APX:AM, affinity reagents or TCRsp
**TRA:** TCR alpha encoding locus. One of the four different locus encoding genes that can form a VDJ recombined TCR chain. Translated TCR alpha chain proteins typically pair with translated TCR beta chain proteins to form alpha/beta TCRsp.
**TRB:** TCR beta encoding locus. One of the four different locus encoding genes that can form a VDJ recombined TCR chain. Translated TCR beta chain proteins typically pair with TCR alpha chain proteins to form alpha/beta TCRsp.
**TRD:** TCR delta encoding locus. One of the four different locus encoding genes that can form a VDJ recombined TCR chain. Translated TCR delta chain proteins typically pair with translated TCR gamma chain proteins to form gamma/delta TCRsp.
**TRG:** TCR gamma encoding locus. One of the four different locus encoding genes that can form a VDJ recombined TCR chain. Translated TCR gamma chain proteins typically pair with translate TCR delta chain proteins to form gamma/delta TCRsp.
**V-region:** Variable region. One of the gene segments that is used to assemble the T-cell receptor. Each individual has a large number of different variations of these regions making it possible for each individual to arm T-cells with a very large variety of different TCR.

## Claims

1. A multicomponent system for generating a population of engineered TCR-presenting cells (eTPC-t), for use in discovery and characterisation of TCRs and T-cell antigens
comprising
a first component which is an engineered TCR-presenting cell (eTPC), designated component A, wherein component A lacks endogenous surface expression of at least one family of analyte antigen-presenting complexes (aAPX) and/or analyte antigenic molecule (aAM)wherein the lack of surface expression is selected as to minimise interference in matched analyses of target analyte antigens , lacks endogenous expression of TCR chains alpha, beta, delta and gamma, and expresses CD3 proteins and contains two further single components designated B and D, which are different and each respectively a genomic receiver site for integration of a single ORF encoding at least one analyte TCR chain of alpha, beta, delta or gamma, and/or two ORFs encoding a pair of analyte TCR chains, and wherein the genomic receiver sites B and D are each selected from
a. A synthetic construct designed for recombinase mediated cassette exchange (RMCE)
b. A synthetic construct designed for site directed homologous recombination;
and
a second component is a genetic donor vector, **designated component C,** for site directed integration of ORF encoding analyte TCR chains, wherein **component C,** is matched to component **B,** and wherein the component **C** is designed to deliver
c. A single ORF encoding at least one analyte TCR chain of alpha, beta, delta and/or gamma and/or
d. Two ORFs encoding a pair of analyte TCR chains.
and wherein c and/or d optionally encodes a selection marker of integration, such that the analyte TCR chains can be expressed as TCR surface protein in complex with the CD3 **(TCRsp)** on component **A.**

2. A multicomponent system according to **claim 1** wherein the family of **aAPX** may be any of the following
a. HLA class I
b. HLA class II
c. non-HLA antigen-presenting complex.

3. A multicomponent system according to **claims 1 or 2,** wherein a further component **designated E** is a genetic vector matched to **D,** wherein component **E** is designed to deliver a single ORF encoding one analyte TCR chain of alpha, beta, delta or gamma, such that when a ORF encoding the complementary paired analyte TCR chain of alpha, beta, delta or gamma is integrated into component **B,** expressed as **TCRsp** on component **A** and optionally wherein component **E** encodes a selection marker of integration.

4. A multicomponent system according to **any of the preceding claims,**
wherein component **A,** further contains a component **designated F,** a synthetic genomic TCR-stimulation response element selected from
a. A single component synthetic construct containing at least one native promoter and/or at least one synthetic promoter and at least one reporter
b. A multi-component synthetic construct designed with at least one native promoter and/or at least one synthetic promoter and at least one reporter.
and wherein activation of a and/or b is dependent on at least one signal transduction pathway selected from a synthetic pathway, a native pathway or a combination thereof.

5. A multicomponent system according to claim 1, wherein the genomic receiver sites B and D are included and are both a synthetic construct designed for recombinase mediated cassette exchange (RMCE).

6. A multicomponent system according to any of the preceding **claims**
wherein the **aAM** is selected from
a. a polypeptide or complex of polypeptides translated from the analyte antigenic molecule ORF(s)
b. a peptide derived from a polypeptide translated from the analyte antigenic molecule ORF(s)
c. a peptide derived from altering the component A proteome
d. a polypeptide derived from altering the component A proteome
e. a metabolite derived from altering the component A metabolome.

7. A multicomponent system according to any of **the preceding claims** wherein component **A** expresses CD4 and/or CD8.

8. A multicomponent system according to any of **the preceding claims** wherein component **A** expresses additional TCR co-receptors.

9. A multicomponent system according to **claim 8** wherein component **A** expresses CD28 and/or CD45.

10. A multicomponent system according to **any of claims 2-9** wherein the **component B and/or D** is included and comprises of at least one of the following genetic elements
a. Heterospecific recombinase sites
b. Homologous arms
c. Eukaryotic promoter
d. Eukaryotic conditional regulatory element
e. Eukaryotic terminator
f. Selection marker
g. Splice acceptor site
h. Splice donor site
i. Non-protein coding gene
j. Insulator
k. Mobile genetic element
l. Meganuclease recognition site
m. Internal ribosome entry site (IRES)
n. Viral self-cleaving peptide element.
o. A Kozak consensus sequence

11. A multicomponent system according to any of **preceding claims** wherein the **component C and/or E** is included and comprises of at least one of the following genetic elements
a. Heterospecific recombinase sites
b. Homologous arms
c. Eukaryotic promoter
d. Eukaryotic conditional regulatory element
e. Eukaryotic terminator
f. Selection marker
g. Selection marker of integration
h. Splice acceptor site
i. Splice donor site
j. Non-protein coding gene
k. Insulator
l. Mobile genetic element
m. Meganuclease recognition site
n. Internal ribosome entry site (IRES)
o. A viral self-cleaving peptide element
p. An antibiotic resistance cassette
q. A bacterial origin of replication
r. A yeast origin of replication
s. A cloning site.
t. A kozak consensus sequence

12. A multicomponent system according to **any of claims 1-11,** wherein the component **B** and/or **D** is included and is for RMCE integration of a single ORF and comprises:
a. A Eukaryotic promoter
b. A pair of heterospecific recombinase sites
c. A Kozak consensus sequence
d. A selection marker
e. A Eukaryotic terminator.

13. A multicomponent system according to **any of the preceding claims** wherein component **C and/or E** is present is for RMCE integration of a single ORF and comprises the following genetic elements:
a. A pair of heterospecific recombinase sites
b. A Kozak consensus sequence
c. An antibiotic resistance cassette
d. A bacterial of replication
e. A cloning site for introduction of a single ORF encoding one or more TCR chains and/or selection marker of integration.

14. A multicomponent system according to **any of the preceding** claims wherein component C and/or E is included and is capable of being combined with at least one ORF encoding at least one analyte TCR chain to obtain component C' and/or E'.

15. A multicomponent system according to **claim 14** wherein the combination can be performed multiple times to obtain a library of component C' and/or E'.

16. A multicomponent system according to claim 14 **or 15,** wherein the analyte TCR chain encoding sequences are derived from
a. Paired cloning of TCR chain ORF sequence(s) from primary T-cells
b. Unpaired cloning of TCR chain ORF sequence(s) from primary T-cells
c. Synthetic TCR chain ORF sequence(s).

17. A multicomponent system according to any of **claims 14-16,** wherein one or more component C' and/or E' can be combined with component A, to integrate two complementary analyte TCR chains encoded in component C' and/or E', into components **B** and/or **D,** to obtain a cell, **designated an eTPC-t,** wherein components B and/or D become components B' and/or D' such that it expresses a **TCRsp** on the surface of component A.

18. A multicomponent system according to any of **claims 14-17,** wherein one of component C' or E' can be combined with component A, to integrate one analyte TCR chain encoded in component C' or E', into components **B** or **D,** to obtain a cell, **designated eTPC-x,** wherein components B or D become components B' or D' such that the eTPC-x expresses a single TCR chain.

19. A multicomponent system according to any of **claims 15-18,** wherein one of component C' or E' can be combined with an **eTPC-x,** to integrate one analyte TCR chain encoded in component C' or E' that is complementary to the TCR chain expressed in the **eTPC-x,** into components **B** or **D,** of the **eTPC-x,** to obtain an **eTPC-t,** wherein components B or D become components B' or D' such that it expresses an **TCRsp** on the surface of the eTPC-t.

20. A method for preparing an eTPC-t as defined in **claim 17,** the method comprising
a. providing the multicomponent system according to **claim 1,**
b. Combining component A, with at least one of component C' and/or E', wherein the one or more component C' and/or E' encode one or more pairs of complementary analyte TCR chains, and combining with integration factors and at least one of
c. Selecting for loss of genomic receiver site selection marker(s)
d. Selecting for gain of a surface expression of the **TCRsp** and/or one or more **CD3** proteins
e. Selecting for gain of one or more of a selection marker of integration.

21. A method according to **claim 20,** wherein the one or more component C' and/or E' encodes a single complementary pair of TCR chains in step b.

22. A method according to **claim 21,** wherein the method is conducted multiple times wherein each time step b is performed using a distinct single complementary pair of TCR chains, such that a distinct **eTPC-t** is obtained, to obtain a library of discrete and defined **eTPC-t.**

23. A method according to **claim 21-22** wherein the one or more component C' and/or E' encodes a mixed pool of two or more distinct complementary pair of TCR chains in step b of claim 22, to obtain a library, wherein the library is comprised of a mixed population of **eTPC-t,** wherein each **eTPC-t** expresses a single **TCRsp** derived from the pool used in step b of claim 23.

24. A method for preparing an **eTPC-x** as defined in **claim 18** the method comprising
a. providing the multicomponent system according to **claim 1,**
b. Combining component A, with one or more of component C' or one or more component E', wherein one or more component C' or one or more E' encodes an analyte TCR chain, and combining with integration factors and at least one of
c. Selecting for loss of genomic receiver site selection marker(s)
d. Selecting for gain of one or more of a selection marker of integration.

25. A method according to **claim 24,** wherein one component C' or one component E' encodes a single analyte TCR chain in step b.

26. A method according to **claim 25** wherein the method is conducted multiple times wherein each time step b is performed using a distinct analyte TCR chain, such that a distinct **eTPC-x** is obtained, to obtain a library of discrete and defined **eTPC-x.**

27. A method according to **claim** 25 **or 26,** wherein one or more component C' or one or more component E' encodes a mixed pool of two or more single analyte TCR chains in step b, to obtain a library, wherein the library is comprised of a mixed population of **eTPC-x,** wherein each **eTPC-x** expresses a single **analyte TCR chain** derived from the pool used in step b.

28. A method for preparing an **eTPC-t** as defined in **claim 18** the method comprising
a. providing the multicomponent system according to **claim 1,**
b. Combining an **eTPC-x,** with one or more of component C' or one or more component E', wherein one or more component C' or one or more E' encodes an analyte TCR chain that is complementary to the integrated analyte TCR chain in the **eTPC-x,** and combining with integration factors and at least one of
c. Selecting for loss of genomic receiver site selection marker(s)
d. Selecting for gain of a surface expression of the **TCRsp** and/or one or more **CD3** proteins
e. Selecting for gain of one or more of a selection marker of integration.

29. A method according to **claim 28,** wherein one component C' or one component E' encodes a single analyte TCR chain in step b.

30. A method according to **claim 29,** wherein the method is conducted multiple times, wherein each time step b is performed using a distinct analyte TCR chain, such that a distinct **eTPC-t** is obtained expressing a **TCRsp,** to obtain a library of discrete and defined **eTPC-t.**

31. A method according to **claim 28-29** wherein one or more component C' or one or more component E' encodes a mixed pool of two or more single analyte TCR chains in step b, to obtain a library, wherein the library is comprised of a mixed population of **eTPC-t,** wherein each **eTPC-t** expresses a single **TCRsp** derived from the pool used in step b.

32. An eTPC-t obtained from the method of claim 20 for use in an eTPC:A analytical device for characterisation of
a. specificity of the expressed analyte **TCRsp** to an **analyte antigen** and/or
b. affinity of the expressed analyte **TCRsp** to an **analyte antigen** and/or
c. a signal response of the expressed analyte **TCRsp** to an **analyte antigen.**
wherein an analyte antigen is selected from an **aAPX:aAM** and/or **aAM** and/or **aAPX** and/or **aAPX:CM** and/or **affinity reagent,** wherein the analyte antigen is in the form of at least of the following, a soluble reagent, an immobilised reagent, presented by a non-cell based particle **(NCBP),** presented on the surface of a cell **(analyte APC),** wherein **aAPX:CM** is an **aAPX** that is loaded with a cargo molecule **(CM),** and wherein **aAPX:aAM** is an **aAPX** is loaded with an **aAM** as **CM.**

33. An analyte eTPC obtainable from the multicomponent system as defined in any of claims 1-19 **characterized in that** it
a. Contains 2 genomic receiver sites, designated B and D, each of which may comprise a single ORF encoding at least one analyte TCR chain of alpha, beta, delta or gamma, and/or two ORFs encoding a pair of analyte TCR chains
b. Expresses CD3 proteins
c. Does not express any other TCR chains than those comprised in B and D,
and wherein the genomic receiver sites B and D are each selected from
a. A synthetic construct designed for recombinase mediated cassette exchange (RMCE)
b. A synthetic construct designed for site directed homologous recombination
and wherein the analyte eTPC has been combined with at least one of C' and/or E' as defined in any one of claims 17 to 19 so that at least one of component B and D has become component B' and/or D', *wherein integration results in the expression of analyte TCR chains by the target eTPC.*

## Patentansprüche

1. Mehrkomponentensystem zum Erzeugen einer Population gentechnisch veränderter TCR-präsentierender Zellen (eTPC-t) zur Verwendung beim Nachweis und der Charakterisierung von TCRs und T-Zell-Antigenen,
umfassend
eine erste Komponente, bei der es sich um eine gentechnisch veränderte TCRpräsentierende Zelle (eTPC) handelt, die als Komponente A bezeichnet wird, wobei Komponente A endogene Oberflächenexpression von mindestens einer Familie von Analyt-Antigen-präsentierenden Komplexen (aAPX) und/oder Analyt-Antigen-Molekülen (aAM) fehlt, wobei das Fehlen von Oberflächenexpression so ausgewählt ist, dass es Interferenz in abgestimmten Analysen von Ziel-Analyt-Antigenen minimiert, endogene Expression von TCR-Ketten Alpha, Beta, Delta und Gamma fehlt und CD3-Proteine exprimiert und zwei weitere Einzelkomponenten enthält, die als B und D bezeichnet werden, die unterschiedlich und jeweils eine genomische Empfängerstelle zur Integration eines einzelnen ORF, der mindestens eine Analyt-TCR-Kette von Alpha, Beta, Delta oder Gamma codiert, und/oder zwei ORFs sind, die ein Paar Analyt-TCR-Ketten codieren, und wobei die genomischen Empfängerstellen B und D jeweils ausgewählt sind aus
a. einem synthetischen Konstrukt, das für Rekombinase-vermittelten Kassettenaustausch (RMCE) konzipiert ist
b. einem synthetischen Konstrukt, das für ortsgerichtete homologe Rekombination konzipiert ist;
und
eine zweite Komponente ein genetischer Donatorvektor ist, der als **Komponente C bezeichnet** wird, zur ortsgerichteten Integration von ORF, der Analyt-TCR-Ketten codiert, wobei **Komponente C** auf Komponente **B** abgestimmt ist, und wobei die Komponente **C** so konzipiert ist, dass sie Folgendes liefert
c. einen einzelnen ORF, der mindestens eine Analyt-TCR-Kette von Alpha, Beta, Delta und/oder Gamma codiert, und/oder
d. zwei ORFs, die ein Paar Analyt-TCR-Ketten codieren,
und wobei c und/oder d gegebenenfalls einen Integrationsselektionsmarker so codieren, dass die Analyt-TCR-Ketten als TCR-Oberflächenproteine im Komplex mit dem CD3 **(TCRsp)** auf Komponente **A** exprimiert werden können.

2. Mehrkomponentensystem nach **Anspruch 1,** wobei die Familie der **aAPX** eines der Folgenden sein kann
a. HLA-Klasse I
b. HLA-Klasse II
c. nicht-HLA-Antigen-präsentierender Komplex.

3. Mehrkomponentensystem nach den **Ansprüchen 1 oder 2,** wobei eine weitere Komponente, die als **E bezeichnet** wird, ein genetischer Vektor ist, der auf **D** abgestimmt ist, wobei Komponente **E** so konzipiert ist, dass sie einen einzelnen ORF liefert, der eine Analyt-TCR-Kette von Alpha, Beta, Delta oder Gamma codiert, sodass, wenn ein ORF, der die komplementäre gepaarte Analyt-TCR-Kette von Alpha, Beta, Delta oder Gamma codiert, in Komponente **B** integriert wird, als **TCRsp** auf Komponente **A** exprimiert wird, und gegebenenfalls wobei Komponente **E** einen Integrationsselektionsmarker codiert.

4. Mehrkomponentensystem nach **einem der vorstehenden Ansprüche,** wobei Komponente **A** weiter eine Komponente, die als **F bezeichnet** wird, ein synthetisches genomisches TCR-Stimulationsreaktionselement enthält, ausgewählt aus
a. einem synthetischen Einkomponentenkonstrukt, das mindestens einen nativen Promotor und/oder mindestens einen synthetischen Promotor und mindestens einen Reporter enthält,
b. einem synthetischen Mehrkomponentenkonstrukt, das mit mindestens einem nativen Promotor und/oder mindestens einem synthetischen Promotor und mindestens einem Reporter konzipiert ist,
und wobei Aktivierung von a und/oder b von mindestens einem Signaltransduktionsweg abhängig ist, ausgewählt aus einem synthetischen Weg, einem nativen Weg oder einer Kombination davon.

5. Mehrkomponentensystem nach Anspruch 1, wobei die genomischen Empfängerstellen B und D inkludiert sind und beide ein synthetisches Konstrukt sind, das für Rekombinase-vermittelten Kassettenaustausch (RMCE) konzipiert ist.

6. Mehrkomponentensystem nach einem der vorstehenden **Ansprüche,** wobei das **aAM** ausgewählt ist aus
a. einem Polypeptid oder Komplex von Polypeptiden, das/der aus dem/den Analyt-Antigenmolekül-ORF(s) translatiert ist
b. einem Peptid, das von einem Polypeptid abgeleitet ist, das aus dem/den Analyt-Antigenmolekül-ORF(s) translatiert ist
c. einem Peptid, das aus einer Veränderung des Proteoms der Komponente A abgeleitet ist
d. einem Polypeptid, das aus einer Veränderung des Proteoms der Komponente A abgeleitet ist
e. einem Metaboliten, der aus einer Veränderung des Metaboloms der Komponente A abgeleitet ist.

7. Mehrkomponentensystem nach einem **der vorstehenden Ansprüche,** wobei Komponente **A** CD4 und/oder CD8 exprimiert.

8. Mehrkomponentensystem nach einem **der vorstehenden Ansprüche,** wobei Komponente **A** zusätzliche TCR-Co-Rezeptoren exprimiert.

9. Mehrkomponentensystem nach **Anspruch 8,** wobei Komponente **A** CD28 und/oder CD45 exprimiert.

10. Mehrkomponentensystem nach **einem der Ansprüche 2-9,** wobei die **Komponente B und/oder D** inkludiert ist und mindestens eines der folgenden genetischen Elemente umfasst
a. heterospezifische Rekombinasestellen
b. homologe Arme
c. eukaryotischen Promotor
d. eukaryotisches bedingtes regulatorisches Element
e. eukaryotischen Terminator
f. Selektionsmarker
g. Splice-Akzeptorstelle
h. Splice-Donatorstelle
i. nicht-proteincodierendes Gen
j. Isolator
k. mobiles genetisches Element
l. Meganuklease-Erkennungsstelle
m. interne Ribosomeneintrittsstelle (IRES)
n. virales selbstspaltendes Peptidelement
o. eine Kozak-Konsenssequenz

11. Mehrkomponentensystem nach einem der **vorstehenden Ansprüche,** wobei die **Komponente C und/oder E** inkludiert ist und mindestens eines der folgenden genetischen Elemente umfasst
a. heterospezifische Rekombinasestellen
b. homologe Arme
c. eukaryotischen Promotor
d. eukaryotisches bedingtes regulatorisches Element
e. eukaryotischen Terminator
f. Selektionsmarker
g. Integrationsselektionsmarker
h. Splice-Akzeptorstelle
i. Splice-Donatorstelle
j. nicht-proteincodierendes Gen
k. Isolator
l. mobiles genetisches Element
m. Meganuklease-Erkennungsstelle
n. interne Ribosomeneintrittsstelle (IRES)
o. ein virales selbstspaltendes Peptidelement
p. eine Antibiotikaresistenzkassette
q. einen bakteriellen Replikationsursprung
r. einen Hefe-Replikationsursprung
s. eine Klonierungsstelle
t. eine Kozak-Konsenssequenz

12. Mehrkomponentensystem nach **einem der Ansprüche 1-11,** wobei die Komponente **B** und/oder **D** inkludiert ist und der RMCE-Integration eines einzelnen ORF dient und umfasst:
a. einen eukaryotischen Promotor
b. ein Paar heterospezifischer Rekombinasestellen
c. eine Kozak-Konsenssequenz
d. einen Selektionsmarker
e. einen eukaryotischen Terminator.

13. Mehrkomponentensystem nach **einem der vorstehenden Ansprüche,** wobei Komponente **C und/oder E** präsent ist, der RMCE-Integration eines einzelnen ORF dient und die folgenden genetischen Elemente umfasst:
a. ein Paar heterospezifischer Rekombinasestellen
b. eine Kozak-Konsenssequenz
c. eine Antibiotikaresistenzkassette
d. ein Replikationsbakterium
e. eine Klonierungsstelle zum Einführen eines einzelnen ORF, der eine oder mehrere TCR-Ketten codiert, und/oder Integrationsselektionsmarker.

14. Mehrkomponentensystem nach **einem der vorstehenden** Ansprüche, wobei Komponente C und/oder E inkludiert ist und mit mindestens einem ORF kombiniert werden kann, der mindestens eine Analyt-TCR-Kette codiert, um Komponente C' und/oder E' zu erhalten.

15. Mehrkomponentensystem nach **Anspruch 14,** wobei die Kombination mehrmals durchgeführt werden kann, um eine Bibliothek von Komponente C' und/oder E' zu erhalten.

16. Mehrkomponentensystem nach Anspruch 14 **oder 15,** wobei die Analyt-TCR-Ketten codierenden Sequenzen abgeleitet sind von
a. gepaarter Klonierung von TCR-Ketten-ORF-Sequenz(en) aus primären T-Zellen
b. ungepaarter Klonierung von TCR-Ketten-ORF-Sequenz(en) aus primären T-Zellen
c. synthetischen TCR-Ketten-ORF-Sequenz(en).

17. Mehrkomponentensystem nach einem der **Ansprüche 14-16,** wobei eine oder mehrere Komponenten C' und/oder E' mit Komponente A kombiniert werden können, um zwei komplementäre Analyt-TCR-Ketten, die in Komponente C' und/oder E' codiert werden, in Komponenten **B** und/oder **D** zu integrieren, um eine Zelle zu erhalten, die als **eTPC-t bezeichnet** wird, wobei Komponenten B und/oder D zu Komponente B' und/oder D' werden, sodass sie einen **TCRsp** an der Oberfläche von Komponente A exprimieren.

18. Mehrkomponentensystem nach einem der **Ansprüche 14-17,** wobei eine von Komponente C' oder E' mit Komponente A kombiniert werden kann, um eine Analyt-TCR-Kette, die in Komponente C' oder E' codiert wird, in Komponente **B** oder **D** zu integrieren, um eine Zelle zu erhalten, die als **eTPC-x bezeichnet** wird, wobei Komponente B oder D zu Komponente B' oder D' werden, sodass die eTPC-x eine einzelne TCR-Kette exprimiert.

19. Mehrkomponentensystem nach einem der **Ansprüche 15-18,** wobei eine von Komponente C' oder E' mit einer **eTPC-x** kombiniert werden kann, um eine in Komponente C' oder E' codierte Analyt-TCR-Kette, die zu der in der **eTPC-x** exprimierten TCR-Kette komplementär ist, in Komponente **B** oder **D** der **eTPC-x** zu integrieren, um eine **eTPC-t** zu erhalten, wobei Komponente B oder D zu Komponente B' oder D' werden, sodass sie eine **TCRsp** an der Oberfläche der eTPC-t exprimieren.

20. Verfahren zum Herstellen einer eTPC-t wie in **Anspruch 17** definiert, wobei das Verfahren umfasst
a. Bereitstellen des Mehrkomponentensystems nach **Anspruch 1,**
b. Kombinieren von Komponente A mit mindestens einer von Komponente C' und/oder E', wobei die eine oder die mehreren Komponenten C' und/oder E' ein oder mehrere Paare komplementärer Analyt-TCR-Ketten codieren, und Kombinieren mit Integrationsfaktoren und mindestens einem von
c. Selektion für Verlust von genomischen Empfängerstellen-Selektionsmarker(n)
d. Selektion für Verstärkung einer Oberflächenexpression des **TCRsp** und/oder eines oder mehrerer **CD3**-Proteine
e. Selektion für Verstärkung eines oder mehrerer von einem Integrationsselektionsmarker.

21. Verfahren nach **Anspruch 20,** wobei die eine oder die mehreren Komponenten C' und/oder E' ein einzelnes komplementäres Paar TCR-Ketten in Schritt b codieren.

22. Verfahren nach **Anspruch 21,** wobei das Verfahren mehrmals vorgenommen wird, wobei Schritt b jedes Mal unter Verwendung eines unterschiedlichen einzelnen komplementären Paars TCR-Ketten durchgeführt wird, sodass eine unterschiedliche **eTPC-t** erhalten wird, um eine Bibliothek diskreter und definierter **eTPC-t** zu erhalten.

23. Verfahren nach **Anspruch 21-22,** wobei die eine oder die mehreren Komponenten C' und/oder E' einen gemischten Pool aus zwei oder mehreren unterschiedlichen komplementären Paaren TCR-Ketten in Schritt b von Anspruch 22 codieren, um eine Bibliothek zu erhalten, wobei die Bibliothek eine gemischte Population von **eTPC-t** umfasst, wobei jede **eTPC-t** einen einzelnen **TCRsp** exprimiert, der aus dem in Schritt b von Anspruch 23 verwendeten Pool abgeleitet ist.

24. Verfahren zum Herstellen einer **eTPC-x,** wie in **Anspruch 18** definiert, wobei das Verfahren umfasst
a. Bereitstellen des Mehrkomponentensystems nach **Anspruch 1,**
b. Kombinieren von Komponente A mit einer oder mehreren von Komponente C' oder einer oder mehreren Komponenten E', wobei eine oder mehrere Komponenten C' oder eine oder mehrere E' eine Analyt-TCR-Kette codieren, und Kombinieren mit Integrationsfaktoren und mindestens einem von
c. Selektion für Verlust von genomischen Empfängerstellen-Selektionsmarker(n)
d. Selektion für Verstärkung eines oder mehrerer von einem Integrationsselektionsmarker.

25. Verfahren nach **Anspruch 24,** wobei eine Komponente C' oder eine Komponente E' in Schritt b eine einzelne Analyt-TCR-Kette codiert.

26. Verfahren nach **Anspruch 25,** wobei das Verfahren mehrmals vorgenommen wird, wobei Schritt b jedes Mal unter Verwendung einer unterschiedlichen Analyt-TCR-Kette durchgeführt wird, sodass eine unterschiedliche **eTPC-x** erhalten wird, um eine Bibliothek diskreter und definierter **eTPC-x** zu erhalten.

27. Verfahren nach **Anspruch** 25 **oder 26,** wobei eine oder mehrere Komponenten C' oder eine oder mehrere Komponenten E' in Schritt b einen gemischten Pool von zwei oder mehr einzelnen Analyt-TCR-Ketten codieren, um eine Bibliothek zu erhalten, wobei die Bibliothek eine gemischte Population von **eTPC-x** umfasst, wobei jede **eTPC-x** eine einzelne **Analyt-TCR-Kette** exprimiert, die aus dem in Schritt b verwendeten Pool abgeleitet ist.

28. Verfahren zum Herstellen einer **eTPC-t,** wie in **Anspruch 18** definiert, wobei das Verfahren umfasst
a. Bereitstellen des Mehrkomponentensystems nach **Anspruch 1,**
b. Kombinieren einer **eTPC-x** mit einer oder mehreren von Komponente C' oder einer oder mehreren Komponenten E', wobei eine oder mehrere Komponenten C' oder eine oder mehrere E' eine Analyt-TCR-Kette codieren, die zu der integrierten Analyt-TCR-Kette in der **eTPC-x** komplementär ist, und Kombinieren mit Integrationsfaktoren und mindestens einem von
c. Selektion für Verlust von genomischen Empfängerstellen-Selektionsmarker(n)
d. Selektion für Verstärkung einer Oberflächenexpression des **TCRsp** und/oder eines oder mehrerer **CD3**-Proteine
e. Selektion für Verstärkung eines oder mehrerer von einem Integrationsselektionsmarker.

29. Verfahren nach **Anspruch 28,** wobei eine Komponente C' oder eine Komponente E' in Schritt b eine einzelne Analyt-TCR-Kette codiert.

30. Verfahren nach **Anspruch 29,** wobei das Verfahren mehrmals vorgenommen wird, wobei Schritt b jedes Mal unter Verwendung einer unterschiedlichen Analyt-TCR-Kette durchgeführt wird, sodass eine unterschiedliche **eTPC-t** erhalten wird, die einen **TCRsp** exprimiert, um eine Bibliothek diskreter und definierter **eTPC-t** zu erhalten.

31. Verfahren nach **Anspruch 28-29,** wobei eine oder mehrere Komponenten C' oder eine oder mehrere Komponenten E' in Schritt b einen gemischten Pool von zwei oder mehr einzelnen Analyt-TCR-Ketten codieren, um eine Bibliothek zu erhalten, wobei die Bibliothek eine gemischte Population von **eTPC-t** umfasst, wobei jede **eTPC-t** einen einzelnen **TCRsp** exprimiert, der aus dem in Schritt b verwendeten Pool abgeleitet ist.

32. eTPC-t, die aus dem Verfahren nach Anspruch 20 erhalten wird, zur Verwendung in einer eTPC:A-Analysevorrichtung zur Charakterisierung von
a. Spezifität des exprimierten Analyten-**TCRsp** gegenüber einem **Analyt-Antigen,** und/oder
b. Affinität des exprimierten Analyten-**TCRsp** zu einem **Analyt-Antigen,** und/oder
c. einer Signalantwort des exprimierten Analyten-**TCRsp** auf ein **Analyt-Antigen,**
wobei ein Analyt-Antigen ausgewählt ist aus einem **aAPX:aAM** und/oder **aAM** und/oder **aAPX** und/oder **aAPX:CM** und/oder **Affinitätsreagenz,** wobei das Analyt-Antigen in Form von mindestens dem Folgendem vorliegt, einem löslichen Reagenz, einem immobilisierten Reagenz, durch ein nicht auf Zellen basierendes Partikel **(NCBP)** präsentiert, an der Oberfläche einer Zelle **(Analyt-APC)** präsentiert, wobei **aAPX:CM** ein **aAPX** ist, das mit einem Frachtmolekül **(CM)** beladen ist, und wobei **aAPX:aAM** ein **aAPX** ist, das mit einem **aAM** als **CM** beladen ist.

33. Analyt-eTPC, die aus dem Mehrkomponentensystem wie in einem der Ansprüche 1-19 definiert erhalten werden kann, **dadurch gekennzeichnet, dass** sie
a. 2 genomische Empfängerstellen enthält, die als B und D bezeichnet werden, von denen jede einen einzelnen ORF, der mindestens eine Analyt-TCR-Kette von Alpha, Beta, Delta oder Gamma codiert, und/oder zwei ORFs umfassen kann, die ein Paar Analyt-TCR-Ketten codieren
b. CD3-Proteine exprimiert
c. keine anderen TCR-Ketten als die in B und D enthaltenen exprimiert,
und wobei die genomischen Empfängerstellen B und D jeweils ausgewählt sind aus
a. einem synthetischen Konstrukt, das für Rekombinase-vermittelten Kassettenaustausch (RMCE) konzipiert ist
b. einem synthetischen Konstrukt, das für ortsgerichtete homologe Rekombination konzipiert ist
und wobei die Analyt-eTPC mit mindestens einem von C' und/oder E' kombiniert wurde, wie in einem der Ansprüche 17 bis 19 definiert, sodass mindestens eine von Komponente B und D zu Komponente B' und/oder D' geworden ist, *wobei die Integration zur Expression von Analyt-TCR-Ketten durch die Ziel-eTPC führt.*

## Revendications

1. Système multicomposant pour générer une population de cellules présentatrices de TCR modifiées (eTPC-t), destiné à être utilisé pour la découverte et la caractérisation de TCR et d'antigènes de lymphocytes T
comprenant
un premier composant, qui est une cellule présentatrice de TCR modifiée (eTPC), appelé composant A, dans lequel le composant A est dépourvu d'expression de surface endogène d'au moins une famille de complexes présentateurs d'antigène analyte (aAPX) et/ou d'une molécule antigénique d'analyte (aAM), dans lequel l'absence d'expression est sélectionnée de manière à réduire au minimum l'interférence entre analyses appariées d'antigènes d'analyte cibles, est dépourvu d'expression endogène des chaînes alpha, bêta, delta et gamma TCR, et exprime les protéines CD3 et contient deux autres composants individuels appelés B et D, qui sont différents et chacun, respectivement, un site récepteur génomique pour l'intégration d'un seul ORF codant pour au moins une chaîne alpha, bêta, delta ou gamma TCR d'analyte, et/ou deux ORF codant pour une paire de chaînes TCR d'analyte, et dans lequel les sites récepteurs génomiques B et D sont chacun sélectionnés parmi
a. une construction synthétique conçue pour l'échange de cassettes médié par recombinase (RMCE)
b. une construction synthétique conçue pour la recombinaison homologue dirigée ;
et
un second composant est un vecteur donneur génétique, **appelé composant C,** destiné à l'intégration dirigée d'un ORF codant pour des chaînes TCR d'analyte, dans lequel **le composant** C est adapté au composant B et dans lequel le composant C est conçu pour produire
c. un seul ORF codant pour au moins une chaîne alpha, bêta, delta ou gamma TCR d'analyte, et/ou
d. deux ORF codant pour une paire de chaînes TCR d'analyte,
et dans lequel c et/ou d code(nt) éventuellement pour un marqueur de sélection d'intégration, de sortie que les chaînes TCR d'analyte puissent être exprimée sous forme de protéines de surface de TCR en complexe avec le CD3 **(TCRsp)** sur le composant A.

2. Système multicomposant selon **la revendication 1,** dans lequel la famille **d'aAPX** peut être l'une quelconque des suivantes
a. HLA classe I
b. HLA classe II
c. complexe présentateur d'antigène non HLA.

3. Système multicomposant selon **la revendication 1 ou 2,** dans lequel un autre composant **appelé E** est un vecteur génétique adapté à **D,** dans lequel le composant **E** est conçu pour produire un ORF unique codant pour une chaîne alpha, bêta, delta ou gamma TCR d'analyte, de telle sorte que lorsqu'un ORF codant pour la chaîne alpha, bêta, delta ou gamma TCR d'analyte appariée complémentaire est intégré dans le composant **B,** exprimé en tant que **TCRsp** sur le composant **A** et éventuellement dans lequel le composant E code un marqueur de sélection d'intégration.

4. Système multicomposant selon **l'une quelconque des revendications précédentes,** dans lequel le composant **A** contient en outre un composant **appelé F,** un élément de réponse à la stimulation du TCR génomique synthétique sélectionné parmi
a. une construction synthétique à composant unique contenant au moins un promoteur natif et/ou au moins un promoteur synthétique et au moins un rapporteur
b. une construction synthétique multicomposant conçue avec au moins un promoteur natif et/ou au moins un promoteur synthétique et au moins un rapporteur
et dans lequel l'activation de a et/ou b dépend d'au moins une voie de transduction de signal sélectionnée parmi une voie synthétique, une voie native ou une combinaison de celles-ci.

5. Système multicomposant selon la revendication 1, dans lequel les sites récepteurs génomiques B et D sont inclus et sont tous deux une construction synthétique conçue pour l'échange de cassettes médié par recombinase (RMCE).

6. Système multicomposant selon l'une quelconque des **revendications** précédentes, dans lequel l'**aAM** est sélectionné parmi
a. un polypeptide ou un complexe de polypeptides traduit à partir du ou des ORF de molécule antigénique d'analyte
b. un peptide dérivé d'un polypeptide traduit à partir du ou des ORF de molécule antigénique d'analyte
c. un peptide dérivé de la modification du protéome du composant A
d. un polypeptide dérivé de la modification du protéome du composant A
e. un métabolite dérivé de la modification du métabolome du composant A.

7. Système multicomposant selon l'une quelconque **des revendications précédentes,** dans lequel le composant **A** exprime CD4 et/ou CD8.

8. Système multicomposant selon l'une quelconque **des revendications précédentes,** dans lequel le composant **A** exprime des co-récepteurs TCR supplémentaires.

9. Système multicomposant selon la **revendication 8,** dans lequel le composant **A** exprime CD28 et/ou CD45.

10. Système multicomposant selon **l'une quelconque des revendications 2-9,** dans lequel le **composant B et/ou D** est inclus et comprend au moins un des éléments génétiques suivants
a. des sites de recombinase hétérospécifiques
b. des bras homologues
c. un promoteur eucaryote
d. un élément régulateur conditionnel eucaryote
e. un terminateur eucaryote
f. un marqueur de sélection
g. un site accepteur d'épissage
h. un site donneur d'épissage
i. un gène non codant pour une protéine
j. un isolant
k. un élément génétique mobile
l. un site de reconnaissance des méganucléases
m. un site d'entrée interne du ribosome (IRES)
n. un élément peptidique viral auto-clivant
o. une séquence consensus Kozak

11. Système multicomposant selon l'une quelconque des **revendications précédentes,** dans lequel le **composant C et/ou E** est inclus et comprend au moins un des éléments génétiques suivants
a. des sites de recombinase hétérospécifiques
b. des bras homologues
c. un promoteur eucaryote
d. un élément régulateur conditionnel eucaryote
e. un terminateur eucaryote
f. un marqueur de sélection
g. un marqueur de sélection d'intégration
h. un site accepteur d'épissage
i. un site donneur d'épissage
j. un gène non codant pour une protéine
k. un isolant
l. un élément génétique mobile
m. un site de reconnaissance des méganucléases
n. un site d'entrée interne du ribosome (IRES)
o. un élément peptidique viral auto-clivant
p. une cassette de résistance aux antibiotiques
q. une origine de réplication bactérienne
r. une origine de réplication de levure
s. un site de clonage
t. une séquence consensus kozak

12. Système multicomposant selon **l'une quelconque des revendications 1-11,** dans lequel le composant **B** et/ou **D** est inclus et est destiné à l'intégration RMCE d'un seul ORF et comprend :
a. un promoteur eucaryote
b. une paire de sites de recombinase hétérospécifiques
c. une séquence consensus Kozak
d. un marqueur de sélection
e. un terminateur eucaryote.

13. Système multicomposant selon **l'une quelconque des revendications précédentes,** dans lequel le composant **C et/ou E** est présent et est destiné à l'intégration RMCE d'un seul ORF et comprend les éléments génétiques suivants :
a. une paire de sites de recombinase hétérospécifiques
b. une séquence consensus Kozak
c. une cassette de résistance aux antibiotiques
d. une bactérie de réplication
e. un site de clonage pour l'introduction d'un seul ORF codant pour une ou plusieurs chaînes TCR et/ou marqueur de sélection d'intégration.

14. Système multicomposant selon **l'une quelconque des revendications précédentes,** dans lequel le composant C et/ou E est inclus et peut être combiné avec au moins un ORF codant pour au moins une chaîne TCR d'analyte pour obtenir le composant C' et/ou E'.

15. Système multicomposant selon **la revendication 14,** dans lequel la combinaison peut être effectuée plusieurs fois pour obtenir une bibliothèque de composant C' et/ou E'.

16. Système multicomposant selon la revendication 14 ou 15, dans lequel les séquences codantes pour la chaîne TCR d'analyte sont issues de
a. un clonage apparié d'une ou plusieurs séquences ORF de chaîne TCR à partir de lymphocytes T primaires
b. un clonage non apparié d'une ou plusieurs séquences ORF de chaîne TCR à partir de lymphocytes T primaires
c. une ou plusieurs séquences ORF de chaîne TCR synthétique.

17. Système multicomposant selon l'une quelconque des **revendications 14-16,** dans lequel un ou plusieurs composants C' et/ou E' peuvent être combinés avec le composant A, pour intégrer deux chaînes TCR d'analyte complémentaires codées dans le composant C' et/ou E', en composants **B** et/ou **D,** pour obtenir une cellule, **appelée eTPC-t,** dans lequel les composants B et/ou D deviennent les composants B' et/ou D' de sorte qu'elle exprime un **TCRsp** à la surface du composant A.

18. Système multicomposant selon l'une quelconque des **revendications 14-17,** dans lequel l'un des composants C' ou E' peut être combiné avec le composant A, pour intégrer une chaîne TCR d'analyte codée dans le composant C' ou E', en composants B ou D, pour obtenir une cellule, **appelée eTPC-x,** dans lequel les composants B ou D deviennent des composants B' ou D' de telle sorte que l'eTPC-x exprime une seule chaîne de TCR.

19. Système multicomposant selon l'une quelconque des **revendications 15-18,** dans lequel l'un des composants C' ou E' peut être combiné avec une **eTPC-x,** pour intégrer une chaîne TCR d'analyte codée dans le composant C' ou E' qui est complémentaire de la chaîne TCR exprimée dans **l'eTPC-x,** en composants **B** ou **D,** de l'**eTPC-x,** pour obtenir une **eTPC-t,** dans lequel les composants B ou D deviennent des composants B' ou D' de telle sorte qu'elle exprime un **TCRsp** sur la surface de l'eTPC-t.

20. Procédé de préparation d'une eTPC-t telle que définie dans **la revendication 17,** le procédé comprenant
a. la fourniture du système multicomposant selon **la revendication 1,**
b. la combinaison du composant A avec au moins un des composants C' et/ou E', dans lequel les un ou plusieurs composants C' et/ou E' codent une ou plusieurs paires de chaînes TCR d'analytes complémentaires, et la combinaison avec des facteurs d'intégration et au moins une parmi
c. la sélection en vue d'une perte du ou des marqueurs de sélection de site récepteur génomique
d. la sélection en vue d'un gain d'une expression de surface du **TCRsp** et/ou d'une ou plusieurs protéines **CD3**
e. la sélection en vue du gain d'un ou plusieurs parmi un marqueur de sélection d'intégration.

21. Procédé selon **la revendication 20,** dans lequel les un ou plusieurs composants C' et/ou E' code une seule paire complémentaire de chaînes TCR à l'étape b.

22. Procédé selon **la revendication 21,** dans lequel le procédé est effectué plusieurs fois dans lequel chaque étape temporelle b est effectuée en utilisant une seule paire complémentaire distincte de chaînes TCR, de telle sorte qu'une **eTPC-t** distincte soit obtenue, pour obtenir une bibliothèque de **eTPC-t** discrètes et définies.

23. Procédé selon **les revendications 21-22,** dans lequel les un ou plusieurs composants C' et/ou E' codent un pool mixte de deux paires complémentaires distinctes ou plus de chaînes TCR à l'étape b de la revendication 22, pour obtenir une bibliothèque, dans lequel la bibliothèque est composée d'une population mixte d' **eTPC-t,** dans lequel chaque **eTPC-t** exprime un seul **TCRsp** issu du pool utilisé à l'étape b de la revendication 23.

24. Procédé de préparation d'une **eTPC-x** telle que définie dans **la revendication 18,** le procédé comprenant
a. la fourniture du système multicomposant selon **la revendication 1,**
b. la combinaison du composant A avec un ou plusieurs composants C' ou un ou plusieurs composants E', dans lequel un ou plusieurs composants C' ou un ou plusieurs E' codent une chaîne TCR d'analyte, et la combinaison avec des facteurs d'intégration et au moins un parmi
c. la sélection en vue d'une perte du ou des marqueurs de sélection de site récepteur génomique
d. la sélection en vue du gain d'un ou plusieurs parmi un marqueur de sélection d'intégration.

25. Procédé selon **la revendication 24,** dans lequel un composant C' ou un composant E' code une chaîne TCR d'analyte unique à l'étape b.

26. Procédé selon **la revendication 25,** dans lequel le procédé est effectué plusieurs fois, dans lequel chaque étape temporelle b est effectuée en utilisant une chaîne TCR d'analyte distincte, de telle sorte qu'une **eTPC-x** distincte soit obtenue, pour obtenir une bibliothèque d'**eTPC-x**distinctes et définies.

27. Procédé selon **la revendication** 25 **ou 26,** dans lequel un ou plusieurs composants C' ou un ou plusieurs composants E' codent un pool mixte de deux chaînes TCR à analyte unique ou plus à l'étape b, pour obtenir une bibliothèque, dans lequel la bibliothèque est constituée d'une population mixte **d'eTPC-x,** chaque **eTPC-x** exprimant une seule **chaîne TCR d'analyte** issue du pool utilisé à l'étape b.

28. Procédé de préparation d'une **eTPC-t** telle que définie dans **la revendication 18,** le procédé comprenant
a. la fourniture du système multicomposant selon **la revendication 1,**
b. la combinaison d'une **eTPC-x,** avec un ou plusieurs composants C' ou un ou plusieurs composants E', dans lequel un ou plusieurs composants C' ou un ou plusieurs E' codent une chaîne TCR d'analyte qui est complémentaire de la chaîne TCR d'analyte intégrée dans **l'eTPC-x,** et la combinaison avec des facteurs d'intégration et au moins l'une parmi
c. la sélection en vue d'une perte du ou des marqueurs de sélection de site récepteur génomique
d. la sélection en vue d'un gain d'une expression de surface du **TCRsp** et/ou d'une ou plusieurs protéines **CD3**
e. la sélection en vue du gain d'un ou plusieurs parmi un marqueur de sélection d'intégration.

29. Procédé selon **la revendication 28,** dans lequel un composant C' ou un composant E' code une chaîne TCR d'analyte unique à l'étape b

30. Procédé selon **la revendication 29,** dans lequel le procédé est effectué plusieurs fois, dans lequel chaque étape temporelle b est effectuée en utilisant une chaîne TCR d'analyte distincte, de telle sorte qu'une **eTPC-t** distincte soit obtenue exprimant un **TCRsp,** pour obtenir une bibliothèque **d'eTPC-t** distinctes et définies.

31. Procédé selon **les revendications 28-29,** dans lequel un ou plusieurs composants C' ou un ou plusieurs composants E' codent un pool mixte de deux chaînes TCR à analyte unique ou plus à l'étape b, pour obtenir une bibliothèque, dans lequel la bibliothèque est constituée d'une population mixte **d'eTPC-t,** chaque **eTPC-t** exprimant un seul **TCRsp** issu du pool utilisé à l'étape b.

32. eTPC-t obtenue à partir du procédé selon la revendication 20, destinée à être utilisée dans un dispositif analytique d'eTPC:A pour la caractérisation de
a. la spécificité de l'analyte **TCRsp** exprimé par rapport à un **antigène analyte** et/ou
b. l'affinité de l'analyte **TCRsp** exprimé pour un **antigène analyte** et/ou
c. un signal de réponse de l'analyte **TCRsp** exprimé à un **antigène analyte** dans laquelle un antigène analyte est choisi parmi un aAPX:aAM et/ou **aAM** et/ou **aAPX** et/ou aAPX:CM et/ou **réactif d'affinité,** dans lequel l'antigène analyte se présente sous la forme d'au moins les éléments suivants, un réactif soluble, un réactif immobilisé, présenté par une particule non cellulaire **(NCBP)** présentée à la surface d'une cellule **(analyte APC),** dans laquelle **aAPX:CM** est un **aAPX** qui est chargé d'une molécule cargo **(CM),** et dans laquelle **aAPX:aAM** est un **aAPX** qui est chargé d'un **aAM** en tant que **CM.**

33. eTPC analyte pouvant être obtenue à partir du système multicomposant tel que défini dans l'une quelconque des revendications 1-19, **caractérisée en ce que**
a. elle contient 2 sites récepteurs génomiques, appelés B et D, dont chacun peut comprendre un seul ORF codant pour au moins une chaîne TCR d'analyte alpha, bêta, delta ou gamma, et/ou deux ORF codant pour une paire de chaînes TCR d'analyte
b. elle exprime les protéines CD3
c. elle n'exprime pas d'autres chaînes TCR que celles comprises en B et D,
et dans laquelle les sites récepteurs génomiques B et D sont chacun sélectionnés parmi
a. une construction synthétique conçue pour l'échange de cassettes médié par recombinase (RMCE)
b. une construction synthétique conçue pour la recombinaison homologue dirigée sur un site
et dans laquelle l'eTPC analyte a été combinée avec au moins l'un de C' et/ou E' tel que défini dans l'une quelconque des revendications 17 à 19 de telle sorte qu'au moins l'un des composants B et D devienne le composant B' et/ou D', *dans lequel l'intégration aboutit à l'expression de chaînes TCR d'analyte par l'eTPC cible.*
